# EUROPEAN PATENT SPECIFICATION

(11) **EP 3 105 198 B1**
(45) Date of publication and mention of the grant of the patent: **16.02.2022**
(21) Application number: 15710595.8
(22) Date of filing: 10.02.2015
(51) Int. Cl.: C05F 11/08

(54) **SOIL BACTERIA FOR INOCULATING STRESS SOILS**
BODENBAKTERIEN ZUM IMPFEN VON BELASTETEN BÖDEN
BACTÉRIES DE SOLS POUR L'INOCULATION DE SOLS STRESSÉS

(30) Priority: 10.02.2014 HU P1400062
(43) Date of publication of application: 21.12.2016
(73) Proprietor: Biofil Mikrobiológiai, Géntechnológiai És Biokémiai KFT., 1139 Budapest (HU)
(72) Inventor: KUTASI, József, H-2132 Göd (HU); KÁRPÁTI, Éva, H-2100 Gödöllõ (HU); KOVÁCS, Rita, H-1044 Budapest (HU); SZKLADÁNYI, Sándor, H-1139 Budapest (HU); IMRE, Csilla, H-1061 Budapest (HU); PÉK, Nikoletta, H-2133 Sz dliget (HU); ERDÉLYI, Balázs, H-1138 Budapest (HU)
(74) Representative: Lengyel, Zsolt
(86) International application number: PCT/IB2015/050996
(87) International publication number: WO 2015/118516

(56) References cited:
- WO-A1-03/016241
- WO-A1-2009/091557
- WO-A1-2010/109436
- WO-A2-99/09834
- WO-A2-2012/093374
- Tállai-Anita Magdolna ET AL: "COMPARATIVE EXAMINATION OF A BACTERIUM PREPARATION (BACTOFIL A10) AND AN ARTIFICIAL FERTILIZER [CA(NO 3 ) 2 ] ON HUMIC SANDY SOIL", , 1 January 2012 (2012-01-01), XP55194535, Retrieved from the Internet: URL:http://protmed.uoradea.ro/facultate/an ale/protectia_mediului/2012A/agr/12.Tallai Magdolna.pdf [retrieved on 2015-06-09]
- Ida Kincses ET AL: "THE EFFECT OF BACTERIA FERTILIZERS ON THE NITROGEN AMOUNT EXTRACTED BY THE YIELD OF PERENNIAL RYEGRASS (LOLIUM PERENNE) ON DIFFERENT SOIL TYPES", , 1 January 2008 (2008-01-01), XP55194541, Retrieved from the Internet: URL:http://protmed.uoradea.ro/facultate/an ale/protectia_mediului/2008/agr/Kincses.pd f [retrieved on 2015-06-09]
- Növény: "Mezogazdasági Szakigazgatási Hivatal", , 3 February 2011 (2011-02-03), XP55420274, Retrieved from the Internet: URL:http://portal.nebih.gov.hu/documents/1 0182/287690/bactofilcsalad.pdf/f1d35acf-6a 5b-4bcf-a904-5e5ba34fe6b5 [retrieved on 2017-10-31]

## Description

In agriculture, chemical fertilization and excessive irrigation often leads to leaching and deterioration of soils, resulting in alkaline-saline, salty and acidic soils. Soil inoculation is a potential tool for the amelioration of deteriorated plow fields. Commercially available soil inoculants are not adapted to extreme soil conditions. A special screening system has been developed for the identification of strains that are able to grow in "stress" conditions - low temperature, extreme pH - and exert their plant growth promoting and soil-life enriching properties. Strains of the inoculants have been selected as to act in synergy to sustain their viability and secrete plant growth stimulating metabolits.

Intensive agricultural production and the use of chemical fertilizers in the past 50 years have deteriorated, impoverished or salinized the soil in 65% of all arable lands (Nature, 2009). According to the FAO database in regard to soil quality, 6% of all soils potentially available for agricultural production is sodic and saline, 15% is strongly acidic (pH<4.5), a further 14% (in areas with heavy rainfall) is weakly acidic. As an inevitable consequence of soil deterioration, the microbial flora of soils impoverish as microbes cannot grow in desiccated soils, or in highly varying pH, which leads to the disintegration of the soil structure. Such destructuring of the soil hinders humification and soil organic matter formation and so the soil loses its fertility in a matter of years, due to the continuously decreasing supplies of nitrogen and phosphorus. In lack of adequate structure, artificial fertilizers cannot bond but leach and contaminate surrounding aquatic habitats.

Increasing demands of the growing population necessitates vast areas of land to be drawn into agricultural cultivation in Africa, Asia and South America in the coming years. It carries the risk of large areas to be desertified in Africa and Asia due to inappropriate - unsustainable - tilling and cultivation methods. The process of desertification has already started. Modern biological methods can prevent the excessive use of artificial fertilization and its adverse consequences - bound to happen in just decades - on soil and natural waters in Africa, where drinking water is precious, and in Asia. The combination of soil inoculation and chemical fertilization (at a reduced rate) could prevent soil degradation problems already present in Europe and North America. Biological soil revitalization is an alternative method which is not labor-intensive, is safe and environmentally friendly (Caplan, 1993; Dua et al., 2002). Soil inoculation is done on-site - without transporting the soil - involving certain plant species and microbial strains that uptake, bond or decompose contaminants (Heitzer and Sayler, 1993) and improve soil life. In the long term, the biological method is economical as crop yields increase, and the quantity of applied fertilizers, and consequently the environmental load, decrease.

In Hungary - and in Europe in general - a considerable rate of arable land used for the cultivation of economically important food and feed crops is made out of deteriorated soils - low fertility soils with unfavorable pH, salt concentration or poor drainage, in summary, stress soils. Soils in Hungary are mostly weakly acidic or carbonated, neutral soils. In a lower rate, but in a considerable area of land, strongly acidic and alkaline-saline soils are present (Stefanovits, 1963) according to the survey of the agro-ecological potential of the country (Várallyay et al., 1980): 43% of soils is weakly acidic, 13% is strongly acidic, 38% is carbonated and 8.1 % is saline. More recent data show that of all soils in Hungary, 8% is strongly acidic (pH KCL < 4,5), 18% is medium acidic soil (pH KCL 4,5-5,5) and 20% is weakly acidic (pH_{KCl} 5,5-6,5). In Hungary, acidic forest soil, meadow soils and sandy soils cover up to 1.5 million ha, sodic and secondary salinized areas make up to 700,000 - 800,000 ha. Sodic soils are found in 8.1 % of the area of the country. Sandy soils are present in also 8%, on an area of 746,000 ha. Since these soils make up a considerable proportion of cultivated lands, they cannot be excluded from agricultural use. Cultivation has to be maintained, in spite of low fertility, and in a sustainable and environmentally friendly way.

Acidification causes the soil to impoverish in basic cations and nutrients - loss of cementing substances -, the breaking down of clay minerals, which eventually leads to disintegration in the soil structure and an increase in the solubility of metallic ions. The microbial flora in acidic soil changes typically, moulds become predominant. These changes bring about loss in crop quality and quantity. One of the primary causes of soil acidification is the overuse of chemical fertilizers. The most commonly applied substance, the ammonium sulphate and superphosphate form an acidic aqueous solution, which increases the H⁺ concentration in the soil colloid, leading to a decrease in pH. Cation-exchange further increases acidity, as cations from the chemical fertilizers - NH4+, K+, and Na+ - are absorbed on the surface of soil colloids, replacing H+ which becomes solubilized. Acidification leads to the leaching of Ca, Mg Na and K ions. In sum, the decrease of pH lessens the amount of available N- and P-supply for the vegetation, and the loss of soil aggregates and cementing substances causes to the soil to lose water restoring capacity, and capping occurs.

Primary or natural salinization is the salt accumulation in areas where the mother rock or the shallow groundwater is rich in salts. Secondary - in most cases anthropogenic - salinization is a result of irrigation and / or inadequate water drainage. The use of different additives (e.g. fertilizers) may cause salinization on cultivated lands with low water permeability. High salt concentration inhibits nutrient uptake and contaminates the water source of plants. High amounts of salt damages soil structure, the soil packs and an impermeable upper layer - a cap or crust - is formed, which deprives soil life from oxygen, causing imbalance in the soil ecosystem. The metabolism of the soil microbes changes, which leads to impaired soil life and a rapid decrease in fertility. Interestingly, sodic soils are potentially rich in nutrients, yet they are unavailable for plants due to bad physical properties or the high salt concentrations. Salinization retards and impairs plant growth due to the toxicity of high salt concentrations and the increase in osmotic pressure in the soil colloid. A white layer is usually observed on the soil surface, which is made up of soluble salt (in alkaline-saline solonchak soil), soda, or - in solonetz soils - amorphous silica (SiO₂). Neutral or acidic salinized soils also occur.

A traditional method for the treatment of acidic soils is liming with beet potash, lime, lime-supplemented wetland soils or muskeg, (in case of acidic forest soils. meadow and sandy soils, acidic salinized soils). Liming acts in multiple ways: reduces abnormal acidity, provides Ca-supply of plants and improves soil structure. However, as liming materials are becoming less available, liming is not widely practiced. Traditional soil ameliorating methods on sodic and secondary salinized cultivated lands have also been less frequently applied in the past decade, primarily due to high costs. According to recent publications, soil inoculants adapted to acidic or alkaline-saline soils and that are generally applicable to several field cultivars have not been routinely used anywhere in the world. A single example is known, the inoculation of leguminous species on strongly acidic soils, as a result of long and intensive research and development in Australia. However, the specificity of these inoculants for leguminous plants limits their range of application for agricultural use.

The native microbiota of acidic, sandy and alkaline soils are specific and typical. Certain indwelling bacterial strains are microbiologically and enzymatically active and effective even is such extreme conditions. These adapted microbes are more rapid in growth, humification and mineralization than soil microorganisms that are normally adapted to more balanced soil conditions when applied in extreme soil conditions. Yet, deteriorated soils are usually inoculated with commercially available soil inoculants, containing strains adapted to "normal" conditions, only. Several soil inoculants - containing strains not adapted to deteriorated soils with extreme conditions - have been applied to stress soils in combination with traditional ameliorating methods, usually without success. Agricultural practice evidently shows that such inoculants are not effective in extreme soil conditions and it is apparent that there is a great need for bacterial preparations that effectively enhance plant growth and soil life in deteriorated or originally low-fertility soils. The invention provides a complex microbial method for the preparation of soil inoculant compositions, specific strains and soil inoculant compositions that tolerate the condition present in stress soils well and effectively enhance the yield of food and feed crops produced on such specific soils.

Endogenous/endophytic bacteria that are naturally present in the plant organism, and the rizospheric microflora colonizing the root region are crucial in initiating nutrient transport and in balancing the carbon- and nitrogen metabolism in the course of soil formation (Morris and Haskin, 1990; Bagwell et al., 1998). Combined use of a plant species and symbiotic bacteria is advantageous in many ways: the plant host promotes the proliferation and metabolism of the microbial population in the rizosphere by excreting nutrients via the roots, which create an environment rich in resources, that stimulate microbial activity (Lugtenber and de Weger, 1992). The plant affects and improves physico-chemical parameters of the soil, and establishes contact between root-associated microbes and resources, and potentially contaminants (Aprill, 1990). On the other hand, rizospheric microorganisms positively influence the nutrient-supply of plants (nutrients are released by microbial mineralization) and plant growth by producing growth-stimulating bioactive substances (Campbell and Greaves, 1990). Soil rehabilitation could be facilitated by industrially produced bacterial soil inoculants that act against abiotic stress-related growth inhibition by producing auxins and cytokinins. They promote seed germination, and enhance biomass production. Experiments proved that bacterial soil inoculants improve viability and water content of plants, maintaining the chlorophyll content and chlorophyll a/b ratio, and promote root growth (Glick, 2003). Certain strains may fix and transform aerial nitrogen, synthesize siderophores that promote iron-uptake from the soil, or solubilize insoluble or poorly soluble minerals, providing e.g. available phosphorus supply for plants (Glick, 2003). These effects greatly contribute to the efficiency and the speeding up of the revitalization process.

In the ameliorization of deteriorated soils it is crucial to provide a stable, constant nutrient supply for the cultivated plants. Nitrogen-supply is especially important in these soil types, known to be severely nitrogen-deficient (Morris, 1991). Chemical nitrogen-supply is of low efficiency, as poor soil structure does not prevent the fertilizer to leach, to be washed out of the soil and cause nitrate contamination in surface and ground waters. The sudden and substantial nitrogen-deficiency can cause the devastation of growing crops. The key of reliable nitrogen-supply is biological nitrogen fixation which means that nitrogen fixing (diazotroph) bacterial species provide a source of nitrogen for the host plants by fixing and reducing aerial nitrogen into ammonia. In contrast with chemical fertilization, a diazotroph bacterial population provides a constant and balanced supply of nitrogen for the plant. Diazotroph bacterial species that also exert a growth promoting effect by producing phytohormone-like substances are of special value in agricultural biotechnology. Such species are especially effective if they are endophytic/endogenous and colonize not only on the surface of roots, but in also in plant tissues (Piceno et al., 1999; Piceno and Lowell, 2000), providing an abundant and almost direct transfer of ammonium and phytohormones to the host plants.

In the rizosphere, namely on the surface and in the close proximity of the roots, a diverse and rich population of bacteria is dwelling, usually forming microcolonies. Some of them simply utilize the nutrients secreted from roots. Others are symbiotic, acting beneficially towards the host plant, by direct or indirect stimulation of growth. These species are termed as 'plant growth promoting rhizobacteria' (PGPR) (Vessey, 2003; Kennedy et al., 2004; Kloepper, 1994).

PGPR can be classified according to their specific effects:
- nitrogen-fixing species that can be considered as biological fertilizers by fixing, transforming and transferring aerial nitrogen to the host plant, in nitrogen-deficient conditions;
- mineral solubilizing species that make insoluble phosphorus and potassium minerals available for plants
- growth stimulating species, that produce hormone-like substances and directly promote growth and development
- species having a biocontrol effect that inhibit the growth and damage of phytopathogens (*Fusarium, Phytium, Botritis, Xantomonas*, etc.)

Nitrogen supply is enhanced by inoculation with nitrogen fixing soil bacterial species. Some diazotroph species are free-living, others are in a more or less tight connection with root surface, or even colonize inside the root tissue. Through biological nitrogen fixation, the bacteria fix aerial nitrogen and transform it into ammonia, available for uptake for the host plant. The root nodules of leguminous plants are colonized by rhizobia (*Sinorhizobium, Rhizobium, Bradyrhizobium,* etc.) living in symbiosis with the host plants (Franche et al., 2009).

The nitrogen fixing species that are found as biological fertilizers in the commercially available soil inoculants mainly are the free-living therefore less effective *Azotobacter* and the more effective, associative symbiontic, diazotroph *Azospirillum brasilense*, and *A. lipoferum* (for cereals, maize) and *Rhizobium, Bradyrhizobium, Sinorhizobium* bacteria (for leguminous plants). The *Azotobacter* species live near the root, but does not come into close contact with the root. They are able to bind to molecular nitrogen under aerobic conditions. The *Azotobacter* species, although are not associative nitrogen fixing bacteria, may live in close contact with the roots of maize, but in contrast to the *Azospirillum,* they do not associate with any single plant family, therefore they are able to stimulate non-specific growth of various plant species. They are free-living in the soil, do not form an associations with the plant (except for *A. paspali*). In the domestic soils, mainly *Azotobacter vinelandii* es *A. chrococcum* species live. The nitrogen fixing capacity of *A. vinelandii* is very high because-contrary to the *Rhizobium* and *Azospirillum* species - it has three types of enzyme complexes (alternative nitrogenases) for the reduction of atmospheric nitrogen. However, it does not establish a direct connection to the plant root, so the ammonia produced during nitrogen fixation can be utilized by the plants with only a greater or lesser loss, so that the amount of nitrogen supplied to the plants is not significant.

The roots of the economically important cereals (wheat, corn, rice, barley, millet, sorghum, etc. are colonized by nitrogen fixating and plant growth-promoting diazotroph aerobic and microaerophilic azospirillums that live on the surface of the roots and some of their strains are able to colonize the inner tissues of the root and stem. Their coexistence with the host plant is called associative symbiosis (reviewed in Dobereiner, 1989).

The positive effect of Azospirillum bacteria on host plants:
- is the result of the hormone-type molecules promoting the growth of the host plant produced by the bacteria, primarily indole-3 acetic acid;
- are capable of nitrogen fixation under microaerophilic conditions, thus the plant is fed nitrogen bound from the air;

- and are also capable of nitrate reduction, and an intermediate product of the process, the nitrite, also has growth promoting effects on plants (Tian et al, 1979; Okon es mtsai., 1976; Hartmann es Zimmer, 1994; Michiels es mtsai., 1994).

The plant growth-promoting abilities of the bacteria are varied, and the production of phytohormone like materials is particularly important in agricultural applications (reviewed by Ahemad and Kibret, 2013). At the same time, Gram negative, facultative anaerobic, e.g. *Pseudomonas* and Gram positive, aerobic, spore-forming, e.g. *Bacillus* species produce plant growth-promoting substances in large quantities at both on the surface and in the proximity of the root. The different Gram positive and Gram negative aerobic, facultative anaerobic strains found in the rhizosphere and on the root surface produce indole-3 acetic acid, gibberellin-like substances, biotin and pantothenic acid. The most important representatives of the auxins is indole-3 acetic acid (IAA), which is found in all plants. The synthesis of indole-3 acetic acid occurs in the shoot tip from tryptophan. The most obvious effect of auxins is the stimulation of growth by elongation and cell division. However, the increased potassium ion concentration causes the water to flow into the cell, and the resulting increase in cell volume eventually leads to cell elongation. In addition to the control of cell elongation, auxin also mediates the action of gravity and light. In addition, the hormone regulates the synthesis of cellulose and pectin materials of the cell wall and lignin production as well. The auxin induces mitosis and delays the detachment of the leaf and fruit, the abscission (Császár, 2004). It also accumulates in the seeds for the germination.

The auxins in plants affect all growth and developmental phenomenon, mostly in cooperation with other hormones. Different plant organs require different auxin concentrations for their growth. Above the optimum quantity, auxin is inhibiting. Together with the gibberellins, auxin has definitive role in the sexual nature of flowers, and they act together in the differentiation of transport tissues. The apical dominance occurs due to the cooperation with abscisic acid. Together with cytokinins, it stimulates cell division. The hormone family of auxins and cytokinins regulates many steps of plant development. They usually exert their effects on the formation of vegetative organs in combination, and the ratio of auxins and cytokinins determines the direction of morphogenesis. The group of gibberellins includes many compounds of similar structure, which exert their effect on the meristematic tissues, both by increasing the proliferation and, secondly, by inducing hypertrophy of undifferentiated cells. The result of their activity is similar to that of the indole-3 acetic acid in many respects, therefore suggesting that they partly act through the stimulation of auxin synthesis.

Soil bacteria are capable of producing indole-3 acetic acid (IAA), belonging to the family of auxins, in large quantities. Most root growth promoting bacteria can produce IAA and their effect on the plant corresponds to the effect of IAA that is administered into the environment of the plant (Glick and Patten, 2002). The IAA-producing soil bacteria can promote the germination of crop plants, resulting in an enriched root system, with the consequent formation of greater absorptive surface. In plants inoculated with *Pseudomonas putida*, 50% longer root growth was noted compared to the IAA defective mutant or un-inoculated plants (Glick and Patten, 2001). The positive effect of inoculation with *A. brasilense* through nitrogen fixation and IAA hormone production has long been known on the fields, especially in the Mediterranean, sub-Mediterranean climate areas (Okon and Labandera, 1994; Okon et al., 1995). The primary effect of azospirillum inoculation is manifested in the intensive growth of the plants root system, enrichment of lateral roots, thereby helping the plant in water and mineral uptake, exploitation of fertilizers, improving the drought tolerance of the plant and the resistance to diseases and stress conditions. Germination and emergence vigor is improved. The successful fertilization increases the total nitrogen and dry matter content of the plant, and the yield of grain. Through the bacterial nitrogen fixation and improved fertilizer utilization due the higher nutrient absorption capacity of the roots, nitrogen fertilizer saving may also be achieved (Fallik et al., 1994; Kapulnik et al., 1985). The *Azospirillum* soil inoculant strains from the Mediterranean and subtropical soils used in European temperate climates are less effective mainly due to their cold sensitivity. At the same time, the European temperate soils contain only low levels of *Azospirillums.* Using soil inoculant compositions containing cold-tolerant strains of *Azospirillum*, they may be enriches in root zone of crop plants.

A further phytostimulating effect is the reduction mechanism of the level of ethylene found in plants, which is used by some plant growth promoting bacteria to promote the growth of the plant. The reduction of ethylene level is carried out by the enzyme 1-aminocyclopropane-1-carboxilate (ACC) deaminase (Glick et al., 2007a, b). To explain the role of ACC deaminase in the growth of plants with respect to free-living soil bacteria associating to the root surface, the following model was established (Glick et al., 1998): the ACC deaminase-expressing bacteria attach to the seed or to the surface of the root, enzymatically cut the ACC secreted by the plant, thereby alpha-ketobutyrate and ammonia are formed. These bacteria are practically operate as an "ACC trapping vessel", thereby reducing the amount of ethylene which produced by the plants under stress conditions (Glick, 2004). Thus they are able to protect the plant from a growth-inhbiting stress condition which may occur for example due to water coverage, extreme temperatures, organic and inorganic toxins, drought or high salt concentrations, plant pathogens (reviewed by Sun et al., 2009). In the case of many plants, ethylene is needed for the germination of the seed, ethylene production rates are increased during germination and seedling growth. Low levels of ethylene enhance the root initiation and effects the elongation of the root; higher levels of ethylene, which are produced by the fast-growing roots, may lead to the inhibition of root elongation (Mattoon and Suttle 1991; Ma et al., 1998).

According to the model by Glick et al., 1998, the PGP bacteria are capable of reducing the ethylene levels and thereby stimulate plant growth (Glick et al. 1998). The bacteria bind to the surface of the seed and the tryptophan or other amino acid exuding from the germinating seed result in the synthesis and secretion of indole acetic acid (IAA). The IAA might be taken up by the seed, and together with endogenous IAA stimulate plant cell division and cell elongation, or induce aminocyclopropane 1-1-carboxylic acid (ACC) synthase synthesis. This enzyme catalyzes the conversion of S-adenosyl-L-methionine (SAM) into ACC, which is the immediate precursor of ethylene (Yang and Hoffman, 1984). It is very probable that the resulting ACC is secreted by the germinating seed; it is taken up by the bacteria, and then hydrolyzed into ammonia and alpha-ketobutyrate using the ACC deaminase enzyme (Glick et al. 1998; Penrose 2000). According to the model, the uptake of ACC and its cleavage by the ACC deaminase lowers the level both of the ACC and that of ethylene in the proximity of the germinating seed, acting as an absorbing vessel (reviewed by Penrose et al., 2001).

The soils under agricultural cultivation contain high levels of water-insoluble phosphorus that is therefore unavailable to plants, in the form of inorganic compounds (apatites and precipitated di- and tricalcium residues from phosphorus-containing fertilizers) and organic compounds (inositol polyphosphates). The phosphate solubilizing bacteria, particularly *Pseudomonas, Bacillus* and *Rhizobium* species solubilize the water insoluble phosphates of the soil into water soluble forms. Because of these properties, they are important components of soil inoculant compositions. In addition to enhancing the P-supply of the plants, their use may increase the yield (Rodriguez and Fraga, 1999, Tao et al.., 2008, Sundara and Sinha, 1962). When inoculating maize, a significant correlation was shown between the increase of the yield and the activity of the phosphate solubilizing *Pseudomonas and Bacillus* species (Kavimandan and Gaur, 1971). By treating cucumber seeds with a mineral phosphate solubilizing *P. fluorescens* strain, faster growth of cucumber could be achieved. Another advantage of the treatment is that in the vulnerable initial phase of the life of the plant the dense population of *Pseudomonas* successfully keeps away the disease-causing microbes (Eklund és Sinda, 1971). The dissolution of mineral (inorganic) phosphates in the most part occurs through the action of organic acids produced by bacteria, primarily by means of gluconic acid (reviewed by Rodrigez and Fraga, 1999). The production of organic acids acidifies both the bacterial cell and the environment thereof. The mineralization of most organic P-containing substance is carried out by bacterial acid phosphatase enzymes in the soil (Tarafdar and Junk, 1987; Tarafdar and Claassen, 1988). The advantageous effects of the soil replenishing inoculation are further increased when mixed with phosphate-dissolving *Pseudomonas* and *Bacillus* species. The N and P uptake, dry matter content and grain yield of the treated plants exceeds the sum of effects achieved by the individual inoculants due to a more balanced supply of nutrients to the plant. Therefore the advantageous effect of soil replenishing inoculation may be further increased if in addition to the nitrogen fixing diazotrophic Azospirillum bacteria, phosphate solubilizing *Pseudomonas* and *Bacillus* species are inoculated in combination.

Some of the PGPR bacteria has biocontrol effects and also capable of inhibiting the soil plant pathogens (Gerhardson 2002). Certain *Pseudomonas* and *Bacillus* species are capable of repel plant pathogenic bacteria and fungi from the rhizosphere by antibiosis - with the production antibiotics (reviewed by Szabo, 1992). The most important biocontrol bacteria occurring in soil inoculant compositions are pseudomonas, bacillus and streptomyces (reviewed by Weller, 1988; Haas and Defago, 2005). Their beneficial effects are primarily the reduction of root impairing fungi and bacteria, the reduction of the damage caused thereby. Their biocontrol activity consists of several factors: (1) quickly and efficiently consume the nutrients, micro and macro elements of the rhizosphere, and the nutrient poor environment is disadvantageous for the pathogens, (2) due to their intensive root colonizing capacity, they expel the pathogens from the surface of the root, (3) they induce so-called systemic resistance in the plant, by which it will be more resistant to a broad range of pathogenic fungi, bacteria and viruses.

The soil bacteria produce siderophores capable of collecting iron, thereby when there are low levels of iron, an iron deficiency occurs in the microenvironment of the bacteria. Since the pathogenic bacteria and fungi proliferating in the rhizosphere cannot utilize the iron from the siderophores, they suffer growth inhibition. The iron plays a central role in the metabolism of obligate and facultative aerobic micro-organisms, even though it is present in very small amounts in the environment. The low availability of iron is the consequence of the low solubility of mainly ferric oxyhydroxide polymers entering the soil from the plants. For this reason, the concentration of iron and / or microelements is one crucial factor that determines the microbial consortium of the microflora of the particular habitat. In such environments, mostly those species can live or "dominate" that have well-built and highly effective iron-acquisition and / or microelement mechanism. At the same time, the plants are able to uptake the siderophore bound iron.

In the case when the iron is less available in the environment of the microorganisms, many organisms starts to produce low molecular weight metabolites, siderophores and outer membrane proteins that are characteristic to the species and has high affinity for Fe3+ (the latter play a role in the recognition of Fe - siderophore complex and the absorption of iron). The siderophores extract the microelements from the minerals and organic compounds (such as transferrin, lactoferrin). Siderophores are small molecular weight, divalent ligand molecules that are mainly attach through the oxygen atom to the Fe(III) ion with six bonds on octaeder directions, and draw the Fe3+ ions from the environment in the form of chelates and transport them into the microbial cell. The transport of microelements into the cell's cytosol is mediated by a specific membrane receptor of the transport system that recognizes the iron - siderophore complexes. Thus the siderophore production of micro-organisms is an important phenomenon for maintaining the propagation capability - especially under conditions of stress soils.

The use of the bacterial inoculant composition will be advantageous for providing a solution for one of the major problems occurring on soils, the deterioration of the soil structure, because the improvement of the soil microstructure is greatly enhanced by the exopolysaccharide (EPS) - soil aggregate promoting ability of our inoculants. The structure of the soil is determined by the combination of holes in the soil, aggregates and micro-structures. This is practically microscopic structure of the soil formed by the soil animals (e.g. earthworm) and soil bacteria by their biological and microbiological activity ("microfabrics" Barratt, Breweer 1964). From a microbiological point of view, the most important aspects is the formation of soil aggregates.

Surprisingly, the formation of these coatings - biofilm - associated to the soil particles may be facilitated by soil inoculants. The specific bacteria introduced into the soil are capable to cement the soil particles formed from the combination of clay, sand and organic material into a soil aggregate by their cellular material, exopolysaccharide (EPS) materials and biofilm formation induced by siderophore production. The soil aggregates generated in large quantities increase the viability of the soil, and the water retention and plant colonization activity thereof. Moreover, as a result of all these effects, the compaction of the soil is reduced, thereby its cultivability is improved. By repeating the appropriate inoculation, the biodynamic of the soil renewed annually and the soil development will be continuous and sustainable with the use of the bacteria.

The formation of aggregates and colonization - the quorum sensing (QS) effect - involve large numbers of *Bacillus, Azotobacter, Pseudomonas* and *Micrococcus* bacteria (Lynch, 1981). Furthermore, the *Stenotrophomonas* and *Sphingobacterium* species are very effective at aggregate formation, as it was shown in barley and alfalfa fields' soil (Caesar-TonThat et al., 2007). The bacteria stick to the soil particles by forming a film like coating ("monolayer") or stick together with filamentous (*Actinomycetes, Streptomyces*) bacteria in plant tissue remains. During the communication between the bacterial cells - quorum sensing (QS) -, the bacteria are capable to synchronize their genetic functions and produce certain compounds when proliferate in high numbers. This occurs when the bacteria reach an amount where they form a layer, "biofilm", for example on the surface of the soil particles. This may occur when they are in sufficient numbers in the soil particles. The increase of the concentration of the secreted QS signal molecules is a signal for the bacteria to start intensive proliferation. The QS molecules are mainly long chain fatty acids, quinols, methyl esters of fatty acids, N-acyl homoserine compounds, and similarly to the siderophores, surprisingly, attach to specific outer membrane protein receptors of the bacteria. According to our hypothesis, these QS molecules may also be siderophores, thus may exert their effects as signal molecules. This helps the communication between the bacteria and thus their proliferation, colonization and biofilm formation.

The colonization ability of inoculant strains affects the reproduction of the others, therefore, a uniquely special colonization - competition (CoCoHelp) method was developed to prepare appropriate soil inoculant strain compositions. The method - where we were looking for answers to the problem of how to find out which bacteria are capable of propagation in cooperation (even achieving a synergistic effect), or to the contrary, they inhibit the growth of each other, or maybe they are not in cooperation but also not inhibiting each other and may propagate next to each other. All these answer the question that which aggressively colonizing or strong siderophore producing, thus inhibiting or, to the contrary, even "helper" bacterial strains could be inoculated into the soil in the same culture, therefore in the inoculated soil they act together, without inhibiting the propagation of each other, or even helping in the colonization in the case of certain strains, thus are suitable as soil inoculant composition.

Several document is known in the literature that relate to the production and use of plant growth promoting rhyzobacteria, in defense against abiotic stress conditions. Damodaran et al. (2013) is a specific example for the isolation and biochemical characterization of rhyzobacteria (e.g. Bacillus strains) tolerant against extreme salt concentration. They studied the strains up to 10% salt concentration.

Kutasi et al. (2009) and Bish et al. (2013) disclose the creation and test of cold stress tolerant Azospirillum and Pseudomonas strains- Tests were performed at 4 ° C, as well.

New et al. (1989) isolated and tested Azospirillum strains with tolerance for extremely low pH. pH was tested at pH 4-8 and they further disclose selection biochemical assays described in the application. Bianco Carmen and Defez Roberto (2011) discuss the bacteria involved in abiotic stress.

The WO 03016241 and WO 2010109436 documents disclose a mixture of bacterial species (e.g.: Azospirillum, Pseudomonas). The latter teaches the search for a synergistic combinations.

The specific soil bacteria disclosed herein are able to contribute effectively and in harmony with the fertility of the soil to enhance growth of crop plants, plant nutrition, soil microstructure (water supply), improving soil life, etc. By isolating and identifying suitable bacterial strains - adapted to the specific conditions -, these may be used in so-called mixed-type inoculation, where the soil inoculant composition already contains the microbes that are proliferating rapidly at the given area, mineralizing, producing hormones, siderophores and exopolysaccharide, as well as the selected N-fixing bacterial strains. The bacterial strains isolated from acidic and saline areas were screened for the microorganisms that are supposed to be able under alkaline, acidic and / or salty soil conditions to enhance humification, soil aggregate formation, nutrient supply - macro and micro-nutrients, primarily nitrogen and phosphorus and iron - and producing materials that promote the development of crops.

By using the so-called Soil Bacteriae Screening System (SBSS) disclosed in the invention, we intend to extend the effectiveness of soil inoculation for enhancing the activity of still prolific, but depleted areas, including the supplementation of the lacking macro (especially nitrogen) and meso-elements (e.g. iron) by using bacterial nitrogen fixation and siderophore production, in addition to stimulating plant growth and improving the soil structure. Accordingly, the soil inoculant composition of the invention is useful for improving the nitrogen-poor soils in depleted areas, thus helping the inclusion of the land in agricultural production.

From soils to be treated and also found in Hungary, we isolated microorganisms that are present on the particular soil types (sandy soils, strongly acid brown forest soils, alkaline saline and acidic saline soils, acidic meadow soils and sour alluvial) and support the vegetation adapted to stress effects (salt and / or sour and / or alkaline pH) and were thus far not known. The isolated and developed bacterial strains are new isolates that have not yet been used and suggested for soil inoculation, as well they have growth-promoting effects, they produce plant hormones and / or are good at nitrogen fixing, which are effective in associations and as free-living bacteria, or favorably help the uptake of various macro and micronutrients, or siderophore producers or produce exopolysaccharides that supposedly improve the soil structure.

With the help of the SBSS selection system disclosed in the present invention, we recovered soil bacteria that are able to propagate in the temperate zone, from the beginning of the spring growing season, at 4-8 ° C soil temperature with high efficiency, appropriate for the particular soil and characteristic in Central European soils, including those in Hungary, that are naturally occurring in the given soil flora therefore correspond to the current environment and are nature-identical. The selection system enables to obtain the strains with the most appropriate propagation capability and microbial activity from the acidic and / or alkaline and / or salty and / or sandy soils. According to the method, in a first step, by applying pH, salt and cold selection pressure, preferably broad pH and / or salt and / or cold tolerant strains may be obtained in a short time from hundreds of strain per soil sample. Using the most suitable selective microbiological culture media, the selection of non human pathogenic soil microbial strains is preferably performed as well.

The hundreds of strains with high growing ability enter the second phase of the SBSS system for testing for Plant Growth Promoting (PGP) effects, such as: the phosphate solubilizing ability provides nutrient supply and phosphorus to the plants, and / or the nitrogen-binding capacity provides increased amount of nitrogen available to the plant for uptake from the atmospheric nitrogen. The phosphate solubilizing ability of the strains is verified by hydroxy apatite dissolution zone assays, the nitrogen fixing ability is verified by classical acetylene reduction gas chromatography measurements. Detection of the plant growth promoting effect of the cultures screened for nitrogen fixation and / or phosphate solubilization was carried out by assaying for the indole-3 acetic acid hormone and for ACC deaminase-producing ability that inhibits ethylene synthesis using quick colorimetric assays. The siderophore-producing and / or nitrogen fixating and / or phosphate solubilizing and / or indole-3 acetic acid-producing strains that are useful for suppressing phytopathogenic microorganisms were obtained by assaying for the ability to decompose chromoazurol S. Strains capable of producing extracellular polysaccharides (EPS) that support soil formation and water retention and / or siderophore-producing and / or nitrogen fixating and / or phosphate solubilizing and / or indole-3 acetic acid producing were obtained on the basis of their fluorescence on Calcofluor containing medium. By obtaining strains capable of producing extracellular polysaccharides (EPS) that support the soil formation and water retention and / or siderophore-producing and / or nitrogen fixating and / or phosphate solubilizing and / or indole-3 acetic acid producing using the SBSS system disclosed in the present invention, the most suitable and most effective micro-organisms are available, which are strains growing at high cell number in soil types in the temperate zone at extreme soil pH - acidic and / or alkaline - and high salt (saline), with high pH and salt-tolerance, plant nutrition, growth promotion, as well for inhibiting soil plant-pathogenic microorganisms, and changing the structure of the soil to help water retention. The system can be extended later to obtain strains obtainable from soils contaminated by oil, chemicals, plastics, or other materials, which are capable to detoxify or tolerate the contamination in addition to the above listed soil fertility promoting and plant growth enhancing properties.

Knowing the strains, strain combinations and selection methods disclosed in the literature, there is a continued need to provide improved soil inoculant compositions that promote the growth of plants under extreme stress conditions. The mere fact that a number of similar solutions exist does not render obvious to provide further such strains or strain mixtures. The present invention provides exactly such novel and particularly preferred strains. Using the selection method disclosed, several new strains, in addition to the strains commonly occurring in the soil inoculant compositions, were identified that have very advantageous effects, either alone or as part of a mixture, due to their specific feature combinations. It should be emphasized that several strains of the invention has not yet been known to be useful as a component of soil inoculant composition. We have surprisingly been able to isolate and identify these strains by the selection method disclosed in the present invention.

The strain disclosed in the present invention is preferably a siderophore producing PGRP strain selected from the group consisting of *Arthrobacter crystallopoietes* S 153 (P) NCAIM B 001424, *Paenibacillus peoriae* S 284 NCAIM (P) B 001430, *Pseudomonas chlororaphis* K2009 13/4 B NCAIM (P) B 001370, *Pseudomonas chlororaphis* K2009 9/4-B NCAIM (P) B 001371, and *Agreia pratensis* S 47 NCAIM (P) B 001431.

According to the present invention, the term stress soil means acidic, saline, salty, and structure-less sandy soils. These include dystric and eutric arenosol, alisol, sodic solonchak, solonetz, dystric gleysol, dystric fluvisol.

As used herein, the term stress conditions means extreme cold tolerance, extreme salt tolerance and extreme pH tolerance.

As the final result of the selection method, the selected strains are identified as stress-tolerant strains suit-able to inoculate stress soils that are capable to grow under acidic, alkaline, saline, cold conditions.

In this respect, the term "capable to propagate" is meant that the given strain is capable to form a large number, at least more than one hundred, preferably several hundred colonies on modified Nutrient Agar medium (NA) plate under the given stress conditions (e.g., 1-8% NaCl salt concentration, or acidic (3-7) or alkaline (7-8) pH at 28 C, and neutral pH in cold (4-8 ° C)) after 168 hours of incubation.

The invention further discloses a ST strain which is further selected by appropriate biochemical assays as Plant Growth Promotion Rhizobacteriaceae (PGPR), preferably for the following properties: nitrogen binding, siderophore production, ACC deaminase production, phosphate mobilization, and indole-3 acetic acid (IAA) production.

The invention discloses an ST strain which is further selected by appropriate biochemical assays for Aggregate Promotion (AP), preferably for exopolysaccharide production.

The invention further discloses a nitrogen binding PGPR strain, preferably selected from the group consisting of *Azospirillum brasilense* NF 11 NCAIM (P) B 001428, *Azospirillum brasilense* 242/9 NCAIM (P) B 001403, *Azospirillum brasilense* NF 10 NCAIM (P) B 001427, *Azospirillum largimobile* B41 NCAIM (P) B 001402, *Azospirillum irakense* NF 6 NCAIM (P) B 001425, and *Azospirillum brasilense* NF 7 NCAIM B (P) 001433.

The invention further discloses a siderophore producing PGPR, preferably selected from the group consisting of *Arthrobacter crystallopoietes S 153* (P) NCAIM B 001424, *Paenibacillus peoriae S 284* NCAIM (P) B 001430, *Azospirillum largimobile* B41 NCAIM (P) B 001402, *Azospirillum irakense* NF6 NCAIM (P) B 001425, *Pseudomonas chlororaphis* K2009 13/4 B NCAIM (P) B 001370, *Pseudomonas chlororaphis* K2009 9/4-B NCAIM (P) B 001371, and *Agreia pratensis S 47* NCAIM (P) B 001431.

The invention further discloses a phosphate mobilizing PGPR strain, preferably selected from the group consisting of *Exiguobacterium acetylicum* LU44 NCAIM (P) B 001423, *Pseudomonas frederikbergensis S33* NCAIM (P) B 001429, *Pseudomonas jessenii S 125* NCAIM (P) B 001422, *Arthrobacter crystallopoietes S 153* (P), NCAIM B 001424, *Agreia pratensis S 47* NCAIM (P) B 001431, *Pseudomonas chlororaphis* K2009 13/4 B NCAIM (P) B 001370, and *Pseudomonas chlororaphis* K2009 9/4-B NCAIM (P) B 001371.

The invention further discloses an ACC deaminase producing PGPR strain, preferably selected from the group consisting *Pseudomonas chlororaphis* K2009 13/4 B NCAIM (P) B 001370, *Pseudomonas frederikbergensis S33* NCAIM (P) B 001429, *Azospirillum brasilense* NF 11 NCAIM (P) B 001428, *Azospirillum brasilense* NF 10 NCAIM (P) B 001427, *Azospirillum irakense* NF 6 NCAIM (P) B 001425, *Pseudomonas jessenii S 125* NCAIM (P) B 001422, *Exiguobacterium acetylicum* LU44 NCAIM (P) B 001423.

The invention further discloses an IAA producing PGPR strain, preferably selected from the group consisting of *Azospirillum brasilense* NF 11 NCAIM (P) B 001428, *Azospirillum brasilense* 242/9 NCAIM (P) B 001403, *Azospirillum brasilense* NF 10 NCAIM (P) B 001427, *Azospirillum largimobile* B41 NCAIM (P) B 001402, *Azospirillum brasilense* NF 7 NCAIM B (P) 001433 *Exiguobacterium acetylicum* LU44 NCAIM (P) B 001423, *Pseudomonas frederikbergensis S33* NCAIM (P) B 001429, *Pseudomonas jessenii S 125* NCAIM (P) B 001422, *Arthrobacter crystallopoietes S 153* (P) NCAIM B 001424, *Pseudomonas chlororaphis* K2009 13/4 B NCAIM (P) B 001370, *Agreia pratensis S 47* NCAIM (P) B 001431, *Kocuria rosea S 225* NCAIM (P) B 001426.

The invention further discloses a exopolysaccharide producing AP strain, preferably selected from the group consisting of *Bacillus simplex S 28* NCAIM (P) B 001432, *Kocuria rosea S 225* NCAIM (P) B, 001426, *Leuconostoc mesenteroides* K2009 25/4 NCAIM (P) B 001372, *Pseudomonas chlororaphis* K2009 13/4 B NCAIM (P) B 001370, *Pseudomonas chlororaphis* K2009 9/4-B NCAIM (P) B 001371.

The invention further discloses a nitrogen binding and IAA producing PGPR strain, preferably selected from the group consisting of *Azospirillum brasilense* NF 11 NCAIM (P) B 001428, *Azospirillum brasilense* 242/9 NCAIM (P) B 001403, *Azospirillum brasilense* NF 10 NCAIM (P) B 001427, *Azospirillum brasilense* NF 7 NCAIM B (P) 001433, *Azospirillum largimobile* B41 NCAIM (P) B 001402.

The invention further discloses a ST strain which is a persistent colonizing strain on highly acidic pH 4.2 - 4.3 soil, having perpetual propagation ability of at least 4 months at steady level, preferably selected from the group consisting of *Azospirillum largimobile* B41 NCAIM (P) B 001402, *Azospirillum brasilense NF 7 NCAIM B (P) 001433*

The invention further discloses a ST strain which produces cytokinin:trans-zeatin and trans-zeatin riboside like auxins and gibberelline-3 hormone, which is preferably *Azospirillum irakense* NF6 NCAIM (P) B 001425.

The invention further discloses a ST strain which is a broad pH tolerant strain that persistently propagates at pH 4.5 and 9.5, which is preferably selected from the group consisting of *Azospirillum largimobile* B41 NCAIM (P) B 001402.

The invention further discloses a ST strain which produces indole-3 acetic acid under alkaline conditions of pH 6.5 - 9.5, which is preferably *Kocuria rosea S 225* NCAIM (P) B 001426.

The invention further discloses a ST strain which inhibits the plant pathogenic fungus *Alternaria alternat*, *Fusarium graminearum* or *Fusarium moniliforme*, which is preferably selected from the group consisting of *Azospirillum largimobile* B41 NCAIM (P) B 001402, *Azospirillum irakense* NF6 NCAIM (P) B 001425, *Exiguobacterium acetylicum* LU44 NCAIM (P) B 001423, *Paenibacillus peoriae S 284* NCAIM (P) B 001430, *Agreia pratensis S 47* NCAIM (P) B 001431, *Arthrobacter crystallopoietes S 153* (P) NCAIM B 001424, *Pseudomonas jessenii S 125* NCAIM (P) B 001422.

Is disclosed a Gram negative or Gram positive PGRP and / or AP strain, which is non-pathogenic in humans and is further characterized for one of the following poroperties: xylose, lactose and / or sucrose negativity, hydrogen sulfide production, polymyxin resistance, mannitol negativity, lecithinase production, and / or lactose negativity, and which is selected from the group consisting of selected from the group consisting of *Lysinibacillus xylanilyticus*, *Bacillus megaterium*, *Bacillus pumilus*, *Brevibacterium frigoritolerans*, *Exiguobacterium acetylicum*, *Agreia pratensis*, *Pseudomonas jessenii*, *Pseudomonas chlororaphis Arthrobacter crystallopoietes*, *Kocuria rosea*, *Bacillus simplex*, *Paenibacillus peoriae*, *Pseudomonas frederikbergensis*, *Leuconostoc mesenteroides, Azospirillum lipoferum*, *Azospirillum irakense*, *Azospirillum largimobile, Azospirillum brasilense.*

Is disclosed a strain belonging to a species selected from the group consisting of *Exiguobacterium acetylicum*, *Agreia pratensis*, *Pseudomonas jessenii*, *Arthrobacter crystallopoietes*, *Bacillus simplex*, *Paenibacillus peoriae*, *Leuconostoc mesenteroides*, *Pseudomonas frederikbergensis*, *Pseudomonas chlororaphis*, and *Azospirillum largimobile.*

In another aspect, the invention provides a mixture of stress tolerant (ST) soil inoculant strains, wherein the strains tolerate the propagation of each other, colonize each other, or help each other, wherein the mixture is obtainable by the Colonization and Competition and Helper (CoCoHelp) method comprising the steps of:
a) examining an ST strain for
   - siderophore production,
   - capability for forming antibiosis against the Gram negative Pseudomonas fluorescens NCAIM B. 01102 and nitrogen binding Raoultella terrigena NCAIM B. 02149 and Gram positive Bacillus circulans NCAIM B. 01115 bacteria characteristically present in soils, and
   - exopolysaccharide production,
b) classifying the strains after the antobiosis studies into aggressive, resistant and sensitive groups,
c) testing the less aggressive, resistant and sensitive strains of step b) in paired inhibition assay;
d) identifying the strains that inhibit each other marginally or partially or help each other as suitable for use in the mixture.

In another aspect of the disclosure the strains most suitable for soil inoculation should be well propagating, only partially or marginally inhibitive against each other and less sensitive, or if they are sensitive, their respective helper pair should be present.

In a further embodiment, the invention provides a soil inoculant composition, comprising the above defined strain mixture.

In a further aspect, the disclosure discloses a soil inoculant composition, which comprises from the above strains or strain mixtures the following:
1- at least one nitrogen binding,
2- at least one siderophore producing,
3- at least one phosphate mobilizing,
4- at least one ACC deaminase producing,
5- at least one IAA producing,
6- at least one exopolysaccharide producing,
7- at least one plant pathogenic fungus inhibiting,
8- at least one persistent soil colonizing,
9- at least one cytokinin -zeatin and trans zeatin riboside producing,
10- at least one gibberellin producing,
11- at least one plant pathogenic fungus inhibiting and at least one strain with other activity,
12- at least one nitrogen binding and at least one IAA producing,
13- at least one nitrogen binding and at least one phosphate mobilizing,
14- at least one nitrogen binding and at least one exopolysaccharide producing,
15- at least one nitrogen binding and at least one plant pathogenic fungus inhibiting,
16- at least one nitrogen binding and at least one persistent soil colonizing,
17- at least one nitrogen binding and at least one cytokinin -zeatin and trans zeatin riboside producing,
18- at least one nitrogen binding and at least one gibberellin producing,
19- at least one nitrogen binding and at least one IAA producing and at least one exopolysaccharide producing,
20- at least one nitrogen binding and at least one IAA producing and at least one ACC deaminase producing,
21- at least one nitrogen binding and at least one IAA producing and at least one phosphate mobilizing,
22- at least one nitrogen binding and at least one IAA producing and at least one siderophore producing,
23- at least one nitrogen binding and at least one IAA producing and at least one plant pathogenic fungus inhibiting,
24- at least one nitrogen binding and at least one IAA producing and at least one persistent soil colonizing,
25- at least one nitrogen binding and at least one IAA producing and at least one cytokinin -zeatin and trans zeatin riboside producing,
26- at least one nitrogen binding and at least one IAA producing and at least one gibberellin producing,
27- at least one nitrogen binding and at least one IAA producing and at least one ACC deaminase producing and at least one exopolysaccharide producing,
28- at least one nitrogen binding and at least one IAA producing and at least one phosphate mobilizing and at least one exopolysaccharide producing,
29- at least one nitrogen binding and at least one IAA producing and at least one siderophore producing and at least one exopolysaccharide producing,
30- at least one nitrogen binding and at least one IAA producing and at least one plant pathogenic fungus inhibiting and at least one exopolysaccharide producing,
31- at least one nitrogen binding and at least one IAA producing and at least one persistent soil colonizing and at least one exopolysaccharide producing,
32- at least one nitrogen binding and at least one IAA producing and at least one cytokinin -zeatin and trans zeatin riboside producing and at least one exopolysaccharide producing,
33- at least one nitrogen binding and at least one IAA producing and at least one gibberellin producing and at least one exopolysaccharide producing,
34- at least one nitrogen binding and at least one IAA producing and at least one ACC deaminase producing and at least one exopolysaccharide producing and at least one phosphate mobilizing,
35- at least one nitrogen binding and at least one IAA producing and at least one plant pathogenic fungus inhibiting and at least one exopolysaccharide producing and at least one phosphate mobilizing,
36- at least one nitrogen binding and at least one IAA producing and at least one persistent soil colonizing and at least one exopolysaccharide producing and at least one phosphate mobilizing,
37- at least one nitrogen binding and at least one IAA producing and at least one cytokinin -zeatin and trans zeatin riboside producing and at least one exopolysaccharide producing and at least one phosphate mobilizing,
38- at least one nitrogen binding and at least one IAA producing and at least one gibberellin producing and at least one exopolysaccharide producing and at least one phosphate mobilizing,
   or
39- at least one nitrogen binding and at least one IAA producing and at least one ACC deaminase producing and at least one exopolysaccharide producing and at least one phosphate mobilizing and at least one siderophore producing,
40- at least one nitrogen binding and at least one IAA producing and at least one plant pathogenic fungus inhibiting and at least one exopolysaccharide producing and at least one phosphate mobilizing and at least one siderophore producing,
41- at least one nitrogen binding and at least one IAA producing and at least one persistent soil colonizing and at least one exopolysaccharide producing and at least one phosphate mobilizing and at least one siderophore producing,
42- at least one nitrogen binding and at least one IAA producing and at least one cytokinin -zeatin and trans zeatin riboside producing and at least one exopolysaccharide producing and at least one phosphate mobilizing and at least one siderophore producing,
43- at least one nitrogen binding and at least one IAA producing and at least one gibberellin producing and at least one exopolysaccharide producing and at least one phosphate mobilizing and at least one siderophore producing,

In an especially preferred embodiment, the invention provides a soil inoculant composition which is useful for inoculating a soil with neutral pH, and is a mixed culture suitable for soil inoculation of the following soil specific stress tolerant soil bacterial strains:
*Pseudomonas jessenii S 125* NCAIM (P) B 001422
*Arthrobacter crystallopoietes S 153* (P) NCAIM B 001424
*Kocuria rosea S 225* NCAIM (P) B 001426
*Azospirillum brasilense* NF 11 NCAIM (P) B 001428
*Azospirillum brasilense* NF 10 NCAIM (P) B 001427
*Azospirillum largimobile* B41 NCAIM (P) B 001402
*Azospirillum brasilense* 242/9 NCAIM (P) B 001403

In another especially preferred embodiment, the invention provides a soil inoculant composition which is useful for inoculating a soil with acidic pH, and is a mixed culture suitable for soil inoculation of the following
*Bacillus simplex S 28* NCAIM (P) B 001432
*Pseudomonas frederikbergensis S33* NCAIM (P) B 001429
*Agreia pratensis S 47* NCAIM (P) B 001431
*Azospirillum brasilense* NF 11 NCAIM (P) B 001428
*Exiguobacterium acetylicum* LU44 NCAIM (P) B 001423
*Azospirillum largimobile* B41 NCAIM (P) B 001402
*Paenibacillus peoriae S 284* NCAIM (P) B 001430

In a further especially preferred embodiment, the invention provides a soil inoculant composition which is useful for inoculating a soil with alkaline pH, and is a mixed culture suitable for soil inoculation of the following soil specific stress tolerant soil bacterial strains:
*Exiguobacterium acetylicum* LU44 NCAIM (P) B 001423
*Pseudomonas jessenii S 125* NCAIM (P) B 001422
*Arthrobacter crystallopoietes S153* (P) NCAIM B 001424
*Pseudomonas chlororaphis* K2009 13/4 B NCAIM (P) B 001370
*Azospirillum brasilense* 242/9 NCAIM (P) B 001403
*Azospirillum largimobile* B41 NCAIM (P) B 001402
*Azospirillum irakense* NF 6 NCAIM (P) B 001425

In further preferred embodiments, the invention provides the strain mixtures described in Claims 5 to 7.

The invention further provides the use of strain mixture or soil inoculant composition for applying in the early vegetative stages of corn, cereals, preferably barley, wheat, sunflower, peas, pepper, tomato, sugar beet, mustard, soy, rape. Further preferred embodiments include the use of a strain mixture or soil inoculant composition according to the invention for the following:
- in agriculture for increasing green mass and root mass and harvest yield;
- for enhancing microbial life and soil replenishing on deteriorated acidified or alkalized fields that are still suitable for agricultural production;
- for enhancing microbial life and soil replenishing of luvisol and solonchak and calcic solonchak, calcaric chernozem and chernozem type soils that are still suitable for agricultural production;
- under extreme pH, salt and cold soil conditions,
- stimulating germination, enhancing shooting, stimulating root growth, including formation of side roots,
- increasing leaf count and flower count and harvest yield, or
- helping soil life and soil water balance.

The present invention further discloses a method, which is the basis for the Stress tolerant Soil bacteria Sorting System (SBSS). The system is mainly suitable for the isolation of stress-tolerant bacteria capable of effective soil inoculation of soils with low fertility due to unfavorable soil pH and salt and water balance conditions (stress soils), for the classification of bacteria affecting plant growth and soil life (soil life health), and for the study of the interaction of the strains by microbial selection and colonization-competition (CoCoHelp). At the same time, surprisingly so-called helper bacteria or fungi may be obtained, which can support the growth of bacteria and mycorrhizal fungi according to the recent literature by their plant hormone production (indole-3 acetic acid, IAA), fermentation nutrients (organic acids) or by complex compounds known to participate in bacterial communication (quorum sensing).

During the SBSS soil microbiology research, 10 specific stress soil types were selected, such as dystric and eutric arenosol, alisol, sodic solonchak, solonetz, dystric gleysol, dystric fluvisol, and these were sampled over different areas of Hungary. More than a thousand bacterial strains were isolated from the collected soils that are capable to propagate in stress soils and characteristic to the given soils. The isolated strains are maintained and a soil microorganism SBSS strain collection was created. PGPR and EPS (exopolysaccharide) producing strains were selected from the strain collection by using stress parameters (pH and salt) for selection, with excluding human pathogenic strains by suitable microbial selection on the one hand, and on the other hand with assaying for plant growth stimulation (PGPR) and soil aggregate formation (AP). At the same time, stress tolerant nitrogen fixing strains were obtained from the stress soils and their nitrogen fixing, plant hormone (IAA) producing, and / or ACC deaminase-producing and / or phosphate mobilizing abilities were measured. From the large number of more than seven hundred studied strains, about 92 potentially suitable test strains were selected. After DNA sequencing and identification, a colonization-competition (CoCoHelp) methodology was developed for the selection of appropriate combinations of soil inoculating bacteria, where the aggressively growing or sensitive and, surprisingly, so called helper bacterial strains suitable for inoculant have been selected, thus setting up the appropriate soil fermenting strain compositions having supplementary and additive PGPR, AP effects. The selected soil inoculant compositions were first tested soil pots then in field trials by inoculating the soil of vegetable plants, monocotyledonous cereal, maize and dicot sunflower, as well as on soya crops, and the effect of the prototype inoculants on the plants was examined. The plant experiments were conducted on different characteristically specific agricultural stress soils. Several hundreds of soil samples were taken from the inoculated and non- inoculated stress soils, and population genetic tests were used to confirm the propagation capability, presence and survival of some selected stress tolerant soil inoculant strains of the stress soils. In addition, vitro proliferation dynamic studies were also performed in stress soils with culturing method and demonstrated the survival of our nitrogen fixing strains. An industrial scale fermentation technology was developed for the fermentation of the bacterial strains required for the preparation of the prototype soil inoculant compositions.

As the result of the use of SBSS, strains uniquely suited for the inoculation of stress soils may be obtained - capable to propagate under acidic, alkaline, saline, soda, cold environmental conditions - that are characteristic to the given stress soil - Stress Tolerant Bactericeae (STB) - which have plant growth-promoting properties, nitrogen fixation, phosphate mobilization, siderophore, ACC deaminase production - summarized as PGPR - and polysaccharide production (exopolysaccharide producing EPS) - thus soil aggregate promoting - supporting (Aggregate Promotion, AP), in summary Stress Tolerant, Plant Growth and Aggregate Formation Promoting Bacteria (Stress Tolerant - Plant Growth Promotion Rhizobacteriaceae and Aggregate Promotion Bacteriaceae) ST PGPR-AP-B strains, or in short STPAP strains, from which competition-colonization (CoCoHelp) strains can be recovered.

One group of the selected strains has the particular significance to be capable of inhibiting the growth of plant pathogenic fungi. The pathogenic fungi tested for inhibition include economically important species such as *Alternaria alternata*, *Fusarium graminearum* or *Fusarium moniliforme.*

The stress tolerant plant stimulating and aggregate promoting, helper and cooperative, tolerant STPAP-CoCoHelp strains produced by the SBSS method are called SBSS bacterial strains.

The SBSS system developed during the research helped to isolated novel bacteria not disclosed in the art of development of soil inoculant compositions that were more effective than the state of the art ones and were capable to propagate in stress soils, such as: phosphate mobilizing and / or antifungal siderophores and / or bacteria producing plant hormones (IAA) and exopolysaccharides that possibly promote the formation soil aggregates. In particular, it should be emphasized that some of these strains were not used in soil inoculant compositions, such as the *Exiguobacterium acetylicum, Pseudomonas frederiksbergensis, Pseudomonas jessenii* phosphate mobilizing and IAA producing, *Arthtrobacter crystallopoietes* or *Agreia pratensis* phosphate mobilizing and siderophore and IAA producing, *Leuconostoc mesenteroides* or *Bacillus simplex* polysaccharide producing or *Kocuria rosea* polysaccharide and IAA producing, *Pseudomonas chlororaphis* polysaccharide and siderophore and IAA producing, and *Paenibacillus peoriae* polysaccharide and siderophore producing microorganisms. The inoculant produced during the development also includes nitrogen fixing soil bacteria with special properties, that are more effective or has not been used in soil inoculant compositions: such as the cold-tolerant and salt-tolerant and acidic pH tolerant effective nitrogen binding and plant hormone producing (IAA) *Azospirillum brasilense* strain, as well as cold-tolerant and salt-tolerant effective nitrogen fixating and / or plant hormone (IAA) producing *Azospirillum irakense* and *Azospirillum largimobile* strains.

These strains were subjected to colonization-competition-helper (HTB) tests, where the right strains that help each other, are not mutually inhibitory, but having good colonization abilities were selected, which are suitable for soil inoculant composition.

From these SBSS strains, soil inoculant compositions were composed according to the pH of the stress soils, and acidic, neutral and alkaline soil inoculant prototypes have been developed for supporting the growth and soil life, as well as helping the soil water balance of the different field and greenhouse crops. These soil inoculant compositions, according to small lot field studies, are able to exert their activity at extreme pH, salt and cold conditions, can effectively stimulate the germination, improve shooting, strongly stimulate root growth-lateral root formation, and at the same time are soil inoculant compositions with profound effect on nitrogen fixation, phosphate and potassium mobilization (chemical fertilizer usage reduction), increase of the number of leaves and flower numbers and yield enhancement and / or specifically producing biocontrol - siderophore type-compounds, inhibiting the root pathogenic bacteria and fungi (reduction of the usage of herbicides), and improve the soil micro-structure.

The following SBSS soil bacterial isolates forming a part of the present invention were deposited at the Corvinus University of Budapest, in the National Collection of Agricultural and Industrial Microorganisms (1118, Budapest, Somlói út 14-16) on October 2, 2009:
***Pseudomonas chlororaphis*** K2009 13/4 B NCAIM (P) B 001370 - surprisingly propagates under cold conditions (4-8°C) and exopolysaccharide producing, potentially promoting soil aggregation and soil amelioration. Capable of phosphate solubilization and siderophore production. Surprisingly all metabolic functions are sustained even in cold climatic conditions.
***Pseudomonas chlororaphis*** K2009 9/4-B NCAIM (P) B 001371 - surprisingly cold propagates under (4-8°C) conditions and exopolysaccharide producing, potentially promoting soil aggregation and soil amelioration. Capable of phosphate solubilization and siderophore production. Surprisingly all metabolic functions are sustained even in cold climatic conditions.
***Leuconostoc mesenteroides*** K2009 25/4 NCAIM (P) B 001372 - surprisingly exopolysaccharide producing, thus potentially promoting soil aggregation and amelioration. At the same time, produces IAA as well, therefore promotes plant growth. Leuconostoc strains have not been used in soil inoculants, yet. The Leuconostoc lactic acid bacteria are highly prevalent in soils and are mainly used in the food conservation due to lactic acid production. The indole-3 acetic acid producing property is known for long, however it has not been exploited for plant growth promotion. We have observed that in most soil types - even in arenosol (sandy soil)-it sustains viability and is able to grow at high temperatures (30-35°C) and promotes the growth of plant vegetation via IAA production. Leuconostoc strains were already known as exopolysaccharide (EPS) - dextran - producing, however it has not been exploited in soil amelioration. According to the present invention, EPS production can limit heat-related water loss from soil due to the aggregation of soil particles in the drying soil. Further, the EPS matrix provides substance for other bacteria - biofilm formation - which promotes the viability of the soil microflora. These are favorable properties in the inoculation of desert soils, in supporting the vegetation, maintaining the soil matrix and water storing capacity.

Further, the SBSS soil bacterial isolates characterized below and forming a part of the present invention were deposited at the Corvinus University of Budapest, in the National Collection of Agricultural and Industrial Microorganisms (1118, Budapest, Somlói út 14-16) on November 27, 2013:
***Azospirillum brasilense*** NF 11 NCAIM (P) B 001428 - surprisingly propagates under weak acidic and alkaline conditions at pH 6.5-8.5, nitrogen-fixing, IAA producing strain.
***Azospirillum brasilense*** 242/9 NCAIM (P) B 001403 - surprisingly propagates under neutral and alkaline conditions at pH 7.0-8.5, nitrogen-fixing, IAA producing strain.
***Azospirillum brasilense*** NF 10 NCAIM (P) B 001427 - surprisingly tolerates cold selection (10°C) and salt-tolerant (4% NaCl), nitrogen-fixing, IAA producing strain, and propagates under neutral and alkaline conditions at pH 7.0-8.0. All metabolic functions are sustained in cold climate (10°C) and salt-accumulated (4%) stress soil conditions.
***Azospirillum largimobile*** B41 NCAIM (P) B 001402 - in contrast to other *Azospirillum* species, it is persistent in acidic soils. Inhibits the growth of certain plant pathogen fungi. Nitrogen-fixing, IAA producing strain which propagates under a wide range of pH at 4.5-9.5. *Azospirillum* spp. - mainly *A. brasilense* and *A. lipoferum* - have already been used for soil inoculation. Applications WO 2014160827 A1 and WO 2014193746 A1 describe *A. lipoferum* for soil inoculation, however, it was not disclosed to have fungicide activity or being persistent in acidic soil. It propagates in wide range of pH - from strongly acidic pH 4.5 to strongly alkaline pH 9.5 -, which is a quality that makes it useful in PGPR and nitrogen-fixing inoculants for both acidic and alkaline soil types. Further, its siderophore producing ability has not been described previously.
***Azospirillum irakense*** NF 6 NCAIM (P) B 001425 - surprisingly cold-tolerant (10°C) and salt-tolerant (4% NaCl), nitrogen-fixing, IAA producing strain that propagates under neural and weak alkaline conditions at pH 7.0-8.0. All of these metabolic functions are sustained in cold climate (10°C) and salt-accumulated (4%) soil conditions. Capable of producing siderophores, indole acetic acid, trans-zeatin and trans-zeatin riboside-like cytokinins and gibberellin-3 acid. Several publications have reported on the ferroxamine type siderophore decomposing ability of *A. irakense* (Winkelman et al.), however, siderophore production has not been described in the species. Application WO/1999/009834 A2 disclose *A. irakense* to be used as a soil inoculant but not on its siderophore producing property. In the US20050060930 and EP2233458 patents, A. irakense are also not associated with siderophore production. Several documents (WO/2014/079754, United States Patent Application 20150011389, WO/2014/147528, WO/2015/011615, WO/2014/053401) report on biopesticides that contain *A*. *irakense* and are effective against *S*. *sclerotiorum,* but it is not disclosed that *A. irakense* would be responsible for the fungicide effect. *A. brasilense* strains are capable of indole acetic acid, cytokinin and gibberellin production, and the cytokinin and gibberellin producing capacity of *A. irakense* have not been published yet. Arkhipova et al. (2006) have studied the effect of cytokinin-producing bacterial strains - e.g. *Azospirillum brasilense* - on the growth of wheat. Besides IAA, at least three different cytokinin-type substances were detected in a concentration equivalent to approx. 0.001 microgram/ml kinetin. The WO/1999/009834, United States Patent Application 20050060930, WO/2014/079754, United States Patent Application 20150011389 and WO/2013/110591 report on *A. irakense* as a soil inoculant but do not mention cytokinin and gibberellin-type plant hormone-like substance production. Plant growth and plant health promoting properties of *A. irakense* inoculants lie on the nitrogen-fixing, indole acetic acid and gibberellin producing capacity that act as phytohormones and the iron-binding siderophore producing ability that inhibits the growth of pathogen microbes and improves nutrient supply of plants.
***Exiguobacterium acetylicum*** LU44 NCAIM (P) B 001423 - surprisingly capable of substantial propagation under cold conditions (4-8°C), and surprisingly highly salt tolerant (8%NaCl) and further surprisingly phosphate solubilizing, which provides P-enrichment of soil for plant supply. Surprisingly produces indole acetic acid which promotes and supports plant growth. The strain surprisingly has a wide pH tolerance, it propagates in the range of pH 4-8. It surprisingly acts in synergy with, as a helper to *Azospirillum* strains. All of these metabolic functions are sustained in cold climatic conditions (4-10°C), and in soils with extreme pH and salt concentrations. *Exiguobacterium* strains have not been used as soil inoculants previously.
***Paenibacillus peoriae** S 284* NCAIM (P) B 001430 - surprisingly produces siderophores therefore has biocontrol effect to inhibit the growth of pathogen microbes. Surprisingly produces exopolysaccharides potentially promoting soil aggregation and amelioration. Surprisingly capable of growth at low pH (pH 4.0-6.0). All of these properties are exhibited also in strongly acidic stress soil types due to its propagation capability at extreme low pH. Surprisingly *Paenibacillus peoriae* strains have not been used as soil inoculants previously. EPS and siderophore producing Gram positive Bacillus strains are known, but PGPR *Paenobacillus peoriae* strains have not been isolated from soils, yet. The spore-forming ability sustains viability in the soil, therefore it is particularly advantageous as a soil inoculating strain. EPS production can limit water loss from soil due to the aggregation of soil particles. The EPS matrix provides substance for other bacteria - biofilm formation - which promotes the viability of the soil microflora. Further, the iron-binding siderophore producing ability inhibits the growth of pathogen microbes and improves nutrient supply of plants. Several studies report on the biocontrol effect of *P. peoriae* against a wide range of phytopathogenic bacteria and fungi (Weid et al., 20023, Weid et al., 2005) but no inhibitory effects is disclosed on *Alternaria alternata, Botrytis cinerea, Sclerotinia sclerotiorum, Fusarium graminearum* and *F. culmorum.* Several patented biopesticides are based on the biofungicide effect of *Paenibacillus* spp. - *P. alveii, P. popilliae, P. polymyxa* - but *P. peoriae* is not includes. Therefore the strain is especially suitable as a component of an inoculant aimed at plant health promotion and soil amelioration.
***Agreia pratensis** S 47* NCAIM (P) B 001431 - surprisingly capable of propagating under cold conditions (4-8°C) and surprisingly phosphate solubilizing, which provides P-enrichment of soil for plant supply. Surprisingly produces indole acetic acid which promotes and supports plant growth. Surprisingly exerts biocontrol effects due to siderophore production that inhibits the growth of pathogen micro-organisms. Surprisingly the strain has a wide pH tolerance it is capable of propagating at the range of pH 4.0-8.5). All of these metabolic functions are sustained in cold climates (4-10°C) and extreme soil pH conditions. Surprisingly *Agreia pratensis* strains have not been used as for soil inoculation previously. *A. pratensis* has not been studied in-depth for plant growth promoting and mineral mobilizing properties. Indole acetic and acid phosphate solubilizing properties have not been described previously. Phosphate solubilizing and IAA producing *A. pratensis* strains have not been previously isolated from soil. It acts as a fungicide against *A. alternata* and *F. moniliforme.* The United States Patent Application 20140004090 A1 lists *Agreia pratensis* among antimicrobial bacteria active against human and bee larvae pathogens, but does not report on a fungicide effect against plant pathogen fungi, especially *F. graminearum* and *A*. *alternata.* These properties make it especially useful for nutrient mobilization in soil, plant growth and plant health promotion.
***Arthrobacter crystallopoietes** S 153* (P) NCAIM B 001424 - surprisingly phosphate solubilizing, promotes P-enrichment of soil for plant supply. Surprisingly produces plant growth promoting indole acetic acid. Surprisingly produces siderophores which inhibit the growth of plant pathogen microbes (biocontrolling effect). It propagates under neutral and alkaline conditions (pH 6-9) therefore it tolerates alkaline soils well. Surprisingly *Arthrobacter* strains have not been used as soil inoculants previously. Phosphate-solubilizing and IAA-producing *A. crystallopoietes* strains have not been isolated from soil previously. These two properties make the strain especially suitable for mobilizing soil nutrients and promoting the growth of plants and therefore the use in soil inoculation. The United States Patent 6318023 patent discloses the application of *A. crystallopoietes* for plant growth promotion and yield enhancement, but the mode of action is not described. The indole acetic acid production capacity is similar to that of a*zospirilli,* with 25-30 microgram/ml broth. Via the synthesis of iron-binding siderophores, the strain can not only limit the growth of pathogens but improve the iron provision of vegetation, contributing to plant growth promotion. The United States Patent 6318023 reports on applying *A*. *crystallopoietes* for plant growth promotion purposes but does not disclose the fungicide effect of the strain against plant pathogen fungi. The US Patent 5696094 investigates the effect of lignosulphonate on Arthobacter strains isolated from the soil and reports on the nematicide effect of *Arthrobacter citreus, A. globiformis,* and *A. crystallopoietes* strains but does not suggest the fungicidal effect against plant pathogens, especially *Botrytis cinerea* and *F. culmorum.* The strains sustains viability in conditions of draught (Boylen, 1972) and is therefore especially suitable for plant growth promoting and soil ameliorating inoculation purposes.
***Pseudomonas frederiksbergensis*** S33 NCAIM (P) B 001429 - surprisingly capable of substantial propagation under cold conditions (4-8°C) and surprisingly salt tolerant (8%). Surprisingly produces IAA therefore promotes and supports plant growth. Surprisingly has a wide pH tolerance and capable of propagating in range of pH 4-8. Capable of phosphate solubilization. All of these metabolic functions are sustained at cold climatic (4-10°C) and varying - acidic or alkaline - soil pH conditions. *Pseudomonas* spp. have been long known as PGPR and phosphate solubilizing species, several strains of Pseudomonas species are known as PGPR and nutrient mobilizing strains. However, Surprisingly the use of PGPR and mineral solubilizing *Pseudomonas frederiksbergensis* strains have not been disclosed yet, as they have not been detected and isolated from soil and thus have not been applied as soil inoculants. Therefore the use of strains of these species is novel.
***Pseudomonas jessenii** S 125* NCAIM (P) B 001422 - surprisingly capable of substantial propagation under cold conditions (4-8°C) and Surprisingly salt tolerant (8%) strain. It surprisingly produces IAA therefore promotes and supports plant growth. Surprisingly has a wide pH tolerance and capable of propagating in range of pH 4.0-8.5. Capable of phosphate solubilization. All of these metabolic functions are sustained at cold climatic (4-10°C) and varying - acidic or alkaline - soil pH conditions. Surprisingly *Pseudomonas* spp. have not been suggested as soil inoculants. Several strains of Pseudomonas species are known as PGPR and nutrient mobilizing strains. PGPR and mineral solubilizing *Pseudomonas jesseni* strains have not been detected and isolated from soil and thus have not been applied as soil inoculants. The S125 strain inhibits the growth of *Sclerotinia sclerotiorum, Alternaria alternata, Fusarium graminearum, F. moniliforme* and *F. culmorum.* The US Patent Application 20150030577 A1 and WO/2013/130680A1 patents describe *P. jessenii* as a phytopathogenic bacterium, and identifies its active substance, but does not report on fungicidal effect against *Sclerotinia sclerotiorum, Alternaria alternata, Fusarium graminearum, F. moniliforme* and *F. culmorum.* The WIPO Patent Application WO/2014/009402 A1 discloses increased plant resistance against *P. jessenii*, but does not mention the biofungicide effect of the species. Keller Costa et al. (2014) reports on the fungicide effect of *P. jesseni* against *F. moniliforme.* Schreiter et al. (2014) and Guinazu et al. (2013) *P. jessenii* have published their findings on the biocontrol effect of the strain against *Rhizoctonia solani.* Weiss et al. (2007) have studied the bacteriocin effect of *P. jessenii* against potentially plant pathogen bacteria originating from vegetables but not against plant pathogen fungi. based on these, it can be stated that the fungicide effect of *P. jessenii* against *Sclerotinia sclerotiorum, Alternaria alternata, Fusarium graminearum* and *F. culmorum* has not been known until now. The use of the strain as a soil inoculant - based on its the recently described properties - is a novel.
***Bacillus simplex** S 28* NCAIM (P) B 001432 - surprisingly produces exopolysaccharides that potentially promote soil aggregation and soil amelioration. It has surprisingly wide pH tolerance, it propagates in the range of pH 4.0-8.5. All of these metabolic functions are sustained at varying - acidic or alkaline - soil pH conditions. *Bacillus simplex* strains have not been used as soil inoculants previously. EPS and siderophore producing Gram positive Bacillus strains are recognized but PGPR *Bacillus simplex* strains have not been isolated from soils, yet. The spore-forming ability sustains long-term viability in the soil therefore it is particularly effective as soil inoculant. EPS production can limit water loss from soil due to the aggregation of soil particles. The EPS matrix provides substance for other bacteria - biofilm formation - which promotes the viability of the soil microflora. The strain is suitable as (a component of) an inoculant aimed at soil amelioration and soil water storage capacity improvement.
***Kocuria rosea*** S 225 NCAIM (P) B 001426 - surprisingly produces exopolysaccharides that potentially promote soil aggregation and amelioration. It is surprisingly produces IAA, therefore promoting and helps the growth of plants. PGPR *Kocuria rosea* strains are already used as soil inoculants. The kocuran polysaccharide produced by Kocuria strains has been described as an antioxidant and an immunostimulant in human medicine. EPS can also promote the aggregation of soil particles preventing the rapid loss of water from destructured soils. The formed EPS adsorbs and promotes substance for other bacteria - biofilm formation - promoting the viability of the soil microflora. Further, the strain is capable of propagation and IAA production at strongly alkaline soil pH, thus contrary to the thus far known K*ocuria rosea* strains, its wide pH tolerance and plant hormone producing ability and EPS production (soil structure improvement) makes the strain especially suitable to be used for soil inoculation.
***Azospirillum brasilense*** NF 7 (deposited on 10 Feb 2014 under No. NCAIM (P) B 001433) - surprisingly cold (4-8°C) and salt tolerant (4% NaCl). It is capable of nitrogen fixing and substantial IAA production. It is surprisingly capable of propagating at highly acidic soil pH (pH 5.5-6.5) in addition of neutral conditions. Sustains viability and propagation in clayey acidic soil (pH_{KCl} 4,2) for more than one month. All of these metabolic functions are maintained at cold climatic (4-10°C) or acidic conditions (pH 4-5) and in the presence of high salt concentration (4%).

The invention is further described in the examples below, referring to the following figures. It is to be understood that the examples disclosed in no way limit the invention, which is only defined by the scope of the attached claims.

### Description of the Drawings

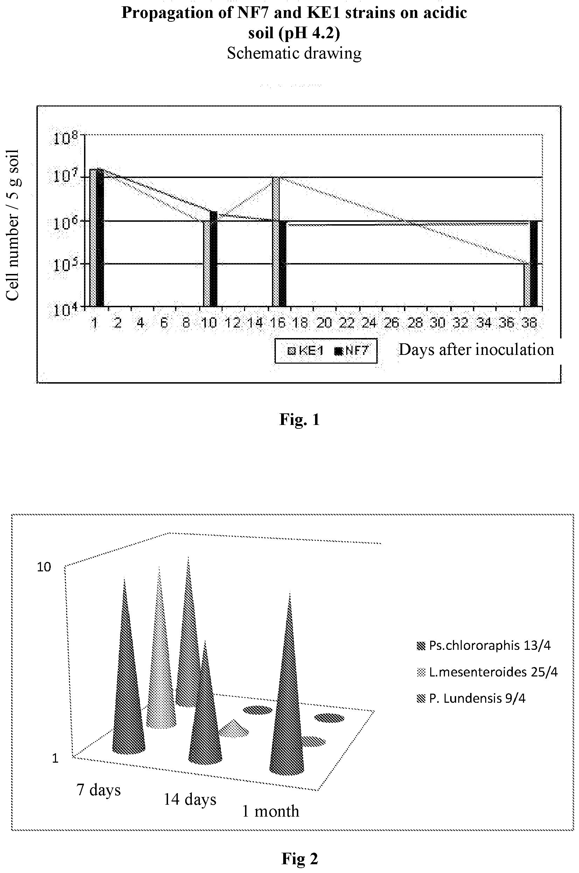

This application contains at least one figure executed in color. Copies of this application with color drawing(s) will be provided upon request and payment of the necessary fee. A brief summary of each of the figures is provided below.
∗∗∗
Figure 1. Growth of strains NF7 and KE1 in acidic soil (ph=4.2). Strain NF7 maintains a cell count of 10⁶ CFU/g of soil concentration until Day38, while the cell count of KE1 reference strain drops to 10⁵ CFU/g of soil.
Figure 2. Viability of exopolysaccharide producing strains in sandy potting soil from Dubai. Strain *Ps. chlororaphis* 13/4 B was found in a concentration of 10⁸ CFU/g of soil after two weeks, and after one month, while the other to strains are present only at a low cell count.

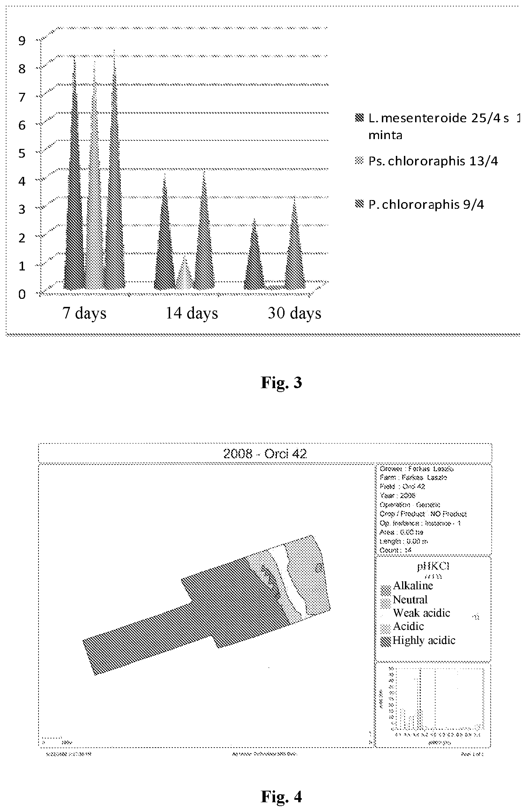

Figure 3. Viability of exopolysaccharide producing strains in desert sand from Dubai. *Ps. chlororaphis* 9/4 B and L. mesenteriodes 25/4 sustain viability and grow in a detectable concentration after two weeks and even one month.
Figure 4. Orczy 42 (Zimány) field plot with mostly acidic soil designated for TRFLP studies. The pH map of the plot reveals more than 50% percent of the area is classified as strongly acidic (red, ph 4.1-5.0), lower proportions are acidic (brown, pH 5.1-5.5), weakly acidic (yellow, pH 5.6-6.5) or neutral (green, pH 6.6-7.5).

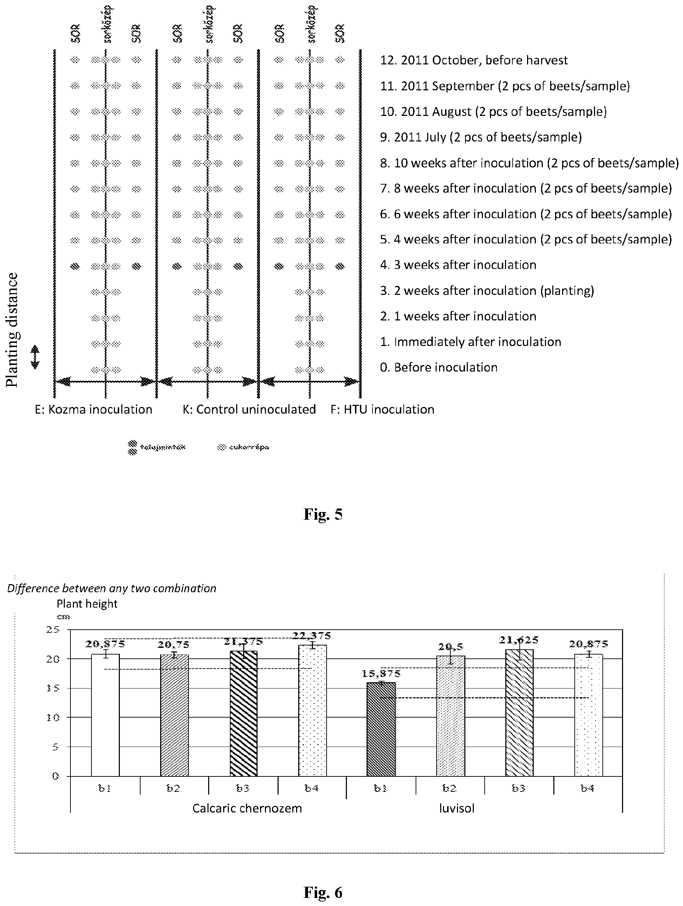

Figure 5. Field sampling scheme from an inoculated plot for population genetics studies of soil inoculant strains. According to the described protocol, sampling times were scheduled as follows: once prior to and once immediately after soil inoculation, once a week in the first four weeks, then once in every two weeks for the following six weeks. Samples were taken from the soil and the roots from both inoculated and uninoculated control plots. At the time of the first four samplings, samples are taken from three different sites of the seeding row, from the third week after inoculation samples are also taken from the inter-row spaces. From the fourth week after inoculation, root samples are also taken from the cultivated species (sugar beet).
Figure 6. The effect of a soil-specific neutral soil inoculant on the height of white mustard in brown forest soil. The neutral soil inoculant has significantly increased (P=0.01) average stem height of white mustard in both applied doses (11/ha and 2 1/ha) (see b3 and b4). On calcareous chemozem, the same doses have increased stem height by 2.5% and 7%, respectively.

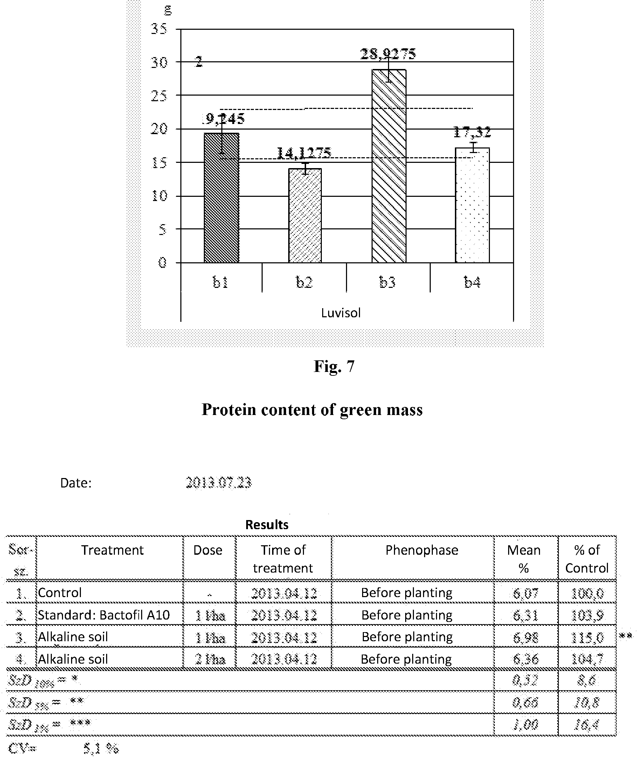

Figure 7. The effect of a soil-specific neutral soil inoculant on the shoot mass of white mustard in brown forest soil. The lower dose (b3, 1 1/ha) has significantly (p=0.01) increased the shoot mass of white mustard, compared to untreated control.

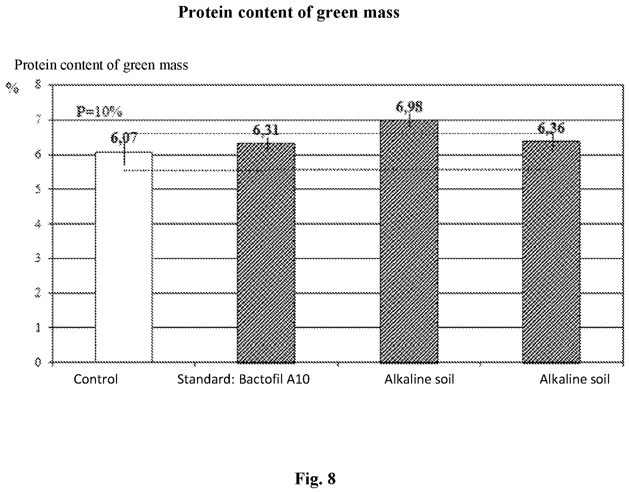

Figure 8. The effect of an alkaline soil inoculant preparation on the protein content of the shoot mass of spring barley on calcic solonchak soil. The figure displays the difference between the protein content of spring barley in uninoculated control fields (6.07%) and that of inoculated with an alkaline soil inoculants in a dosage of 1 1/ha and 2 1/ha (6.98% and 6.36% protein content, respectively) or inoculated with positive control Bactofil A10, in a dosage of 11/ha (6.31%).

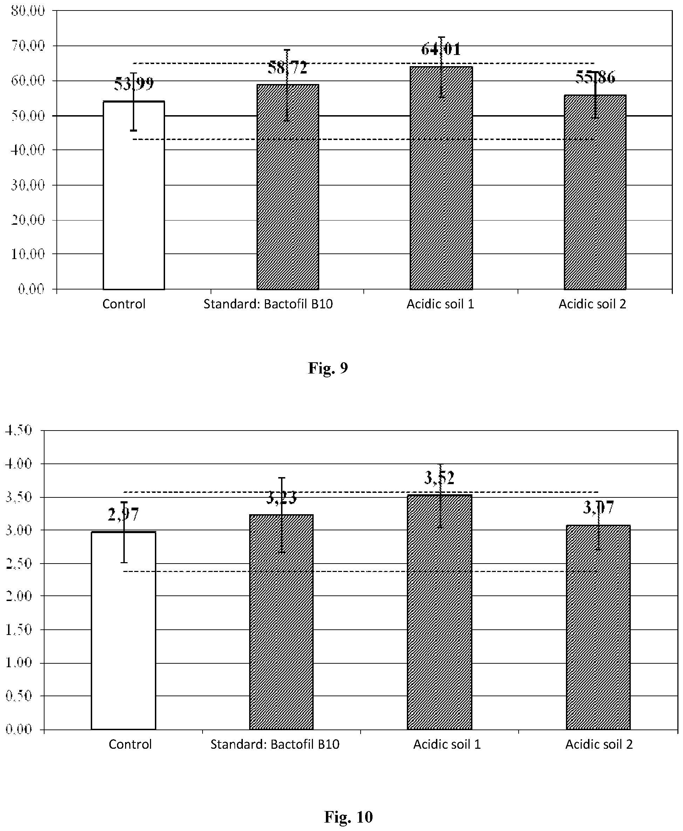

Figure 9. Comparison of sunflower head weights in respect of different treatments. The highest average head mass (64.01 t/ha) was achieved by 1 1/ha dose of the acidic soil inoculant, highly exceeding that of a 21/ha dose (55.86 t/ha) and positive control Bactofil B10 (58.72 t/ha). All three inoculation protocols have significantly increased crop yield compared to untreated control (53.99 t/ha).
Figure 10. Comparison of sunflower crop yields in respect of different treatments. The highest average yield of sunflower crop (64.01 t/ha) was achieved by 1 1/ha dose of the acidic soil inoculant, highly exceeding that of the 2 1/ha dose (55.86 t/ha) and the positive control Bactofil B10 (58.72 t/ha). All three inoculation protocols have significantly increased crop yield compared to untreated control (53.99 t/ha).

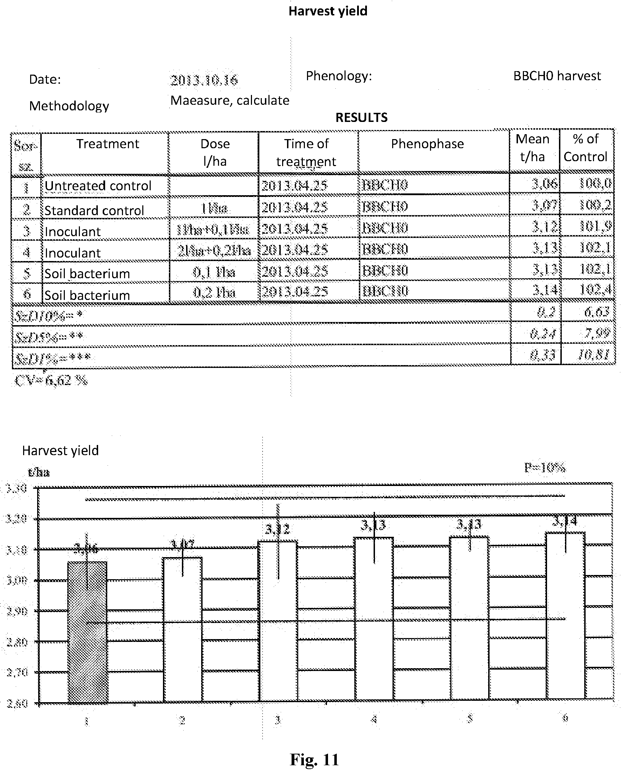

Figure 11. Soy crop yield on strongly acidic soil. Both the 1 1/ha (column 3) and 2 1/ha (column 4) dose of the soil inoculant for acidic soil, and also the 0.11/ha (column 5) and 0.2 1/ha dose of the soy-specific soil inoculant have significantly increased crop yield compared to control (column 1) and standard control (column 2) results, by an average 3.12-3.14 t/ha

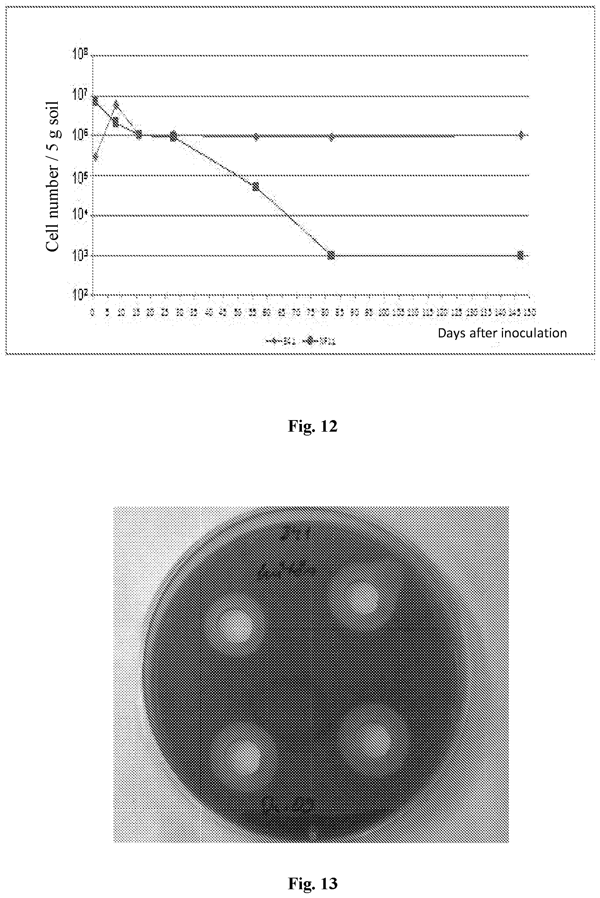

Figure 12. Cell count of strains B41 and NF11 in acidic soil in a period of 147 days following inoculation. Strain B41 is persistent in acidic soil, displaying a steady-paced proliferation in the first 8 days after inoculation, then, after a slight decrease, maintains a 10⁶ CFU/5g of soil plateau for more than 3 months. Strain NF11 prefers a neutral soil pH, as shown by a continuous decrease in cell number from the day of inoculation reaching the level of detection 10³ CFU/5 g of soil.
Figure 13. Detection of siderophore production of *A. largimobile* B41 on King B medium containing Chrome Azurol S. After 48h, a positive color reaction indicates the siderophore synthesis in the cultures inoculated in the agar holes.

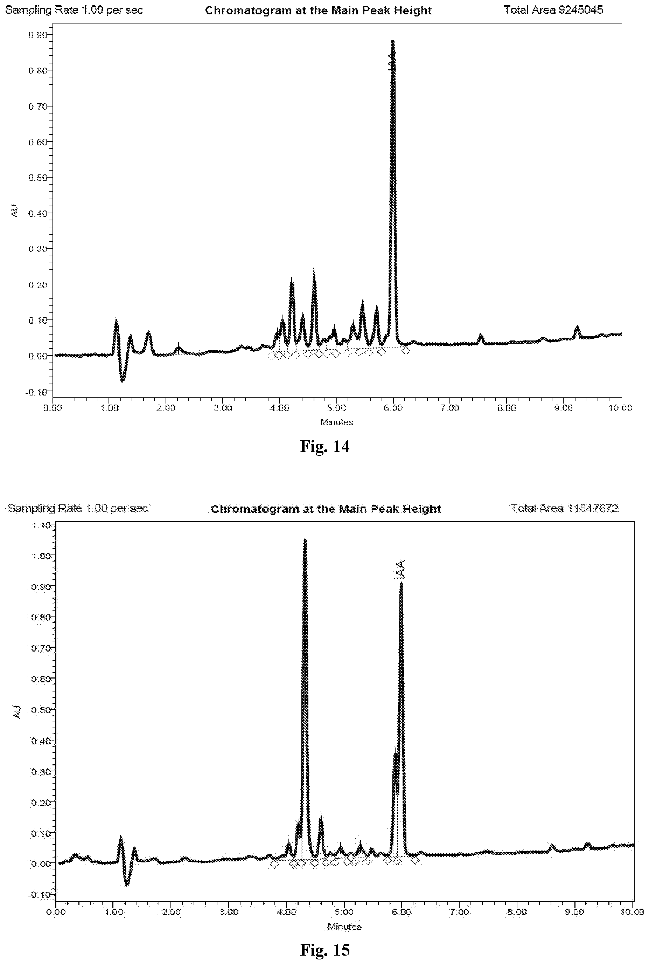

Figure 14. The chromatogram of *Arthrobacter crystallopoietes* S153. The ethanol extract of *A. crystallopoietes* S153 cultures contain 25.3 µg/ml indole acetic acid according to the relevant retention time.
Figure 15. The chromatogram of *Azospirillum brasilense* 242/9. The ethanol extract of *A. brasilense* 242/9 cultures contain according to the retention time 24.4 µg/ml indole acetic acid.

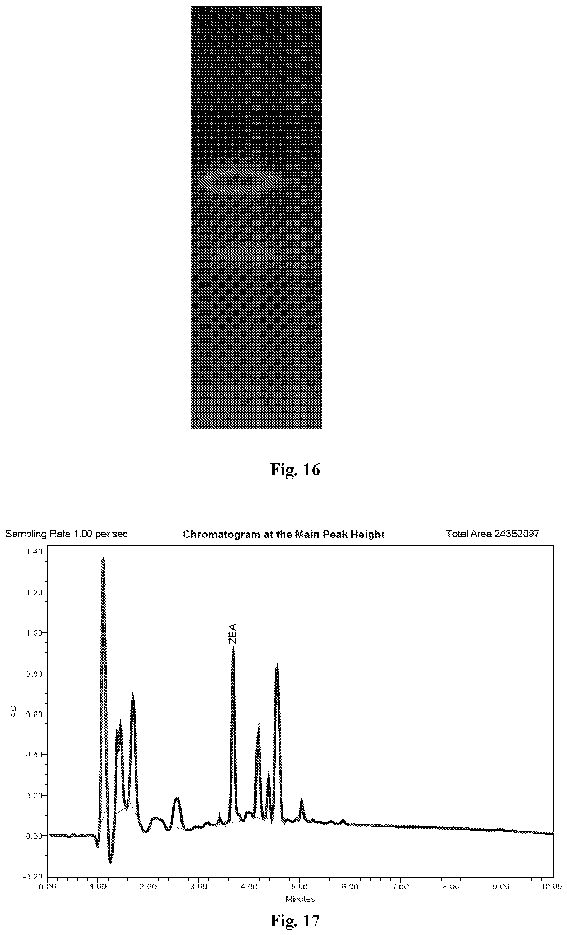

Figure 16. The chromatogram of *Azospirillum irakense* NF6 **NCAIM** (P) B **001425**. The extract of the cultures contain ghiberrellin-3 acid (retention factor: 0.3) and indole acetic acid (retention factor: 0.55).
Figure 17. The HPLC chromatogram of *Azospirillum irakense* NF6. Cytokinine-type substances equivalent to Trans-zeatine and Trans-zeatine-riboside chromophores, according to the relevant retention times.

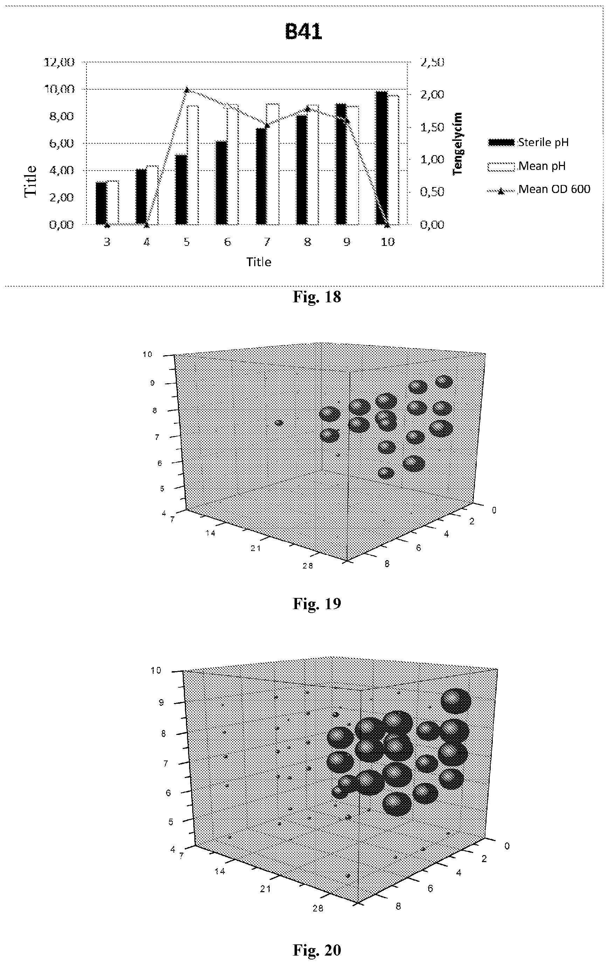

Figure 18. The growth of *Azospirillum largimobile* B41 NCAIM (P) B 001402 between ph 4.5 and 9.5.
Figure 19. Indole acetic acid production of *Kocuria rosea* ***S 225* NCAIM** (P) B 001426 at different salt concentrations and pH values. The strain produces indole acetic acid in a concentration of 10-20 µg/ml concentration in the pH interval of 6.5-9.0.
Figure 20. The growth of *Kocuria rosea* ***S 225* NCAIM** (P) B 001426 at different salt concentrations and pH values. The average cell count of 24h cultures in the ph interval of 6.5-9.0 is 10⁸-10⁹ CFU/ml.

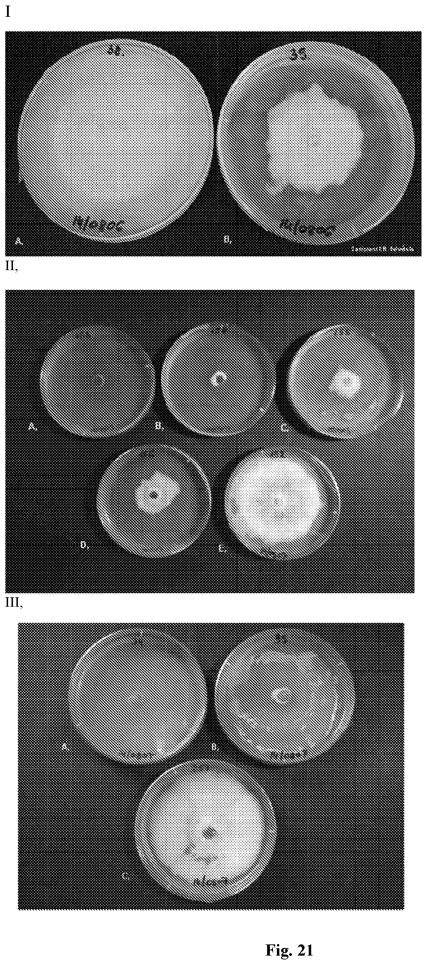

Figure 21. Inhibitory effect of *Azospirillum largimobile* B41 in a dual culture (bacterial lawn, fungal agar disc in the center) on the growth of plant pathogen fungi: (I) *A. alternata*, (II) *F. graminearum* and (III) *F. moniliforme.* I. A, *A. alternata* control; B, B41 (2 × 10⁵/ml) bacterial lawn + *A. alternaria,* incubated at room temperature for 16 days. II. A, *F. graminearum* control; B, B41 (3 × 10⁸/ml) bacterial lawn + *F. graminearum*; C, B41 (3 × 10⁷/ml) bacterial lawn + *F. graminearum*; D, B41 (3 × 10⁶/ml) bacterial lawn + *F. graminearum*; E, B41 (3 x 10⁵/ml) bacterial lawn + *F. graminearum,* incubated at room temperature for 90 days. III. A, *F. moniliforme* control; B, B41 (4 × 10⁶/ml) bacterial lawn + *F. moniliforme*; C, B41 (4× 10⁵/ml) bacterial lawn + *F. moniliforme,* incubated for 90 days at room temperature.

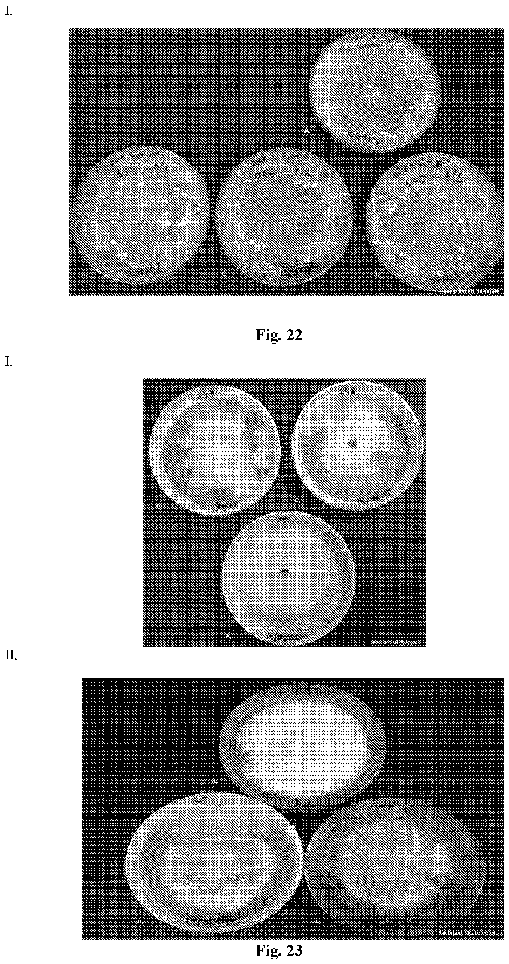

Figure 22. Inhibitory effect of *Azospirillum irakense* NF6 in a dual culture (bacterial lawn, fungal agar disc in the center) on the growth of plant pathogen fungi *Sclerotinia sclerotiorum.* I. A, *S*. *sclerotiorum* control; B, C, D, NF6 (2 × 10⁵/ml) bacterial lawn + *S*. *sclerotiorum*, incubated at room temperature for 31 days
Figure 23. Inhibitory effect of *Exiguobacterium acetylicum* LU44 in a dual culture (bacterial lawn, fungal agar disc in the center) on the growth of plant pathogen fungi (I,) *Fusarium graminearum* and (II,) *Fusarium moniliforme.* I. A, *F. graminearum* control; B, LU44 (2 × 10⁶/ml) bacterial lawn + *F. graminearum,* C, LU44 (2 × 10⁵/ml) bacterial lawn + *F. graminearum,* incubated at room temperature for 9 days II. A, *F. moniliforme* control; B, LU44 (2 × 10⁶/ml) bacterial lawn + *F. moniliforme,* C, LU44 (2 x 10⁵/ml) bacterial lawn + *F. moniliforme,* incubated at room temperature for 15 days

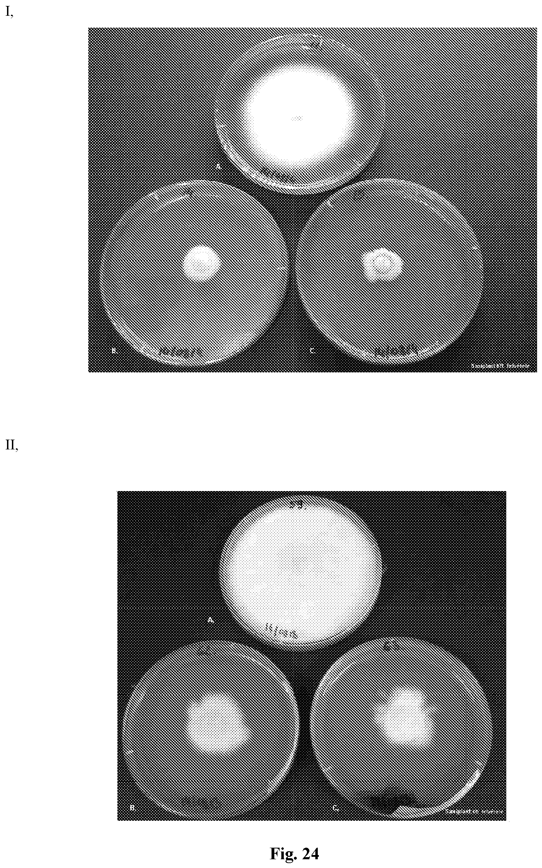

Figure 24. Inhibitory effect of *Paenibacillus peoriae* S284 in a dual culture (bacterial lawn, fungal agar disc in the center) on the growth of plant pathogen fungi (I,) *A. alternata* and (II,) *F. graminearum.* I. A, *A. alternata* control; B, S284 (2 × 10⁶/ml) bacterial lawn + *A. alternata,* C, S284 (2 × 10⁵/ml) bacterial lawn + *A. alternata*, incubated at room temperature for 8 days II. A, *F. graminearum* control; B, S284 (2 × 10⁶/ml) bacterial lawn + *F. graminearum,* C, S284 (2 × 10⁵/ml) bacterial lawn + *F. graminearum,* incubated at room temperature for 9 days.

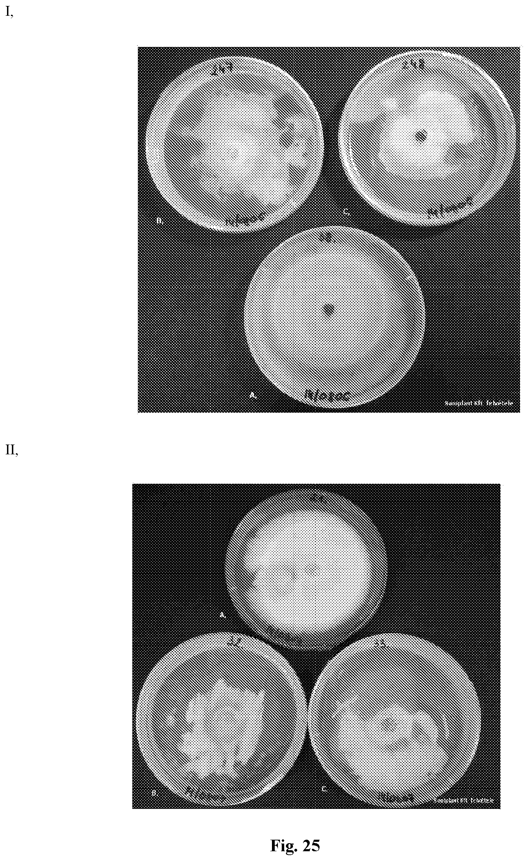

Figure 25. Inhibitory effect of *Agreia pratensis* S47 in a dual culture (bacterial lawn, fungal agar disc in the center) on the growth of plant pathogen fungi (I,) *A. alternata,* and (II,) *F. moniliforme.* I. A, *A. alternata* control; B, S47 (2 × 10⁶/ml) bacterial lawn + *A. alternata*, C, S47 (2 × 10⁵/ml) bacterial lawn + *A. alternata*, incubated at room temperature for 90 days. II. A, *F. moniliforme* control; B, S47 (2 × 10⁶/ml) bacterial lawn + *F. moniliforme,* C, S47 (2 × 10⁵/ml) bacterial lawn + *F. moniliforme,* incubated at room temperature for 15 days.

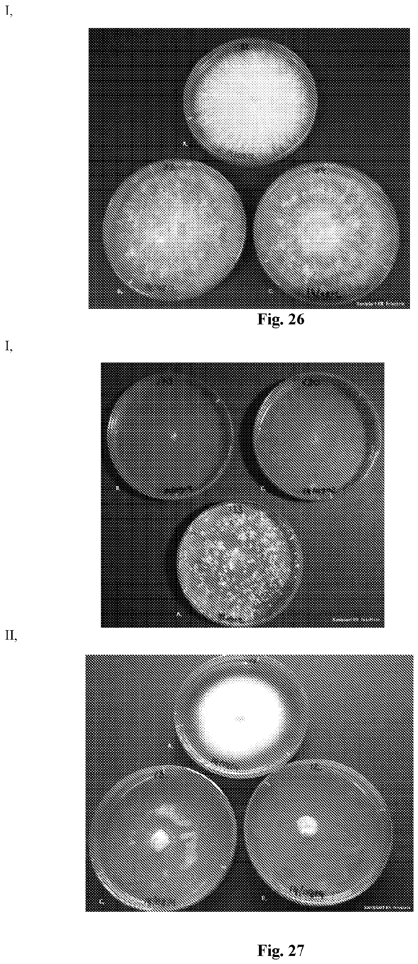

Figure 26. Inhibitory effect of *Arthrobacter crystallopoietes* S153 in a dual culture (bacterial lawn, fungal agar disc in the center) on the growth of plant pathogen fungi (I,) *F. culmorum.* I. A, *F. culmorum* control; B, S153 (2 x 10⁶/ml) bacterial lawn + *F. culmorum,* C, S153 (2 x 10⁵/ml) bacterial lawn + *F. culmorum,* incubated at room temperature for 10 days.

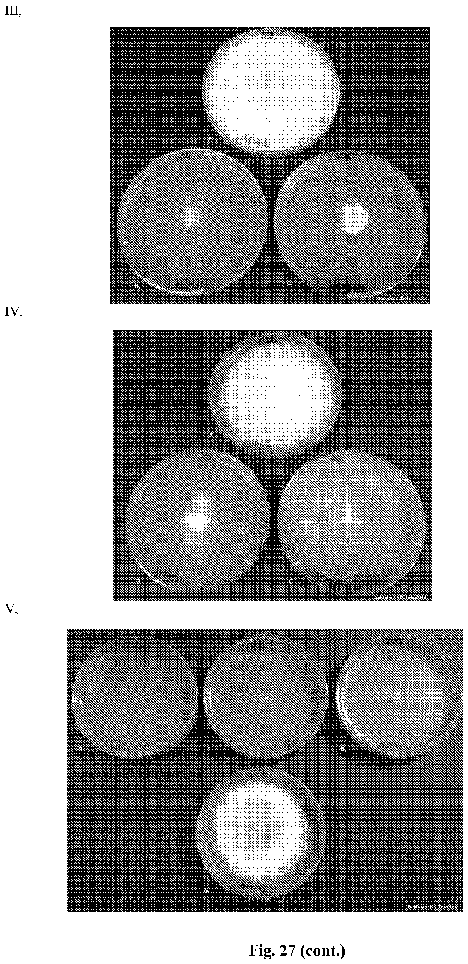

Figure 27. Inhibitory effect of *Pseudomonas jessenii* S125 in a dual culture (bacterial lawn, fungal agar disc in the center) on the growth of plant pathogen fungi (I,) *S*. *sclerotiorum,* (II,) *A. alternata,* (III,) *F. graminearum,* (IV,) *F. culmorum and* (V,) *F. moniliforme.* I. A, *S*. *sclerotiorum* control; B, S125 (2 × 10⁶/ml) bacterial lawn + *S*. *sclerotiorum*, C, S125 (2 × 10⁵/ml) bacterial lawn + *S*. *sclerotiorum*, incubated at room temperature for 60 days. II. A, *A. alternata* control; B, S125 (2 × 10⁶/ml) bacterial lawn + *A. alternata*, C, S125 (2 x 10⁵/ml) bacterial lawn + *A. alternata*, incubated at room temperature for 8 days. III. A, *F. graminearum* control; B, S125 (2 × 10⁶/ml) bacterial lawn + *F. graminearum,* C, S125 (2 × 10⁵/ml) bacterial lawn + *F. graminearum,* incubated at room temperature for 9 days. IV. A, *F. culmorum* control; B, S125 (2 × 10⁶/ml) bacterial lawn + *F. culmorum*, C, S125 (2 × 10⁵/ml) bacterial lawn + *F. culmorum,* incubated at room temperature for 10 days. V. A, *F. moniliforme* control; B, S125 (2 × 10⁶/ml) bacterial lawn + *F. moniliforme,* C, S125 (2 × 10⁵/ml) bacterial lawn + *F. moniliforme,* D, S125 (2 × 10⁴/ml) bacterial lawn + *F. moniliforme,* incubated at room temperature for 90 days.

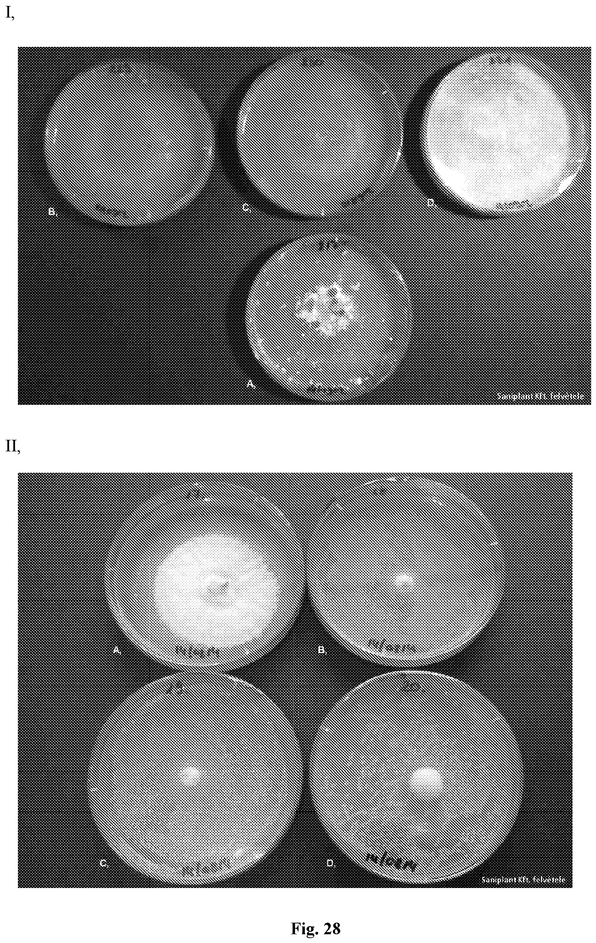

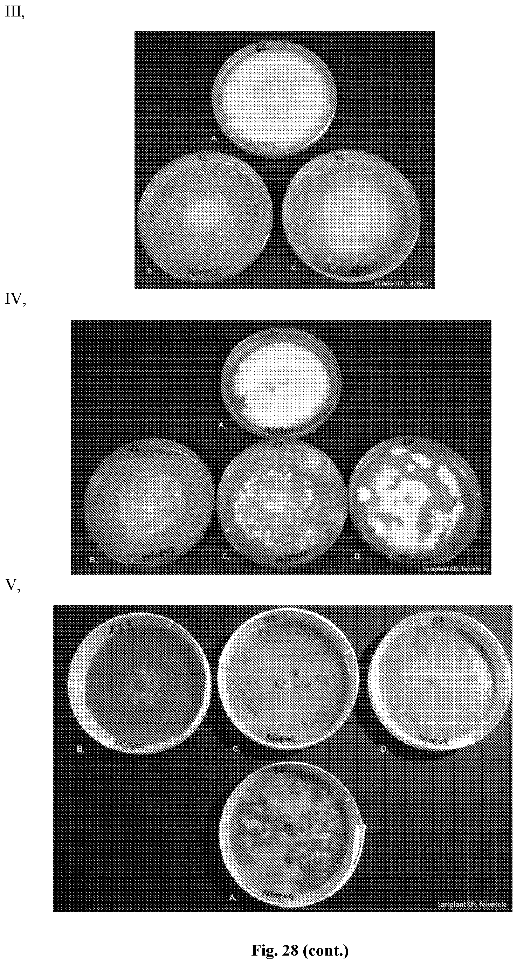

Figure 28. Inhibitory effect of *Pseudomonas chlororaphis* 13/4B in a dual culture (bacterial lawn, fungal agar disc in the center) on the growth of plant pathogen fungi (I,) *S*. *sclerotiorum,* (II,) *A*. *alternata,* (III,) *F. graminearum,* (IV,) *F. culmorum* and (V,) *F. moniliforme.* I. A, *S*. *sclerotiorum* control; B, 13/4 B (2 × 10⁶/ml) bacterial lawn + *S. sclerotiorum*, C, 13/4 B (2 × 10⁵/ml) bacterial lawn + *S. sclerotiorum*, D, 13/4 B (2 × 10⁴/ml) bacterial lawn + *S*. *sclerotiorum*, incubated at room temperature for 60 days. II. A, *A. alternata* control; B, 13/4 B (2 × 10⁵/ml) bacterial lawn + *A. alternata*, C, 13/4 B (2 × 10⁴/ml) bacterial lawn + *A. alternata*, D, 13/4 B (2 × 10³/ml) bacterial lawn + *A. alternata*, incubated at room temperature for 8 days. III. A, *F. graminearum* control; B, 13/4 B (2 × 10⁵/ml) bacterial lawn + *F. graminearum,* C, 13/4 B (2 × 10⁴/ml) bacterial lawn + *F. graminearum,* incubated at room temperature for 9 days. IV. A, *F. moniliforme* control; B, 13/4 B (2 × 10⁵/ml) bacterial lawn + *F. moniliforme,* C, 13/4 B (2 × 10⁴/ml) bacterial lawn + *F. moniliforme,* D, 13/4 B (2 × 10³/ml) bacterial lawn + *F. moniliforme,* incubated at room temperature for 18 days. V. A, *Botrytis cinerea* control; B, 13/4 B (2 × 10⁵/ml) bacterial lawn + *Botrytis cinerea*, C, 13/4 B (2 × 10⁴/ml) bacterial lawn + *Botrytis cinerea;* D, 13/4 B (2 × 10³/ml) bacterial lawn + *Botrytis cinerea*, incubated at room temperature for 90 days.

### Example No.1: Isolation of bacteria from deteriorated soils via SBSS and NSBSS methods

In the course of the SBSS technique, stress conditions are directly applied - as selective pressure - to obtain acidic, neutral, alkaline, high osmotic-tolerant or cold-tolerant suspension cultures from low-fertility, deteriorated soils. Phosphate and / or potassium mobilizing and / or siderophore producing strains are isolated from the soil suspension enriched in culturing conditions modeling abiotic stress. In another protocol of SBSS phosphate and / or potassium mobilizing and / or siderophore producing strains are directly isolated from soil suspensions.

Nitrogen-fixing strains were isolated by both the routine isolation protocols and the novel - previously not described - stress-selective isolation method for nitrogen-fixing bacteria (Nitrogen-fixing SBSS, NSBSS).

Soil samples were taken from different areas under agricultural cultivation with low fertility, deteriorated soil types that are typical in Hungary: alisol, solonchak, calcic solonchak, solonetz , dystric gleysol and dystric fluvisol. 10 types of soil samples were examined: 7 types of brown forest soil - podsolic, non-podsolic and clayey alisol (strongly and slightly acidic), and 3 types of salinized soils: solonetz, acidic or alkaline, clayey or with a high salt concentration (4.8% NaCl).

**Table 1: Type, pH and salt concentration of soil samples via the SBSS method**

| **Salinized meadow soils** | **identif.** | **soil pH** | **salt conc.** |
|---|---|---|---|
| Meadow solonetz, deep | 1c | 6.4 | high |
| Solonetz | 3b | 4.92 | high |
| Clayey meadow solonetz, deep | RF | 8.00 | high |
| | | | |

| **Brown forest soils** | **ident.** | **soil Ph** | **salt conc.** |
|---|---|---|---|
| Alisol, not podzolic brown forest soil | 7a | 4.94 | low |
| | 7b | 4.4 | low |
| Podsolic brown forest soil | 8a | 6.56 | low |
| | 8b | 4.67 | low |
| | 8c | 6.31 | low |
| | 8d | 5.84 | low |
| Clayey alisol, podzolic (strongly acidic) | 9 | 5.69 | low |

### I. Isolation via the SBSS method

Stress-selective isolation: 10 g of soil is suspended in 100 ml of water and 2 ml of nystatin (10mg/ml concentration) is added to inhibit the growth of filamentous fungi usually predominant in acidic soils. After one hour of shaking (at 250 rpm) 3-3 mls of the suspension are inoculated in 100-100 ml of acidic (pH=4), alkaline (pH=9), 6% NaCl-containing or untreated neutral (ph=7) Modified Nutrient Broth (2 ml nystatin/100 ml). The inoculated broths are incubated for 12 h for the enrichment of viable, stress-tolerant bacterial strains.

### Modified Nutrient Broth (NB):

13 g of the Nutrient Broth powder (Oxoid; lot No: CM 0001B), 5.0 g of glucose, 5.0 g of saccharose and 3.0 g of yeast extract (BBL Yeast Extract, Becton Dickinson, BD) is added per every liter of distilled water. Components are solubilized by slight heating, pH adjustment is not necessary (pH=7.4 ± 0.2). Sterilization is done at 121 °C, 1.2 bars, for 15 min.

Acidic, alkaline and salt-containing suspensions are incubated at 28 °C, the neutral suspension is incubated at 10 °C overnight, shaken at 250 rpm. The bacterial suspension are then streaked onto NA, CAS, KB, HP and K agar plates.

### Culture media

### Modified Nutrient agar NA (slant. Petri)

| | |
|---|---|
| Glucose | 5 g |
| Saccharose | 5 g |
| Yeast | 3 g |
| Nutrient Agar (Oxoid CM003B) | 28 g |

The components are added to 1 liter of distilled water, pH is adjusted to 7, and sterilized for 20 min, at 120 °C, at saturated steam pressure.

### CAS (siderophore) agar medium

| | |
|---|---|
| 6.0 g | piperazin- N,N-bis (2-ethanesulfonic acid) |
| 0.6 g | NaOH, |
| 15.0 g | protease-pepton, |
| 15.0 g | MgSO4 x 7 H2O, |
| 1.5 g | K₂HPO₄, |
| 10.0 g | glycerol, |
| 20.0 g | agar. |

The components are added to 900 ml of distilled water, pH is adjusted to 7.0, and sterilized for 20 min, at 120 °C, on saturated steam pressure.

The following components are filtered through a sterile filter and added after sterilization:
60.5 mg Chrome azurol S (CAS) dye in 50.0 ml of distilled water, 10.0 mg FeCl3 x 6 H₂O dye, and 72.9 mg hexadecyl-trimethyl-ammonium-bromide (HDTMA) in another 50.0 ml of distilled water.

### HP (phosphate mobilizing) culture medium

| | |
|---|---|
| hydroxylapatite | 10 g |
| (NH₄)₂SO₄ | 0.5 g |
| NaCl | 0.2 g |
| KCl | 0.2 g |
| MgSO₄×7H₂O | 0.1 g |
| yeast extract | 0.5 g |
| agar | 15 g |
| glucose | 10 g |
| FeSO₄ | 1% 20 mg/100 ml |
| MnSO₄ | 1% 20 mg/100 ml |

The components are added to 1 liter of distilled water, pH is adjusted to 7.2, and sterilized for 20 min, at 120 °C, on saturated steam pressure. Iron-sulphate and manganese sulphate solutions are sterilized before being added to the sterilized broth.

### King B, KB(fluorescent agar) medium

| | |
|---|---|
| pepton | 2 g |
| glycerol | 1 g |
| K₂HPO₄ | 0.15g |
| MgSO₄× 7H₂O | 0.15g |

The components are added to 1 liter of distilled water, pH is adjusted to 7.2, and sterilized for 20 min, at 120 °C, on saturated steam pressure.

### KP culture medium (Aleksandrov culture medium)

| | |
|---|---|
| 5 g | glucose |
| 0.5 g | MgSO₄×7H₂O |
| 0.005 g | FeCl₃ |
| 0,1 g | CaCO₃ |
| 2,0 | hydroxylapatite |
| 2.0 g | K-Al-silicate (HML-5) |
| 20 g | Agar |

The components are added to 1 litre of distilled water, pH is adjusted to 7.0, and sterilized for 20 min, at 120 °C, on saturated steam pressure.

### II. Isolation according to the NSBSS method

I. Isolation
   1. Routine isolation method
   2. Cold enrichment
   3. Isolation from alkaline-saline soils
II. Samples
   1. alisol (brown forest soil, soil "B")
   2. sodic solonchak (I 23 06, 5b; pH 8,39)
   3.: 1 intact specimen of northern wild rice with the associated rizospheric soil, (site of sampling: Mezőtúr), soil pH 7.1 - 7.3

### 1. Routine isolation method

Selective isolation of nitrogen-fixing soil bacteria from soil and plant samples is a two-phase process:
- first, the bacterial population is preenriched in the given sample, as nitrogen-fixers are naturally very low in number. A higher cell count improves isolation efficiency.
- second, the strains are actually isolated from the preenriched samples.

The protocol for the selective enrichment of the nitrogen-fixing microbes was nitrogen-free soft agar technique (Döbereiner, 1989), using two different pre-enrichment media:
OK nitrogen-free minimal soft agar (Okon et al., 1976)

29.0 mmol KH₂PO₄, 26.0 mmol K₂HPO₄, 37.0 mmol malic acid, 75.0 mmol NaOH, 1.7 mmol NaCl, 0.81 mmol MgSO₄, 0.13 mmol CaCl₂, 0.01 mmol MnSO₄, 0.02 mmol Fe₂SO₄, 0.02 mmol Na₂EDTA, 0.004 mmol Na₂MoO₄, 4.0 µmol biotin, agar 0.15 % (w/v), 1000 ml distilled water.
pH 6.8-7.0.

Combined nitrogen-free minimal soft agar (Rennie, 1981):
5.0 g saccharose, 5.0 g mannitol, 0.5 ml (60 %, v/v) Na-lactate, 0.80 g K₂HPO₄, 0.20 g KH₂PO₄, 0.20 g MgSO₄×7H₂O, 0.06 g CaCl₂, 0.10 g NaCl, 100 mg yeast extract, 25 mg Na₂MoO₄×2H₂O, 28 mg Na₂FeEDTA, 5.00 µg biotin, 10 µg PABA, agar 1.5 g
pH 7.0, 1000 ml of distilled water

The nitrogen-free malate soft agar is the pre-enrichment medium most commonly used for the isolation of associative nitrogen-fixers living on the surface of, or in the close environment of roots of monocotyledons. The glucose/saccharose/mannite supplemented nitrogen-free soft agar is usually used for the preenrichment of free-living soil bacteria that are capable of utilizing saccharides.

The soft agar technique makes it possible to simultaneously enrich diazotroph strains - within the same soil sample - that differ in their oxygen requirement regarding nitrogen-fixation and nitrogen-dependent growth. Selective enrichment is a consequence of the aerotaxis of bacteria, namely moving according to the oxygen concentration gradient in the soft agar (based on the diffusion rate of oxygen). As the soft agar is nitrogen-free, nitrogen requirements can only be met by fixing atmospheric nitrogen. Bacteria are localized in the zone of the soft agar medium where local oxygen-concentrations are optimal for nitrogen-fixation and nitrogen-dependent growth.

The enrichment protocol is performed as follows:
The selected soft agar medium is prepared in hermetically sealable vials. A few particles of soil/roots are placed or 20-30 µl of soil suspensions are pipetted in the vials. It is advised not exceed those amounts, as high nitrogen-concentration of the samples can mask the signs of nitrogen-fixation. Practically, 10-15 parallel measurements are initiated from each sample. Vials are incubated at 28 °C for 3-5 days. Visible pellicles, diffuse or distinct rings in the medium indicate the alignment of bacterial populations.

After incubation, the nitrogenase enzyme activity is measured by acetylene-reduction assay.

Vials expressing enzyme activity above the determined threshold are subcultured in a new set of vials containing nitrogen-free soft agar media. Vials with no measurable enzyme activity are excluded from further analysis. Samples showing low levels of enzyme activity are further tested, if required. A minimum of 4-6 passages are performed.

Isolation of nitrogen-fixing bacteria from pre-enriched samples:
When isolating microaerophilic bacteria, the preenriched culture is streaked onto solid agar plates containing chemically bond nitorgen and yeast extract (as required). Each of the developed colonies are inoculated in nitrogen-free soft agar, passaged for 3-4 times and again streaked onto solid agar plates. The developed colonies are classified according to morphology, and inoculated into nitrogen-free soft agar, passaged for 3-4 times and plated. The aim is to obtain pure cultures, checking after every passage whether the culture has retained its initial nitrogen-fixation capacity. The strains isolated from pure cultures are measured for nitrogen-fixation capacity.

Measurement of nitrogen-fixation capacity (Burris, 1972)
Nitrogen-free soft agar is prepared in hermetically sealable vials. The selected strains are incubated for ...nights in minimal nutrient broth containing chemically bound nitrogen. The enriched liquid cultures are washed in minimal broth and resuspended to obtain a 1-2 × 10⁸ CFU/ml cell count. 10-20 ..1 are inoculated into soft agar.

The vials are hermetically closed and 5% acetylene (vol _{acetylene} / vol ₐᵢᵣ) is added. Vials are incubated at 28°C for 2-3 days.

### Measurement of the nitrogen-fixation capacity

Nitrogenase enzyme activity is determined by the efficiency of the enzyme-catalysed reduction of acetylene, as determined by the gas chromatographic quantification of etylene and acetylene.

Nitrogen-fixation capacity is defined according to the measured quantity of etylene and cell count of the culture in nmol C₂H₄/10⁷ CFU/ml. In lack of an etylene standard, nitrogen-fixation capacity is described as the etylene/acetylene ratio (Et%) of per unit of cell count (Et%/10⁷ CFU/ml).

Cell count of cultures are determined in the same culture medium as that of the initial culture by the spread-plate method.

### 2. Cold enrichment

Pre-enrichment is performed as follows:
The selected soft agar medium is prepared in hermetically sealable vials. A few particles of soil/roots are placed or 20-30 µl of soil suspensions are pipetted in the vials. It is advised not exceed those amounts, as high nitrogen-concentration of the samples can mask the signs of nitrogen-fixation. Practically, 10-15 parallel measurements are made from each sample. Vials are incubated at 6-8 °C for 3-5 days. Visible pellicles, diffuse or distinct rings in the medium indicate the alignment of bacterial populations.

After incubation, the nitrogenase enzyme activity is measured by acetylene-reduction assay.

Vials expressing enzyme activity above the determined threshold are subcultured in a new set of vials containing nitrogen-free soft agar media. Vials with no measurable enzyme activity are excluded from further analysis. Samples showing low levels of enzyme activity are further tested, if required. Cultures are twice passaged at 6-8 °C. A further 3-4 passages are done at 28°C, as described earlier.

### 3. Isolation from alkaline-saline soils

A protocol is arranged on the basis of previously described methods and culture media considering the characteristics of the strain (growth, nitrogen-fixation, nutrient utilization).

Pre-enrichment soft agars are prepared according to the soil ecological conditions of the diazotroph bacterial population (soil pH, salt concentration). Enrichment of samples deriving from alkaline-saline soils can be started in nitrogen-free soft agar containing a double concentration of NaCl/NaHCO₃.

The osmolyte concentration can be increased during the phase of enrichment, but not practical, as nitrogen-fixation is not soon detectable during the course of serial subculturing.

It is practical to work with lower osmolyte concentrations and investigate the salt-tolerance of nitrogen-fixation in the isolated pure cultures.

### II. NSBSS isolation from low fertility, deteriorated soil

### 1. Alisol (brown forest soil, soil "B")

The soil sample did not contain vegetation. Preenrichment was initiaited from soil particles, 18 vials in paralell. 5 cultures were passaged 5 times, inbetween the cultures become visibly clearer with distinct patterns of bands, according to the oxygen requirements of the enriched bacterial populations. The presence of different patterns indicates that the diazotroph population comprises of several species, differing in their metabolic requirements.

From pre-enriched cultures, 300 pure cultures were obtained and measured for nitrogenase-activity. We obtained 48 diazotroph isolates, the results of nitrogenase activity measurements are registered in the laboratory records. Out of the 48, 11 strains were deposited in the laboratory strain collection (at - 70°C), and 6 strains were selected for further analysis.

### Detection of nitrogen-fixation and measurement of nitrogen-fixation capacity

### Starter cultures

Strains cultures are streaked on - according to the strains requirements and aim of the measurement - nutrient agar, sugar-supplemented nutrient agar, Okon minimal agar or combined minimal agar. Plates are incubated at 28 °C for 24-48 h.

After incubation, shaken cultures with similar culturing media are prepared, incubated at 28°C overnight, with constant aeriation. Cell count shall reach 1-2 × 10⁹/ml. Cell counting is done by the plate count method.

Obligate anaerobic strains are cultured according to their oxygen and growth requirements. Cell counting is done by an appropriate method.

### Preparation of nitrogen-fixing cultures, measurement of nitrogenase enzyme activity (Burris, 1972) and nitrogen-fixation capacity

In aerobic and microaerophil conditions, on soft agar medium (Döbereiner, 1989)
Nitrogen-free soft agar medium is prepared in hermetically sealable vials. The enriched liquid cultures incubated overnight are washed in nitrogen-free minimal broth and resuspended to obtain a 1-2 × 10⁸ CFU/ml cell count. 10-20 ..1 are inoculated into soft agar.

The vials are hermetically closed and 5% acetylene (vol _{acetylene} / vol ₐᵢᵣ) is added. Vials are incubated at 28°C for 2-3 days.

### Measurement of the nitrogen-fixation capacity

Nitrogenase enzyme activity is determined by the efficiency of the enzyme-catalysed reduction of acetylene, as determined by the gas chromatographic quantification of etylene and acetylene.

Nitrogen-fixation capacity is defined according to the measured quantity of etylene and cell count of the culture in nmol C₂H₄/10⁷ CFU/ml. In lack of an etylene standard, nitrogen-fixation capacity is described as the etylene/acetylene ratio (Et%) of per unit of cell count (Et%/10⁷ CFU/ml).

Cell count of cultures are determined in the same culture medium as that of the initial culture by the spread-plate method.

On a solid agar medium, in aerobic and anaerobic conditions, the exact quantification of nitrogen-fixation is disabled as measured values cannot be determined per unit of cell count. Capacity can be estimated by comparison of the etylene/acetylene ratios of the studied and a control strain (with known characteristics).

### 2. Calcic solonchak (I 23 06 kódszámú, 5b; pH 8,39)

The soil sample contained grass tuft, thus pre-enrichment samples included soil particles, and the rizospheric soil, root surface, surface disinfected root tissue and stem of grass.

28 samples were incubated in parallel. Cultures were passaged 6 times. The cultures become visibly clearer with distinct patterns of bands, according to the oxygen requirements of the enriched bacterial populations. The presence of different patterns indicates that the diazotroph population comprises of several species, differing in their metabolic requirements.

From pre-enriched cultures, 200 individual cultures were obtained and measured for nitrogenase-activity. We obtained 120 diazotroph isolates, the results of nitrogenase activity measurements were registered in the laboratory records. 20 strains of prominently high nitrogen-fixation capacity were deposited in the laboratory strain collection (at - 70°C).

Cultures originating from surface disinfected root and stem samples expressed no detectable level of nitrogen-fixation. Nitrogen-fixing bacteria are presumably not associate to root tissue and stem. Isolated strains are either rizospheric or associated to root surface.

### 3.: 1 intact specimen of northern wild rice with the associated rizospheric soil, (site of sampling: Mezőtúr)

The pH of the soil sample: pH 7,1 - 7,3

Pre-enrichment of diazotrophs were done at 10 °C and at 28°C. In the course of the cold enrichment root and rizospheric soil samples were used, subcultured twice, with 3-4 days incubation. Pre-enrichment at 28 °C were done as described earlier, originating from rizospheric soil, root and stem samples from the most bottom internodium. Samples were not subjected to surface disinfection.

### Results of cold treatment

5 samples were incubated in parallel. Cultures were passaged 3 times. The cultures become visibly clearer with distinct patterns of bands, according to the oxygen requirements of the enriched bacterial populations. The presence of different patterns indicates that the diazotroph population comprises of several species, differing in their metabolic requirements.

From pre-enriched cultures, 160 individual cultures were obtained and measured for nitrogenase-activity. We obtained 60 diazotroph isolates, the results of nitrogenase activity measurements are registered in the laboratory records. 28 strains of prominently high nitroegn-fixation capacity were deposited in the laboratory strain collection (at - 70°C).

Results of enrichment at 28 °C. 26 samples were incubated in parallel. Cultures were passaged 6 times. The cultures become visibly clearer with distinct patterns of bands, according to the oxygen requirements of the enriched bacterial populations. The presence of different patterns indicates that the diazotroph population comprises of several species, differing in their metabolic requirements.

From pre-enriched cultures, 260 individual cultures were obtained and measured for nitrogenase-activity. We obtained 60-80 diazotroph isolates, the results of nitrogenase activity measurements are registered in the laboratory records. 12 pure cultures strains of prominently high nitrogen-fixation capacity were deposited in the laboratory strain collection (at - 70°C). The purification of other strains are currently in progress.

### Example No.2: Screening of bacterial strains suitable for soil inoculation. SBSS screening system

We have isolated over a thousand stress-tolerant bacterial strains that are able to grow in low fertility, deteriorated soils in extreme conditions. The isolated strains are sustained and included in the soil microbial SBSS strain collection. Strains in the collection are further screened for stress-tolerance (pH, salt), human pathogenity (positive strains are excluded from potential inoculants), plant growth promotion (PGPR) and promotion soil aggregate formation (EPS). In parallel, novel diazotroph strains are isolated from low-fertility soils and studied for plant growth promoting properties.

**Table 2: SBSS vizsgálati szempontok and associated measurement methods**

| Tolerance | Plant health and growth promotion, soil amelioration and soil structure improvement | | | Pathogenity studies on selective media | | |
|---|---|---|---|---|---|---|
| Buffering effect in a range of pH 3-8 | Nitrogen-fixation | Nutrient refill | acetylene reduction test | Exclusion of human pathogen strains | MYP | Bacillus, Proteus and Staphylococcus |
| Salt tolerance Growth in 1-8% NaCl concentration | phosphate mobilization | | HP and KP culture medium | | XLD | E.coli, Enterobacter, Aeromonas, Klebsiella, Serratia, Shigella, Citrobacter, Proteus |
| Cold tolerance Culturing at 4-8 °C | indole acetic acid production | measurement of hormone-production | spectrophotometry wit Salkowsky reagent; thin layer chromatography with Ehrlich reagent | | MC | E. coli, Enterobacter, Klebsiella, Proteus, Shigella, Pseudomonas |
| | siderophore synthesis | biocontrol, iron supply and quorum sensing | CAS agar medium (Chrome Azurol S reagent) | | CFC | Fluorescent Pseudomonas spp. |
| | exopolysaccharide production | soil aggregate formation | macro morphological studies for viscosity of the culture, ethanol extraction | | LSB | coliform spp. |
| | ACC deaminase production | Measurement of enzyme activity | DF culture medium containing ACC, spectrophotometric assay of alpha-ketobutyrate | | | |

**Strains obtained in the SBSS screening system are classified by scoring.** The maximal score of acidic pH-, salt- and cold-tolerant strains (the widest range of tolerance) is 6 points. The strains most effective in plant growth promotion *and* soil aggregate formation are scored a maximal of 36 points

**Table 3.: Scoring of strains according to the SBSS screening system**

| Studied quality | | Associated parameters | Categories | Maximal score | Minimal score |
|---|---|---|---|---|---|
| Stress tolerance | Growth rate at different levels of pH | rate of growth in Nutrient broth adjusted to different pH values | no signs of growth(-2), narrow range of tolerance (0), grows at all pH values(2) | 2 | -2 |
| | Salt tolerance | Quantified level of NaCl-induced osmotic stress | growth in the presence of NaCl in the concentration of 1-2% (-1), 3-4% (0), 5-6% (1), 7-8% (2) | 2 | -1 |
| | Growth rate at 4-8 °C | Quantified level of cold tolerance | signs of growth (2), no signs of growth (-2) | 2 | -2 |
| Overall score | | Sum of scores quantifying the levels of stress tolerance | | 6 | -5 |

| PGPR, nutrient refill, soil structure improvement | Nitrogen-fixation | Acetylene reduction | not capable (0), capable(6) | 6 | 0 |
|---|---|---|---|---|---|
| | Siderophore production | Quantified level of siderophore production | no discoloration zone (0), 1-2 mm | 6 | 0 |
| | | | discoloration zone (2), 3-4 mm | | |
| | | | discoloration zone (4), > 4 mm | | |
| | | | discoloration zone (6) | | |
| | Polysaccharide production | Polysaccharide synthesis | positive (6), negative (0) | 6 | 0 |
| | ACC deaminase production | Quantified level of deaminase production | no α-ketobutyrate (0), 1 µM α-ketobutyrate mg/h (2), 5 µM α- α-ketobutyrate mg/h(4), 10 µM α- α-ketobutyrate mg/h(6) | 6 | 0 |
| | Phosphate solubilization | Quantified level of phosphate solubilization | No clearance (0), 1-2 mm zone of clearance (2), 3-4 mm zone of clearance (4), > 4 mm zone of clearance(6) | 6 | 0 |
| | Indole acetic acid production | Quantified level of indole acetic acid production | 1,0 OD 530 felett (6), 0,2 és 1,0 OD 530 között (3) nincs színreakció (0) | 6 | 0 |
| Overall score | | Sum of scores quantifiying growth promozing and soil ameliorating properties | | 36 | 0 |

### Example No.3: Salt tolerance testing

Strains isolated according to Example 1 were subjected to subculturing in Modified Nutrient Agar (NA) containing NaCl in a concentration of 0.1%; 1; 2%; 3%; 4%; 5%; 6%; 7% and 8%, respectively. The highest concentration that the strain is able to intensively grown in, is registered.

### Modified Nutrient Agar (NA):

### Agar plates signalled "NA" are prepared as follows:

28 g of the Nutrient Agar powder (Oxoid; Cat. No: CM 0003), 5.0 g of glucose, 5.0 g of saccharose and 3.0 g of yeast extract (BBL Yeast Extract, Becton Dickinson, BD) is added per every litre of distilled water. Components are solubilized by slight heating, pH adjustment is not necessary (pH=7.4 ± 0.2). Sterilization is done at 121 °C, 1.2 bars, for 15 min.

**Table 4.: Number and distributiuon of slat-tolerant, moderately salt tolerant and not salt-tolerant strains isolated from low fertility soils.**

| **NaCl concentration** | **Number of strains** | **Percental rate (%)** | **Strain** | **Range of tolerance** |
|---|---|---|---|---|
| 1% | 10 | 5 % | ES212, ES218, ES255, **S268, S279, S281, S296, S305, S315, S316, B41, S47, 13/4B, S225, S28** | not salt-tolerant |
| 2% | 10 | 5% | ES282, **S266, S278, S280, S284, S292, S298, S300, S309, S317, 242/9, NF11** | |
| 3% | 11 | 5% | ES279, ES315, **S276, S295,** LU3, LU11, LU14, LU17, LU23, LU48, LU51 | moderately salt tolerant |
| 4% | 39 | 19% | ES205, ES214, ES215, ES220, ES225, ES230, ES241, | |
| | | | ES243, ES244, ES246, ES251, ES252, ES254, ES258, ES259, ES260, ES262, ES265, ES269, ES284, ES287, ES288, ES291, ES295, ES298, ES300, ES301, ES311, ES313, ES319, S125, **S272, S274, S275, S289,** LU15, LU21, LU32, LU34, LU49, NF6, NF10 | |
| 5% | 42 | 20 % | ES203, ES204, ES207, ES211, ES216, ES219, ES223, ES224, ES228, ES233, ES236, ES242, ES245, ES248, ES249, ES272, ES274, ES275, ES276, ES277, ES278, ES283, ES290, ES292, ES293, ES294, ES302, ES303, ES305,ES306, ES310, ES322, ES324, S94, **S270, S273. S297, S314, S319,** LU1, LU5, LU16, LU46 | sótűrő |
| 6% | 17 | 8% | ES200, ES201, ES202, ES234, ES235, ES237, ES257, ES261, ES289, ES296, ES314, ES318, ES320, ES321, ES325, **S318,** LU4 | |
| 7 % | 5 | 2% | ES209, ES239, ES253, **S269,** LU35 | |
| 8 % | 72 | 35 % | ES206, ES213, ES217, ES221, ES222, ES226, ES231, ES232, ES238, ES240, ES250, ES256, ES266, ES267, ES268, ES270, ES271, ES273, ES285, ES286, ES297, ES299, ES304, ES307, ES308, ES309, ES312, ES316, ES317, ES323, ES326, ES327, S33, **S282, S299, S320.** LU2, LU6, LU7, LU8, LU9, LU10, LU13, LU19, LU20, LU22, LU24, LU25, LU26, LU27, LU28, LU29, LU30, LU31, LU33, LU36, LU37, LU38, LU39, LU40, LU41, LU42, LU43, LU44, LU45, LU47, LU50, LU52, LU53, LU54, LU55, LU56, LU57 | |
| In total | 206 | 100 | | |

A total of 206 strains were tested. We have classified strains as "salt tolerant" (5-8% NaCl), 'moderately salt-tolerant' (3-4% NaCl) and 'not salt-tolerant' (1-2%). 45-45% of strains proved to be salt-tolerant, or moderately salt tolerant, respectively, and only 10% was sensitive to higher concentrations. Out of the 206 strains, 186 strains more or less tolerated the presence of salt in higher concentrations: in 1-2% NaCl 20 strains, in 2-5% NaCl 92 strains, and in 6-8% NaCl 94 strains were able to grow.

### Example No.4: pH-tolerance studies

Strains isolated according to Example No.1 were subjected to culturing in Nutrient Broth (NB) medium with a pH level of 3,4,5,6,7 and 8, respectively, and the signs of growth and the pH of the culture were registered after 24 hours. Cell count at the time of inoculation is recorded (+), and rate of growth is classified in four groups (-,+,++,+++). A wide range of pH tolerance or effective buffering effect are favourable properties in potential soil inoculants.

The pH of the nutrient broth is adjusted according to the pH of the soil the given strain is derived from and sterilized. Each strain is inoculated in 4ml of Nutrient broth medium in a test tube and incubated at 28 °C overnight in a rotator.

A pH-gradient series of nutrient broth is prepared, adjusted to pH 3,4,5,6,7 and 8, prior to sterilization. 4ml-4ml is pipetted in test tubes and inoculated with 0.5 ml of the overnight grown NB cultures and incubated at 28 °C overnight in a rotator. After 24 hours, cultures showing signs of growth are selected and the pH of the culture is measured. If signs of growth are not apparent, the tube is incubated for another 24 hours. pH of 48h cultures are also measured and registered.

We have observed that strains tend to adjust the pH of their environment to acidic, neutral or alkaline. In regard to the buffering effect, strains can be classified in three categories (no buffering capacity, moderate and good buffering capacity) based on the difference between the pH of the culture and the control broth.

In respect to the rate of growth, strains were classified in also three categories (wide range of pH tolerance: growing at all pH levels, narrow range of tolerance: growing at a limited range of pH, or no growth at any of the pH values). Strains growing at all pH-levels were classified as having a wide range of tolerance, the strains that grew only in a given range of pH were classified as having a narrow range of pH tolerance.

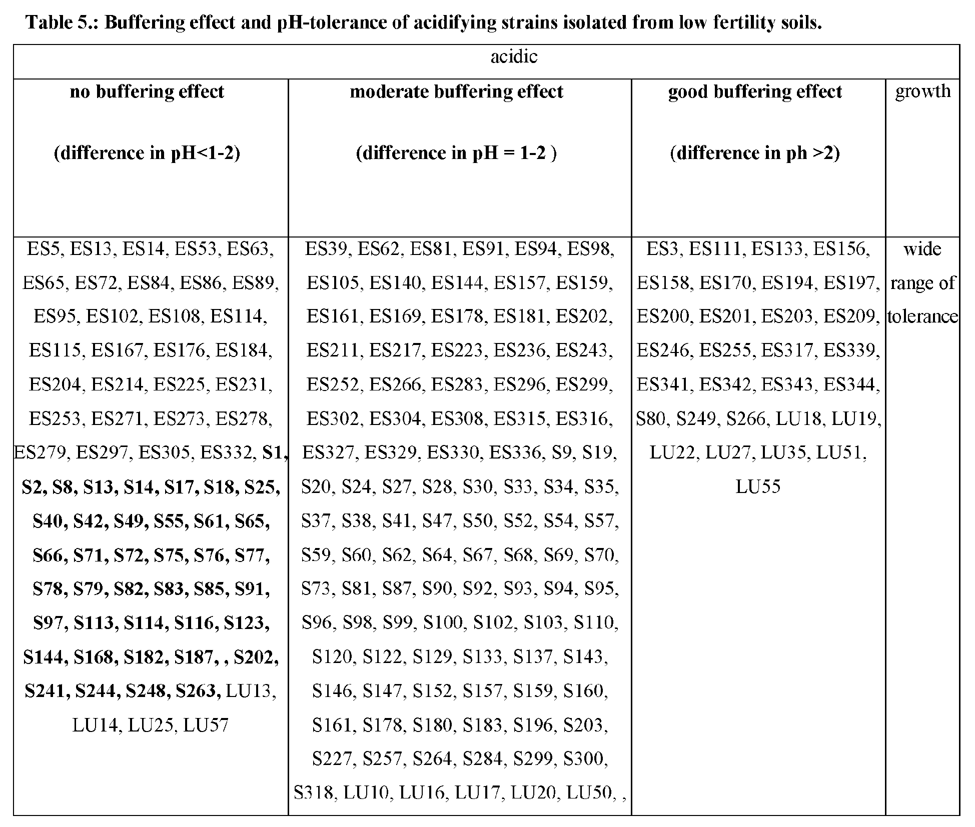

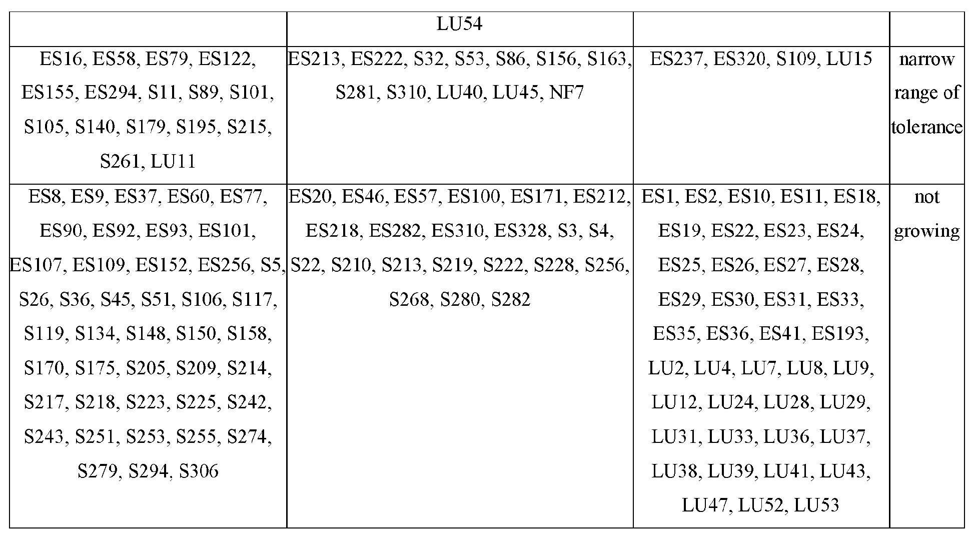

Strains with a moderate or good buffering effect are able to decrease the pH of the culture medium from neutral to acidic. Strains with no buffering effect do not alter the pH of the medium, compared to control.

**Table 6: Buffering effect and pH-tolerance of alkalifying strains isolated from low fertility soils.**

| alkaline | | | |
|---|---|---|---|
| **no buffering effect (difference in pH<1-2)** | **moderate buffering effect (difference in pH = 1-2 )** | **good buffering effect (difference in ph >2)** | growth |
| | 13/4B | ES4, ES7, ES32, ES48, ES49, ES55, ES56, ES59, ES64, ES66, ES70, ES73, ES74, ES76, ES80, ES82, ES87, ES97, ES106, ES112, ES113, ES125, ES126, ES128, ES130, ES136, ES137, ES138, ES141, ES145, ES147, ES148, ES149, ES150, ES151, ES153, ES172, ES175, ES185, ES198, ES205, ES224, ES228, ES233, ES235, ES241, ES242, ES245, ES250, ES254, ES276, ES277, ES284, ES287, ES288, ES289, ES290, ES292, ES293, ES298, ES300, ES301, ES303, ES321, ES322, S10, S23, S58, S63, S88, S107, S111, S115, S118, S125, S126, S127, S128, S132, S138, S151, S153, S155, S165, S166, S174, S181, S184, S186, S188, S189, S192, S193, S194, S258, S267, S295, S297, S302, S303, S304, S314, LU1, LU5, LU21, LU32, LU34, LU 44, LU56 | wide range of tolerance |
| | B41, NF11, 242/9, NF10, NF6 | ES61, ES67, ES96, ES99, ES142, ES196, ES238, ES251, ES261, ES269, ES291, S301 | narrow range of tolerance |

Strains with a moderate or good buffering effect are able to increase the pH of the culture medium from acidic to neutral. Strains with no buffering effect do not alter the pH of the medium, compared to control.

**Table 7:Buffering effect and pH-tolerance of neutralizing strains isolated from low fertility soils.**

| neutral | | | |
|---|---|---|---|
| **no buffering effect (difference in pH<1-2)** | **moderate buffering effect (difference in pH = 1-2 )** | **good buffering effect (difference in ph >2)** | growth |
| | ES6, ES54, ES78, ES83, ES88, ES110, ES116, ES119, ES134, ES154, ES163, ES174, ES180, ES182, ES183, ES191, ES206, ES207, ES208, ES215, ES216, ES221, ES234, ES239, ES244, ES249, ES258, ES270, ES274, ES285, ES307, ES312, ES313, ES331, S121, S142, S145, S149, S171, S172, S197, S201, S206, S211, S250, S254, S296, LU6, LU26, LU49 | ES12, ES15, ES17, ES21, ES51, ES69, ES85, ES103, ES118, ES124, ES129, ES135, ES143, ES160, ES165, ES177, ES187, ES188, ES199, ES220, ES226, ES232, ES240, ES267, ES268, ES272, ES275, ES281, ES286, ES295, ES309, ES323, ES324, ES333, ES335, ES338, S16, S21, S43, S84, S104, S112, S130, S131, S135, S139, S154, S164, S169, S176, S177, S190, S191, S198, S199, S226, S265, S275, S276, S292, S309, S313, LU42 | wide range of tolerance |
| | ES50, ES68, ES117, ES121, ES179, ES248, ES306, ES340, S273, S288, S289, S298, S319 | ES120, ES139, ES189, ES219, ES230, ES257, ES318, ES319, ES326, ES337, S108, S141, S173, S208, S262, S270 | narrow range of tolerance |
| | ES75, ES311, S212, S269, S272, S283, S305, S316 | ES104, ES123, ES127, S204, S308 | not growing |

Strains with a moderate or good buffering effect are able to increase the pH of the culture medium from acidic and neutral to alkaline.

### Example No.5: Cold tolerance studies

Cold-tolerance of the isolates obtained according to Example 1 and the SBSS system was investigated on agar medium, incubated at 4-8 °C. Isolated were oculated on NA plates and incubated in the refrigerator at 4-8 °C for 4 days.

**Table 8: Number and distribution of cold-tolerant and cold-sensitive strains isolated from low fertility soils.**

| cold-tolerance | number of strains | percental rate (%) |
|---|---|---|
| cold-sensitive | 196 | 60, 5 % |
| cold-tolerant | 128 | 39,5 % |
| cold-tolerant strains | | |
| S121, ES126, ES134, ES140, ES176, ES178, ES180, ES181, ES182, ES183, ES184, ES185, ES202, ES204, ES205, ES207, ES209, ES210, ES211, ES214, ES215, ES216, ES219, ES220, ES225, ES228, ES230, ES231, ES232, ES233, ES234, ES235, ES236, ES238, ES239, ES244, ES246, ES248, ES249, ES250, ES251, ES252, ES253, ES255, ES256, ES257, ES260, ES261, ES262, ES265, ES266, ES271, ES272, ES273, ES274, ES275, ES281, ES283, ES289, ES297, ES298, ES305, S47, S 125, **S147, S165, S176, S177, S178, S182, S191, S197, S201, S206, S215, S227, S272. S273, S274, S275, S281, S295, S300, S309, S314, S316,** LU2, LU3, LU4, LU5, LU7, LU8, LU9, LU13, LU19, LU21, LU22, LU23, LU24, LU25, LU26, LU27, LU28, LU29, LU31, LU32, LU33, LU34, LU35, LU37, LU38, LU39, LU40, LU41, LU42, LU43, LU44, LU45, LU46, LU47, LU48, LU49, LU50, LU51, LU52, LU53, LU54, LU55, LU56, LU57, NF7, S33 | | |

324 strains were subjected to cold tolerance testing. 39.5% was shown to be cold tolerant. 128 out of the 324 strains were able to grow at 4-8 °C.

### Example No.6: Genus classification according to the SBSS screening system, and exclusion of human pathogen strains

It is important to identify and exclude human pathogen strains in accordance with the methodology of the SBSS. MYP, XLD, MC, CFC solid agar media and LSB broth were used for the selective culturing of strains. The MYP agar is suitable for the detection of *Bacillus subtilis, Proteus mirabilis* and *Staphylococcus strains.* The XLD agar is used for the detection of *E. coli, Enterobacter, Aeromonas, Klebsiella, Serratia, Hafnia, Citrobacter, Proteus, Shigella* and *Salmonella* spp. The CFC medium is selective for UV-fluorescent *Pseudomonas* spp.

Isolates were identified according to routine microbiological practice based on Gram test, phase contrast microscopy, colonial morphology and catalase-oxydase test.

### Detection of coliforms:

### Laurvl-sulphate broth (LSB)

| | |
|---|---|
| Natrium-lauryl-sulfate | 0.1 g |
| Pancreatic digest of casein | 20 g |
| Lactose | 5 g |
| Dipotassium phosphate | 2.75 g |
| Monopotassium phosphate | 2.75 g |
| Sodium chlorid | 5 g |

1 litre of distilled water is added to the components, the pH is adjusted to 6.8 and the broth is sterilized at 120°C for 15 min, at saturated steam pressure. The genus of the strains is identified according to the characteristics of growth: rapid proliferation, gas formation: *E*. *coli, Klebsiella, Citrobacter;* moderate rate of growth: *Salmonella, Proteus vulgaris, Enterococcus faecalis, Serratia;* low rate of growth: *Staphylococcus aureus, Micrococcus luteus*

**Table 9: Number and distribution of strains isolated from low fertility soils showing coliform-type growth in LSB medium, according to the SBSS principles**

| **LSB** | +++ | ++ | + | - | Number of strains |
|---|---|---|---|---|---|
| **in total** | 161 | 192 | 168 | 106 | 627 |
| **in percentage** (%) | 26% | 31% | 27% | 17% | 100% |

106 strains showed no signs of growth, 627 isolates could proliferate in the lauryl sulfate broth selective for coliform bacteria. Intensive growth was observed in 161 strains.

### Detection of Bacillus, Proteus and Staphylococcus:

### MYP agar medium

| | |
|---|---|
| Beef extract | 1 g |
| Pepton | 10 g |
| D-mannitol | 10 g |
| Sodium-chloride | 10 g |
| Phenol red | 0.025g |
| Agar | 15 g |

23 g of MYP agar is added to 450 ml distilled water and heated until dissolved, then the broth is sterilized at 120°C for 15 min, at saturated steam pressure. When cooled down to 45 °C, 8.2 ml polymyxin and 25 ml egg extract is added. The polymyxin is to be dissolved in 5ml of sterilized water.

The genus is identified according to colour of colonies and presence of precipitation (halo). *Bacillus:* red colonies, halo. *Bacillus subtilis*: yellow or pink colonies, halo. *Proteus mirabilis:* red colonies, no halo, *Staphylococcus*: yellow colonies, halo.

**Table 10: Number and distribution of polymyxin resistent/sensitive, mannitol negative, lecithinase producing and morphologically Bacillus-like or Staphylococcus-like strains isolated from low fertility soils according to the SBSS screening protocol**

| **MYP** | **polymyxin resistent** | **polymyxin sensitive** | **mannitol negative** | **lecithinase producing** | ***Bacillus-like*** | ***Staphylococcus-*like** | **number of strain** |
|---|---|---|---|---|---|---|---|
| **in total** | 572 | 64 | 121 | 185 | 115 | 14 | 636 |
| **in percentage** (%) | 90% | 10% | 19% | 29% | 18% | 2% | 100% |

Out of the 636 strain that could grow on MYP medium, 572 were polymyxin resistent and 64 were polymyxin sensitive. 121 strains were mannitol negative, 185 strains are able to produce lecithinase. 115 showed *Bacillus-like*, 32 showed *Staphylococcus-*like colony morphology.

### Detection of enteropathogens (primarily Shigella and Salmonella)

### XLD agar medium

| | |
|---|---|
| Yeast extract | 3 g |
| L-lysin | 5 g |
| Xylose | 3,5 g |
| Lactose | 7.5 g |
| Sucrose | 7.5 g |
| Sodium-deoxycholate | 2.2 g |
| Ferric ammonium citrate | 0.8 g |
| Sodium thiosulfate | 6.8 g |
| Sodium chloride | 5 g |
| Agar | 13.5 g |
| Phenol red | 0.08 g |

### XLD medium is selective for enteropathogens, primarily Shigella and Salmonella.

Preparation: 1 litre of distilled water, a watch glass and a measuring spoon are sterilized. 55g of XLD agar is measured out to the watch glass with the spoon and added to the distilled water in a sterile chamber, heated to a boil, and poured to form agar plates.

**Table 11: Number, distribution and classification of xylose, lactose, sucrose negative/positive, hydrogen-sulfide producing or Gram negative facultative pathogen strains isolated from low fertility soils according to the SBSS screening protocol**

| | | | | | | |
|---|---|---|---|---|---|---|
| **XLD** | **no growth** | **xylose/lactose/sucrose positive** | **hydrogen-sulfide producing** | **E. coli, Enterobacter, Aeromonas, Klebsiella, Serratia, Citrobacter, Proteus** | ***Salmonella, Shigella, Pseu domonas*** | **number of strains** |
| **in total** | 502 | 43 | 2 | 42 | 35 | 637 |
| **in percentage (%)** | 79% | 7% | 0,3% | 7% | 6% | 100% |

43 of the studied strains could utilize xylose, lactose or sucrose as a carbon source. These strain are members of the ***E. coli, Enterobacter, Aeromonas, Klebsiella, Serratia, Citrobacter és Proteus*** genes as confirmed by colonial morphology on MC medium. 502 isolates did not grow on XLD medium. 35 strains were typified as Salmonella, Shigella or Pseudomonas, amongst which beneficial Pseudomonas strains can also occur.

### Detection of UV-fluorescent Pseudomonas

| | |
|---|---|
| Peptone | 16.0 g |
| Casein | 10.0 g |
| Potassium-sulfate | 10.0 g |
| Magnesium chloride | 1.4 g |
| Agar | 11.0 g |
| Cetremide | 100.0 mg |
| Nalidixic acid | 7.5 mg |

26 strains of the 324 could be classified as UV-fluorescent Pseudomonas.

### Classification of enteropathogen species

### MacConkey, MC

| | |
|---|---|
| Pancreatic digest of gelatine | 17 g |
| Meat-Peptone | 3 g |
| Lactose | 10 g |
| Bile salts | 1.5 g |
| Sodium chloride | 5 g |
| Agar | 13.5 g |
| Neutral red | 0.03 g |
| Crystal violet | 0.001 g |

Components are added to 1 litre of distilled water, the pH is adjusted to 7.1 and the mixture is boiled until dissolved. The agar medium is sterilized at 120 °C, for 15 min at saturated steam pressure.

Classification of strains according to colony morphology: *Shigella, Salmonella:* colourless, transparent colonies, medium turns yellow; *E.coli:* bigger, red colonies with an opalesque zone, medium turns red; *Enterobacter, Klebsiella:* bigger, pink, mucous colonies; *Enterococci, Staphylococci:* small, not transparent, isolated colonies.

**Table 12: Number, distribution and classification of lactose negative or Gram negative facultative pathogen strains isolated from low fertility soils and cultured in MC medium according to the SBSS screening protocol**

| **MC** | **growth** | ***E.coli*** | ***Proteus, Shigella, Salmonella*** | ***Enterobacter, Klebsiella*** | ***lactose negative*** | **Number of strains** |
|---|---|---|---|---|---|---|
| **in total** | 223 | 0 | 12 | 27 | 32 | 632 |
| **in percentage** (%) | 35% | 0% | 2% | 4% | 5% | 99% |

The 223 colonies that showed growth were Gram negative. None of them were identified as E.coli. 12 strains belong to the group of *Proteus, Shigella* and Salmonella, which was also confirmed by culturing on XLD medium. 27 strains were classified in the group of *Enterobacter* and *Klebsiella,* that was also confirmed by XLD agar culturing. 32 of the 632 isolates were lactose negative.

**Table 13.: Number, distribution and classification of strains according to the SBSS screening protocol.**

| identified genera | number of strains | in percentage % (722 isolates) |
|---|---|---|
| *Arthrobacter* | 32 | 4% |
| *Bacillus* | 219 | 30% |
| *Burkholderia* | 49 | 7% |
| *Enterobacter* | 42 | 6% |
| *Yeast* | 21 | 3% |
| *Lysinibacillus* | 30 | 4% |
| *Micrococcus* | 9 | 1% |
| *Paenibacillus* | 25 | 3% |
| *Pseudomonas* | 81 | 11% |
| *Staphylococcus* | 14 | 2% |
| *Streptomyces* | 24 | 3% |
| in total | 546 | 76% |
| unidentified strains | 176 | 24% |

Strains isolated selectively and non-selectively could be classified to 11 genera. 24% of the 722 strains was unidentified. The majority of the strains (30%) belong to the Bacillus genus. Pseudomonas and Burkholderia spp. were also isolated in a relatively higher proportion.

### Example No.7.: Description and in vitro effect of nitrogen-fixation capacity of cold- and / or salt-tolerant strains isolated via cold treatment or sodic-saline enrichment to plant root and stem mass

According to the NSBSS screening protocol, the following strains were isolated:
Strain B41/8: referred to as B41 : *Azospirillum largimobile*
average nitrogen fixation capacity (Et %/10⁷ CFU/ml); 45.6; microaerophilic
Strain B146: referred to as NF2 *Leclercia adecarboxylata*
average nitrogen fixation capacity (Et %/10⁷ CFU/ml); 39.4; aerobic
Strain B149: referred to as NF3 *Enterobacter cloacae subsp. dissolvens;*
average nitrogen fixation capacity (Et %/10⁷ CFU/ml); 49%, microaerophilic
Strain B162/2-1: referred to as NF4 *Leclercia adecarboxylata*
average nitrogen fixation capacity (Et %/10⁷ CFU/ml); 42.7, microaerophilic
Strain B167
average nitrogen fixation capacity (Et %/10⁷ CFU/ml); 33.5, microaerophilic
Strain B159: referred to as NF14 *Leclercia adecarboxylata*
average nitrogen fixation capacity (Et %/10⁷ CFU/ml); 39.8; aerobic
**Strain 242/9 törzs: referred to as 242/9**
   - species: *Azospirillum brasilense* - based on 16S rRNA gene partial sequencing
   - isolated from: grass root surface
   - average nitrogen fixation capacity (Et %/10⁷ CFU/ml): 53.9, microaerophilic
**Strain 242/10-3: referred to as NF1**
   - species: *Azospirillum brasilense* - based on partial sequencing of the 16S rRNA gene
   - isolated from: grass root surface
   - average nitrogen fixation capacity (Et %/10⁷ CFU/ml): 49.9, microaerophilic
   - Strain NF1 induces a medium rate improvement in root growth (root mass) as a single inoculant. In combination with Bacillus circulans, a considerable increase is observed in root and stem mass. Exhibits active nitrogen-fixation in the rizosphere of the inoculated plants (Et %/0.5 cm of soil associated rizospheric root): 63,1
**Strain 641/1-1: referred to as NF5**
   - species: *Azospirillum lipoferum* - based on partial sequencing of the 16S rRNA gene
   - isolated from: Northern wild rice rizospheric soil and roots
   - cold-tolerant (+10°C)
   - average nitrogen fixation capacity (Et %/10⁷ CFU/ml): 49,9; microaerophilic
   - Exhibits active nitrogen-fixation in the rizosphere of the inoculated plants (Et %/0.5 cm of soil associated rizospheric root): 44,9.
**Strain 638/1-1: referred to as NF6**
   - species: *Azospirillum irakense* based on partial sequencing of the 16S rRNA gene
   - isolated from: Northern wild rice rizospheric soil and roots
   - cold-tolerant (+10°C)
   - average nitrogen fixation capacity (Et %/10⁷ CFU/ml): 49,9; microaerophilic

Exhibits active nitrogen-fixation in the rizosphere of the inoculated plants (Et %/0.5 cm of soil associated rizospheric root): 51,1.

### Strain 616/1-4: referred to as NF7

- species: *Azospirillum brasilense* based on partial sequencing of the 16S rRNA gene
- isolated from: Northern wild rice rizospheric soil and roots
- cold-tolerant (+10°C)
- average nitrogen fixation capacity (Et %/10⁷ CFU/ml): 49,9; microaerophilic

Exhibits active nitrogen-fixation in the rizosphere of the inoculated plants (Et %/0.5 cm of soil associated rizospheric root): 38,8.

### Strain 453/7: referred to as NF10

- species: *Azospirillum brasilense* based on partial sequencing of the 16S rRNA gene
- isolated from: Northern wild rice rizospheric soil and roots
- cold-tolerant (+10°C)
- average nitrogen fixation capacity (Et %/10⁷ CFU/ml): 65,1; microaerophilic

### Strain 672/1-1: referred to as NF11

- species: *Azospirillum brasilense* - based on partial sequencing of the 16S rRNA gene
- isolated from: lowest internodium of the stem and root neck region of Northern wild rice without surface desinfection
- average nitrogen fixation capacity (Et %/10⁷ CFU/ml): 49,9; microaerophilic
- **capable of growth, nitrogen-fixation and indole acetic acid production at slightly acidic conditions (pH 6-7)**
- NF11: considerable, near-significant improvement in root mass, dose-dependent effect

### Strain 672/3-1: referred to as NF12

- species: *Pseudomonas geniculata* based on partial sequencing of the 16S rRNA gene
- isolated from: lowest internodium of the stem and root neck region of Northern wild rice without surface desinfection
- average nitrogen fixation capacity (Et %/10⁷ CFU/ml): 42,3 ; microaerophilic

Phylogenetic classification of *Pseudomonas geniculata* was changed in 2000, currently classified in the *Stenotrophomonas* rRNA lineage (Anzai et al., 2000, Int. J. Syst. Evol. Microbiol., 50 : 1563-1589).

### Strain 672/5-1: referred to as NF15

- species: not identified
- isolated from: lowest internodium of the stem and root neck region of Northern wild rice without surface desinfection
- average nitrogen fixation capacity (Et %/10⁷ CFU/ml): 49,9 ; microaerophilic

### Example No.8: Inoculation of alisol and arenosol type soils with stress-tolerant soil bacterial strains and detection of growth by culturing

### In vitro inoculation of low fertility, acidic soils with abiotic stress-tolerant Azospirillum brasilense

The aim of the study is to monitor the growth of the diazotroph acidic pH-tolerant *Azospirillum brasilense* NF7 strain in low fertility acidic soils by culturing methods to predict growth rate in on-site inoculation in acidic soil conditions. Acidic clayey soil was used with a pH_{KCl} of 4.2, originating from Nagyrákos, Vas county, Hungary.

Strain NF7: The strain has a narrow range of pH tolerance, the optimal pH level for for growth is 4.2 (in NB medium). Based on observations in NB medium, we have studied the growth pattern of the strain in acidic Okon media, to determine the optimal pH for proliferation. In contrast to the NB medium, the Okon medium contains no sugars and the medium is buffered. The optimal pH for growth in the medium was pH 6.0 ± 0.5. The strain had a buffering effect that increased the pH of the culture medium in spite of the added buffers. On the basis of studies on the growth of the strain at different levels of acidic pH in OK medium, we found it reasonable to monitor the rate of growth in vitro in acidic soil.

A reference strain (not stress-tolerant) was cultured in parallel: *A. brasilense* KE1 NCAIM (P), B 001324 that grows optimally in a neutral (pH 6.8-7.0) medium.

Sterilized soil was prepared in 200 ml culturing pots, holding approximately 150 g of soil/pot. Sterility was checked by streaking soil suspension on OK medium.
Inoculant cultures
NF7: overnight shaken cultures incubated at 28 °C in OK medium, cell count approx. 1-2 × 10⁹ CFU/ml
KE1: overnight shaken cultures incubated at 28 °C in OK medium, cell count approx. 1-2 × 10⁹ CFU/ml inoculation dose: 1-2 × 10⁷ CFU/g soil
Samplings (Day0: inoculation)
   1. Day1
   2. Day10
   3. Day16
   4. Day38
Cell counting of samples were measured in OK medium.

**Table 14: Cell count of KE1 and NF7 at the time of samplings in a 38 day-interval after inoculation**

| | Sampling | CFU/5 g of soil | | | |
|---|---|---|---|---|---|
| | | | NF7 | | KE1 |
| | 1. | | 2.5 × 10⁷ | | 1.7 × 10⁷ |
| | 2. | | 2.0 × 10⁶ | | 1.0 × 10⁶ |
| | 3. | | 8.0 × 10⁵-1.0 × 10⁶ | | 8.0 × 10⁶ -1.0 × 10⁷ |
| | 4. | | 8.0 × 10⁵-1.0 × 10⁶ | | ≤ 1.0 × 10⁵ |

Figure 1. displays that cell count of the NF7 strains on Day10 was only 10% of the inoculation dose, and has maintained a cell count around 10⁶ CFU/5g of soil until Day38. (Cell count on Day 1 in considered as the inoculation dose.)

The cell count of the KE1 strain showed a drop - similar to strain NF7 - in the first 10 days, followed by rapid proliferation reaching the inoculation dose at Day16. The cell count has again decreased, at Day38 it was only 10⁵ CFU/5g of soil. It is an order of magnitude lower than the cell count of NF7 at the same time point.

The drop in cell count of 10-day samples is presumably a result of adaptation of inoculant cultures, it is generally observed in similar experiments. A temporary increase in the cell count of the KE1 strain is possibly due to the utilization of intercellular nutrient storages.

Colonies on the OK agar plates used for cell counting show gradually increasing mucus production, possibly a reaction against the inappropriate medium. Mucus production was not observed in NF7 colonies.

Results confirm the increased viability and colonization abilities of the acidic pH-tolerant NF7 strain over that of the KE1 strain that prefers neutral pH. The ability to sustain a cell count of 10⁶ CFU/5g of soil for a 38-day interval after inoculation is an exploitable property. The results of the experiment confirms that the strain is suitable for field use in acidic soil pH.

### Inoculation of dystric arenosol type soils with abiotic stress-tolerant exopolysaccharide producing strains

We have selected two of the Gram negative and one Gram positive not spore-forming, polysacharide producing strains, - presuming that exopolysaccharide production sustains draught tolerance of bacteria that do not form endospores - : *Leuconostoc mesenteroides* K2009 25/4 NCAIM 001372, *Pseudomonas chlororaphis* K2009 13/4 B NCAIM 001370 and *Pseudomonas chlororaphis* K2009 9/4-B NCAIM 001371. Two types of desert sand samples from Dubai - arensol type potting soil and desert sand, pH 5.5 - were inoculated with the cultures of the strains (1 ml of culture/ 10 g of soil, 4-5 × 10⁹ CFU/ml). Samples were incubated at 38 °C for 14 days (till exsiccation), then sustained at 35 °C for another 14 days. Persistence of strains was determined on the basis of viable cell counts (plate count method), displayed in Figure 2. and 3.

In the potting soil (Figure 2.) the *Pseudomonas* strains showed active proliferation, reaching a cell count of 8.1 × 10⁸ CFU/g of soil by the end of the incubation period. Cellular polysacharides of *Leuconostoc mesenteroides* K2009 25/4 NCAIM 001372 and / or *Pseudomonas chlororaphis* K2009 13/4 B NCAIM 001370 provide mucilage to adhere soil particles and within these soil aggregates moisture content and temperature is provided for the continuous biological degradation of the adhesive material and cell proliferation.

The exopolysaccharide produced by *Ps. chlororaphis* promotes the formation of soil aggregates that are resistent against drying out, this way the soil that is apparently dried out still holds enough water to sustain bacterial life - e.g the growth of *Pseudomonas.* Consequently, *Ps. chlororaphis*13/4 B is able to proliferate and enrich microbial activity in arenosol type soils.

Surprisingly, in the desert sand from Dubai (Figure 3.) the exopolysaccharide producing strains showed a high survival rate. *Leuconostoc mesenteroides* K2009 25/4 NCAIM 001372 was present in a cell count of 4.1 × 10⁴ CFU/g of soil and 2.5 × 10²CFU/g of soil on the 14th and 30th day after inoculation, respectively. Apparently, the cell count has exceeded that of the native microbiota (1.3 × 10² CFU/g of soil) by two orders of magnitude, for a period of at least two weeks. Phosphate-solubilizing *Pseudomonas chlororaphis* K2009 9/4-B NCAIM 001371 also sustained its viability (4.4 × 10³ CFU/g of soil). It is likely that polysacharides of the Leuconostoc cell walls promote the viability and growth of Ps. chlororaphis K2009 9/4-B, suggesting that synergy exists between the two strains.

Dextrane and succinoglucane polysacharides of *Pseudomonas chlororaphis* K2009 13/4 B and 9/4B and *Leuconostoc mesenteroides* K2009 25/4 can potentially promote soil formation in desert pioneer soils or deteriorated soils (terra fusca) - where the degrading capacity of the species-poor microbial population is low.

### Studies on the colonizing abilities of strain B41 in strongly acidic clayey soil

Soil colonizing ability and viability of the B41 strains was tested against the reference strain *A*. *brasilense* NF11 that prefer neutral pH conditions for growth. Sustaining a 10⁶ CFU/ 5 g of soil cell count through a 140-day period from the time of inoculation was a favourable ability. Practically, 140 days is the length of a spring-summer vegetation period.
Soil sample: clayey soil with acidic pH (pH_{KCl} 4,37) originating from Vas county
Similarly to the previous study, 200 ml culturing pots were used (approx. 150 g soil/pot). Tyndallized soil was used.

### Inoculant strains:

*A. largimobile* B41 strain: the alkalifying capacity of the strain is high, the optimal pH level of growth is pH 8 in vitro.
*A. brasilense* NF11 strain: the optimal pH level for growth is neutral pH 6,8 - 7,0.

### Inoculant cultures:

B41: overnight shaken cultures incubated at 28 °C in OK medium, cell count approx. 1-2 × 10⁹ CFU/ml
NF11: overnight shaken cultures incubated at 28 °C in OK medium, cell count approx. 1-2 × 10⁹ CFU/ml
Inoculation dose was set at approx. 1-2 × 10⁷ CFU/ml. 2 inoculations were done per each strain.
Time of samplings (Day0: inoculation):
   1. Day1
   2. Day8
   3. Day16
   4. Day28
   5. Day56
   6. Day82
   7. Day147
Cell count was measured on Okon medium (plate count method).

**Table 15. Average cell count of B41 and NF11 strains at the time of samplings**

| Sampling | | Average CFU/5 g of soil | |
|---|---|---|---|
| | | B41 | NF11 |
| | | | |
| 1. | | 3 × 10⁵ | 7 × 10⁶ |
| 2. | | 6 × 10⁶ | 2 × 10⁶ |
| 3. | | 1 × 10⁶ | 1 × 10⁶ |
| 4. | | 1 × 10⁶ | 9 × 10⁵ |
| 5. | | 9 × 10⁵ | 5 × 10⁴ |
| 6. | | 9 × 10⁵ | < 10³ |
| 7. | | 1 × 10⁶ | < 10³ |

Cell count on Day1 is considered as the inoculation dose.

The B41 culture was inoculated in a dose one order of magnitude lower than the NF11 strain. This difference is possibly a consequence of flocculation in the liquid inoculant culture which influenced the proper dose-adjustment.

Strain B41 is proliferating till Day8 then a slight decrease in cell count is observed till Day16 and that level is maintained a 140-day interval.

The cell count of NF11 gradually decreased from the time of inoculation. On Day28, it was an order of magnitude lower, on Day56, it was nearly two orders of magnitude lower than at the time of inoculation. After 82 days, cell count has dropped below the detection limit (<10³ CFU/5g of soil) (Figure 12.)

### Example No.9: Phosphate solubilization

The phosphate solubilizing activity can be detected by culturing on hydroxyl-apatite containing medium according to Example and the SBSS screening protocol. The principle of detection is that the medium opalesque of the insoluble phosphate clears up as a result of phosphate solubilization, in the form of a transparent zone around the colonies (Sundara and Sinha, 1963; Varam and Mathur, 1989; Kopoor, 1989). Phosphate solubilizing properties of strains were studied on modified Pikovskaya medium containing 1% hydroxylapatite and supplemented with tricalcium-phosphate and a carbon source. If the strain is capable of solubilizing both forms of phosphate, it bears good phosphate solubilizing properties.

**Table 16: Phosphate solubilizing capacity and pathogenity of abiotic stress-tolerant strains**

| **Phosphate solubilization** | +++ | ++ | + | - | **number of studied strains** |
|---|---|---|---|---|---|
| **in total** | 1 | 36 | 155 | 444 | 636 |
| **in percentage** (%) | 0% | 6% | 24% | 70% | 100% |
| **pathogens** | 1 | 1 | 29 | 16 | 47 |
| **pathogens in percentage** (%) | 100% | 3% | 19% | 4% | 7% |

30% of the strains is capable of phosphate mobilization, to a different degree. The optimal phosphate solubilization was shown by strain S298, yet, genetical identification revealed its pathogenity (*Burkholderia stabilis*)*.* 36 strains have a good phosphate solubilizing capacity: ES1, ES2, ES3, ES10, ES11, ES17, ES18, ES19, ES22, ES23, ES24, ES25, ES26, ES27, ES28, ES29, ES30, ES31,ES33, ES35, ES36, ES41, ES176, ES187, ES282, ES283, **S25, S30, S33, S34, S37, S47, S125, S153, S275, S301,** LU36, LU44, LU47, 13/4B. Pathogen strains are most prevalent (19%) among strains with a poor phosphate solubilizing capacity (+).

*Pseudomonas chlororaphis* K2009 13/4 B NCAIM 001370a, *Pseudomonas chlororaphis* K2009 9/4-B NCAIM 001371 and *Leuconostoc mesenteroides* K2009 25/4 NCAIM 001372 strains were tested besides positive control *Bacillus megaterium var. phosphaticum* M326 NCAIM /P/ B 001291. The zone of clearance after 3-4 days incubation at 28 °C on HP agar were compared.

**Table 17: Comparative study on phosphate solubilizing of B. megaterium var.phosphaticum , Ps. chlororaphis K 2009 9/4B and 13/4B and L. mesenteroides K2009 25/4 strains**

| | | Hydroxylapatite zone of clearance | |
|---|---|---|---|
| | *B. megaterium phosphaticum* | +++ | wide zone of clearance |
| | *P. chlororaphis* K2009 13/4 B NCAIM 001370 | +++ | wide zone of clearance |
| | *P. chlororaphis* K2009 9/4-B NCAIM 001371 | ++ | wide zone of clearance |
| | *L. mesenteroides* K2009 25/4 NCAIM 001372 | + | minimal clearance |

The highest phosphate solubilizing activity was observed in *P. chlororaphis* K2009 13/4 B NCAIM 001370 and the reference strain *B. megaterium var. phosphaticum,* the other *Pseudomonas* strain and the *Leuconostoc* strain exhibited lower activity.

### Example No.10: Studies on polysacharide production

Polysacharide production was studied on strains selected by phase contrast microscopy based on the presence of a capsule or viscous substance. The studied strains were culture in Sach II broth.

### Sach II broth for the detection of polysacharide production:

8% saccharose; 0.51% KNO₃; 0.1% K₂HPO₄; 0.08 KH₂PO₄; 0.04% MgSO₄×7H₂O; 0.002% FeSO₄×7H₂O; 0.001% CaCl₂; 0.001% MnSO₄; 0.1% NaCl; 0.01% yeast extract; 0,01% triptone; 0.01% soya peptone; 1ml/l trace element solution, pH before sterilization: 7.0; sterilization at 121° C, for 25 min.

Composition of the trace-element solution: 0.3g H₃BO₃; 0.2g CoCl₂.6H₂O; 0.1g ZnSO₄.7H₂O; 30mg Na₂MoO₄.2H₂O; 20mg NiCl₂.6H₂O; 10mg CuSO₄.5H₂O, in 1 litre IN HCl.

The studied strains are incubated in 100 ml cultures in the Sach II broth at 28°C, shaken at 300rpm for 7 days. Cell count of the inoculum: at least 10⁹ CFU/ml, inoculation rate 3%.

After incubation, the cultures are centrifuged for 30 min, at 9000 rpm. The supernatant is diluted to twice its volume with distilled water and shaken at 40 °C for 2h. 2-propanol is added to the mixture in three times the amount of the supernatant. In case of EPS production, the propanol precipitates the extracellular polysacharides.

9 strains were capable of propanol-precipited polysacharide production: ES 17, S 17, S 28, S 62, S 120, S 127, 13/4 B, 25/4, S284

The type of produced polysacharide was determined by inoculation onto Calcofluor medium and incubation overnight. The Calcofluor is binded by the polysacharides - if present - and fluoresces under UV light as a part of the UVP Biodoc-It documentation system. If the produced polysacharide is diffusable (e.g. exopolysaccharide) the medium around to colines also emit light. If the polysacharide is absorbed to the cellular wall, only the colonies fluoresce. 2 of the studied strains gave a considerable calcofluor reaction: *Pseudomonas chlororaphis* K2009 13/4 B NCAIM 001370 and *Leuconostoc mesenteroides* K2009 25/4 NCAIM 001372.

### Example No.11: Detection of siderophore production and selection of strains

Siderophore production was detected by the method of Schwyn and Neilands on King's B solid medium containing chrome-azurol. The siderophores are detected based on their high affinity to ferric(III)-ions, irrespectively of their structure.

A strong ligand is added to the bright-coloured iron-dye complex. The binding of the iron-ligand complex releases the dye as indicated by the change of colour (appearance of an orange colour). 5 uL of the cultures of siderophore strains - cultured in liquid King's B medium for no more than 24h - are inoculated in wells of an agar plate. After 48h incubation at 28°C, the diameter of the orange zone on the originally blue medium is evaluated (Oldal, 2002).

### Measurement method for siderophores

### Siderophore medium

| | |
|---|---|
| 6,0 g | piperazine- *N*,*N-*bis (2-ethanesulfonic acid) |
| 0,6 g | NaOH, |
| 15,0 g | protease-pepton, |
| 15,0 g | MgSO4 × 7 H2O, |
| 1,5 g | K₂HPO₄, |
| 10,0 g | glycerol, |
| 20,0 g | agar. |

The components are added to 900 ml of distilled water, the pH is adjusted to 7.2 and sterilized at 120°C for 20min, at saturated steam pressure.

The following components are filtered and added after sterilization:
60.5 mg Chrome-Azurol S dye in 50.0 ml of distilled water; 10.0 mg FeCl₃ × 6 H₂O dye and 72.9 mg of hexadecyl-trimethyl-ammonium-bromide (HDTMA) in another 50.0 ml of distilled water.

Siderophore production is studied on ChromeAzurol S containing medium.

### Detection and measurement of siderophore producing capacity

Method 1.

24h cultures are inoculated in siderophore medium (see above) and incubated for 72h. The diameter of the rings around the colonies serve as a basis for the comparison of siderophore producing capacity of strains.

Siderophores show high affinity to ferric (III) ions, irrespectively of their structure. Siderophores release the dye from iron-dye complexes due to binding to the iron. The culturing medium around the siderophore-producing strains changes colour from the originally blue to orange (Oldal et al., 2002) (Figure 13.)

The following strains were proven to produce siderophores: *Serratia plymuthica (ER 180), Bacillus megaterium (S 41, ES 217), Paenibacillus peoriae (S* 284), *Pseudomonas frederiksbergensis (S 33), Agreia pratensis (S 47), Arthrobacter crystallopoietes (S 153), Pseudomonas brassicacearum (S 177)* es *Pseudomonas chlororaphis (13*/*4 B és 9*/*4 B),* ***Azospirillum largimobile* B41 NCAIM** (P) B 001402, *Azospirillum irakense* NF6 **NCAIM** (P) B **001425**

**Table 18: Siderophore producing stress-tolerant soil bacteria**

| | |
|---|---|
| ES215 | *Pseudomonas chlororaphis subsp. aurantica* |
| ES217 | *Bacillus megaterium* |
| **S153** | *Arthrobacter crystallopoietes* |
| **S33** | *Pseudomonas frederiksbergensis* |
| **S41** | *Bacillus megaterium* |
| **ER 180** | *Serratia plymuthica* |
| **S 284** | *Paenibacillus peoriae* |
| **S 47** | *Agreia pratensis* |
| **S 177** | *Pseudomonas brassicacearum* |
| **13/4B** | *Pseudomonas chlororaphis* |
| **9/4 B** | *Pseudomonas chlororaphis* |

Siderophore-producing properties were also tested by E.coli inhibition on KingB medium. On iron-free agar medium the isolates inhibit the growth of overspreaded E.coli cultures, to a different extent, creating inhibition zones (King 1984, Kloepper, 1980).

Siderophore production was detected by inoculating the studied strain in King B medium and sprayed with the culture of *E. coli* MC1061. Parallel culturing on iron-containing medium is performed to confirm that the antibiotic effect is contributed only to siderophore production.

*E.coli* inhibition was measured after incubation at 35°C for 48h. Siderophore production was expressed in *Ps. chlororaphis* K2009 9/4-B, while *Ps. chlororaphis* K2009 13/4 B induced strong inhibition on both medium, suggesting that antibiotic effect is not solely due to siderophore production, but also other biocontrolling mechanisms.

**Table 19: Comparison of 2 P. chlororaphis K 2009 strains and L. mesenteroides K2009 25/4 in respect to siderophore producing capacity and inhibitory effect on the growth of E.coli test organism**

| | King B | King B + Fe |
|---|---|---|
| | zone of inhibition | |
| *Ps. chlororaphis* K2009 13/4 B NCAIM 001370 | +++ | +++ |
| *Ps. chlororaphis* K2009 9/4-B NCAIM 001371 | + | - |
| *L. mesenteroides* K2009 25/4 NCAIM 001372 | - | - |

### Example 12.: Selection of strains producing plant-growth promoting indole-3-acetic acid (IAA), cytokinins - t-zeatin and t-zeatin riboside

Selection of indole acetic acid producing strains form aisolates according to he SBSS protocol was done by means of culturing on TSB medium without adding tryptophane. Salkowsky-reagent is added to the samples after centrifugation. The reagent turns red in the presence of indole-3-acetic acid.

Submerse cultures for the measurement of indole-3-acetic acid concentration were prepared as follows: 4-4ml of NB broth is distributed in test tubes, sterilized and inoculated with the strains with an inoculation loop. The cultures are incubated at 28 °C for one day in a rotator. 0.5 ml of the 24h inoculant cultures is inoculated into 4ml of sterile TSB broth. 4 parallel inoculations are done for each strain. The tubes are incubated at 28°C for 96h in a rotator.

For blind samples, 1ml of the NB is added with 2ml of S-reagent. The average of the results of parallel IAA measurements are converted to µg/ml concentration with the help of a calibration curve. The calibration curve is obtained by serial measurements of an IAA dilution series. Concentrations in the dilution series range between 0.1-0.001 µg/ml.

127 (21%) strains of the studied 631 were capable of indole-3-acetic acid production. 10 strains produced IAA in a high concentration, 17 strains showed a moderate, and 100 showed a low rate of production. Out of the 27 strains that proved to have a good IAA-producing capacity, 16 is pathogen. Nearly 60% of all indole acetic acid producing strains are pathogen. Strain with optimal IAA production capacity: ES330, S116, S177, S273, LU44, ES307, ES343, ES344, S33, S47, S125, S153, S225, S158, S162, B41, NF10, NF11, 242/9, NF7, NF6.

**Table 20: Rate of IAA production and pathogenity of IAA producing strains**

| **IAA production** | **OD above 2** | **OD between 1-2** | **OD between 0-1** | **not producing IAA** | **number of studied strains** |
|---|---|---|---|---|---|
| **in total** | 10 | 17 | 100 | 504 | 631 |
| **in percentage** | 2% | 3% | 16% | 80% | 100% |
| **pathogen** | 5 | 11 | 8 | 22 | 46 |
| **pathogen** % | 50% | 65% | 8% | 4% | 7% |

### Detection of auxins and IAA with thin layer chromatography

The NF 6 strain of *A. irakense* was incubated in 100ml modified nutrient broth inoculum in 500 ml Erlenmeyer flasks, shaken at 250 rpm on 28°C for 48h. Aerobic soil microbes were provided with oxygen by shaking, that allowed the cultures to reach the stationer phase of growth in 48h.

20-20 ml of the samples were measured into centrifuge tubes and centrifuged for 10 min at 4500 rpm/rcf (Hermle Z300).

The pH of the centrifuged sampes were adjusted to 2.8 with IN HCl.The samples were extracted with 20-20 ml of ethylacetate in a separatory funnel and dehydrated with anhydrous sodium sulfite. The desiccant was removed by filtration. Excess solvent was removed by a rotatory evaporator, and the crystals were dissolved in 1ml of methanol.

For the detection of indole-derivatives 5553 TLC silica gel 60 plates were used, 5 µl of the samples were spotted by a Hamilton syringe. For the detection of auxins a chloroform:ethylacetate:formic acid solvent system was used. Plates were fixed at 45°C for 5 min, developed by the Ehrlich reagent and fixed at 100°C for 5 min.

### Measurement of indole-3-acetic acid (IAA) via HPLC

### HPLC chromatography

### Auxins were detected from the extracted buthanol phase (Figure 14-15.)

| | |
|---|---|
| Stationary phase: | Kinetex-C18 (2,6 µm particle size, core shell technology) (Phenomenex Inc.) |
| Column dimensions: | 4,6 mm diameter, 50 mm length |

### Mobile phase and solvent development:

| | |
|---|---|
| Start "A" mobile phase: | 0,1 v/v% trifluoroacetic acid (>99% purity, Sigma-Aldrich Ltd.) |
| Limit "B" mobile phase: | acetonitrile 90%/water 10% containing 0,1 v/v% trifluoro-acetic acid (HPLC quality acetonitrile, Sigma-Aldrich Kft.) |
| Gradient programme: | 0-10 min 5%B→100%B, 10-11 min 100B, |
| | 11-12 min 100%B →5%B, 12-20 min 5%B |
| Chromatogram run time: | 12 min |
| Total cycle time (incl. equilibration)): | 20 min |
| Separation temperature: | ambient temperature |
| Chromatography system: | Waters2695 (Alliance) |

### Linear gradient elution, 0,5 ml/min flow rate, 5 µl injected volume

### Waters 996 photodiode array detector, 215 and 254 nm dual channel monitoring wavelengths

### Detection of gibberellin-3-acid and indole-3-acetic acid via TLC

The NF 6 strain of *A. irakense* was incubated in 100ml modified nutrient broth inoculum in 500 ml Erlenmeyer flasks, shaken at 250 rpm on 28°C for 48h. Aerobic soil microbes were provided with oxygen by shaking, that enabled the cultures to reach the stationer phase of growth in 48h.

20-20 ml of the samples were measured into centrifuge tubes and centrifuged for 10 min at 4500 rpm/rcf (Hermle Z300).

The pH of the centrifuged sampes were adjusted to 2.8 with IN HCl.The samples were extracted with 20-20 ml of ethylacetate in a separatory funnel and dehydrated with anhydrous sodium sulfite. The desiccant was removed by filtration. Excess solvent was removed by a rotatory evaporator, and the crystals were dissolved in 1ml of methanol. Gibberellins were detected on 5554 TLC silica gel 60 F₂₅₄ plates using the same solvent system as with the auxins. The plates are fixed at 45C for 5 min and developed in sulphuric acid:ethanol mixture. After fixation at 100°C for 5 min, the gibberellins fluoresce in a greenish-blue colour in 366 nm wavelength UV light (Figure 16.).

### TLC and HPLC detection of cytokinins

The NF 6 strain of *A. irakense* was incubated in 100ml modified nutrient broth inoculum in 500 ml Erlenmeyer flasks, shaken at 250 rpm on 28°C for 48h. Aerobic soil microbes were provided with oxygen by shaking, allowing to reach the stationer phase of growth in 48h.

20-20 ml of the samples were measured into centrifuge tubes and centrifuged for 10 min at 4500 rpm/rcf (Hermle Z300).

### Zeatin and t-zeatin-roboside type cytokinins were detected.

### TLC chromatography

Cytokinin-like compounds were detected from the buthanol extracted phase. Obtained samples were spotted on 5554 TLC silica gel 60 F₂₅₄ plates and placed in the solvent. After fixation at 100 °C, cytokinins were visualized in 254 nm wavelength UV light, Rf.

### HPLC chromatography

Cytokinins - zeatin and t-zeatin-roboside type - were detected from the extracted buthanol phase. (Figure 17.)

| | |
|---|---|
| Stationary phase: | Kinetex-C18 (2.6 µm particle size, core shell technology) (Phenomenex Inc.) |
| Column dimensions: | 4.6 mm diameter, 50 mm length |

### Mobile phase and solvent development:

| | |
|---|---|
| Start "A" mobile phase: | 0.1 v/v% trifluoroacetic acid (>99% purity, Sigma-Aldrich Ltd.) |
| Limit "B" mobile phase: | acetonitrile 90%/water 10% containing 0.1 v/v% trifluoro-acetic acid (HPLC |
| | quality acetonitrile, Sigma-Aldrich Kft.) |
| Gradient program: | 0-10 min 5%B→100%B, 10-11 min 100B, |
| | 11-12 min 100%B →5%B, 12-20 min 5%B. |
| Chromatogram run time: | 12 min |
| Total cycle time (incl. equilibration)): | 20 min |
| Separation temperature: | ambient temperature |
| Chromatography system: | Waters2695 (Alliance) |

### Linear gradient elution, 0,5 ml/min flow rate, 5 µl injected volume

### Waters 996 photodiode array detector, 215 and 254 nm dual channel monitoring wavelengths

### Example No.13.: Growth and indole acetic acid production of wide-pH-tolerant strains on alkaline pH

100ml/500ml Nutrient broth inoculum is prepared from ***Azospirillum largimobile,* B41 NCAIM** (P) B 001402. The flask is inoculated with 1 deep-frozen sample and incubated overnight. 3x8 flasks containing 100-100 ml of Nutrient Broth is are sterilized. The pH of the broths are adjusted to 3,4,5,6,7,8,9 and 10, respectively. An alkaline Nutrient broth is not stable, sterilization reduces pH. The pH levels of 8,9 and 10 are thus adjusted after sterilization. Cultures are processed after 2 days. Purity of the cultures is checked by phase contrast microscopy, and also register any morphological changes. The pH and OD 600 values of strains are measured. (Figure 18.)

100ml/500ml Nutrient broth inoculum is prepared from *Kocuria rosea* S 225 NCAIM (P) B 001426. The flask is inoculated with 1 deep-frozen sample and incubated overnight. 3x8 flasks containing 100-100 ml of Nutrient Broth is are sterilized. The pH of the broths are adjusted to 3,4,5,6,7,8,9 and 10, respectively. An alkaline Nutrient broth is not stable, sterilization reduces pH. The pH levels of 8,9 and 10 are thus adjusted after sterilization. Cultures are processed after 2 days. Purity of the cultures is checked by phase contrast microscopy, and also register any morphological changes. The pH and OD 600 values of strains are measured. (Figures 19 and 20.)

### Example No.14.:Bacterial inhibition of fungal growth in dual culture tests (bacterial lawn, fungal agar disc in the center)

Maintenance of fungal strains: Potato Dextrose Agar (PDA5) pH 5.0. Transfer to PDA6 (pH6) or Triptone-soy-agar (TSA,6, pH6) plates 10-14 days prior to dual culturing, incubation at ambient temperature.

Maintenance of bacterial strains: sugar supplemented nutrient plates (NC). Transfer to NC plates in the week prior to dual culturing, incubation for 24-48h.

Preparations of dual culturing: bacterial strains are inoculated in 10 ml PDA6 or TSA6 broth and incubated at 28°C overnight, shaken. A serial dilution is prepared from the incubated cultures and streaked on PDA6 or TSA6 plates (4 plates in parallel for each dilution).

Agar discs 0.6 mm in diameter are cut from the fungal PDA6 or TSA6 plates and placed in the center of the bacterial and uninoculated control plates in a sterile chamber. In case of *Sclerotina sclerotiorum,* a sclerotium is placed in the center of bacterial and control plates. The plates are covered and incubated at room temperature. The growth of fungal hyphae are monitored daily for 7 days, then once in a week.
Evaluation: the average is derived from the growth rates observed in the four parallel cultures and compared to control (the difference is given in proportion of the control value). Figures 21-28. display that due to the effect of bacterial cultures (plates B,C,D,E) the diameter of the zone of fungal growth around the disc is varying, which is compared to uninoculated control (plate A). The degree of inhibition correlates with the degree of dilution.

### Example No.15.: Selection of ACC-deaminase producing PGPR strains

According to Example 1 and the SBSS screening system, ACC-deaminase production is detected by culturing firstly in nitrogen-rich medium followed by culturing in minimal medium containing ACC as a N-source.

### Culturing conditions to induce ACC deaminase activity (Penrose and Glick, 2003)

As a nitrogen-rich medium, Triptone Soy Broth is a good option. Each strain is inoculated in 2x7.5 ml broth. The strains are cultured to reach mid-log or late-log phases of growth in shaken cultures (200 rpm, overnight, adequate temperature).

The cell suspensions (the 2 × 7.5 ml are united) are centrifuged at 8000g for 10 min at 4°C. The supernatant is poured off, the cells are washed in 5 ml of DF medium followed by centrifugation at 8000g for 10 min at 4°C. The pellet is suspended in 7.5 ml DF medium and added with 45 µl of 0.5M ACC solution (end concentration 3.0 mmol) and incubated in shaken cultures as described earlier, for 24 h. The obtained cell suspension is centrifuged at 8000g for 10 min at 4°C.

The supernatant is poured off, the pellet is washed with 5ml of 0.1 mol Tris-HCl (pH 7,6) in case of ACC deaminase activity measurements or in 0.03 MgSO4 for HPLC or plant inoculation. The washing is repeated twice. The pellet is stored at -20°C until deaminase determination or at 4°C in case of HPLC or plant studies.

### Enzyme activity measurements

The enzyme activity is determined according to the method of Honma and Shimomura (1978). The enyzme hydrolyses ACC to alpha-ketobutyrate. the amount of alpha-ketobutyrate is calculated from absorption at 540 nm, as referred to the alpha-ketobutyrate absorption calibration scale, ranging from 0.1-1.0 mmol.

100 mmol alpha-ketobutyrate strain solution is prepared in 0.1 mol 1mol Tris -HCl pH 8,5 buffer and stored at 4°C-on tároljuk. Prior to use, the strain solution is diluted with the buffer used for the preparation.

Samples of the dilution series: 200 µl of the diluted alpha-ketobutyrate is added with 300 µl 2,4-dinitrophenyl-hydrazine reagent (0.2% 2,4-dinitrophenyl-hydrazine in 2 mol HCl), vortexed and incubated at 30°C for 30min. The colour of the formed phenylhydrazone is developed with 2.0 ml 2.0 mol NaOH, vortexed and absorption at 540nm is measured.

The pellets prepared as described earlier are suspended in 1.0ml 0.1 mol Tris-HCl pH 7.6. The suspension is centrifuged at 16,000g for 5min, the supernatant is poured off, and the pellet is resuspended in 600 µl 0.1 mol Tris-HCl pH 7.6.

30 µl toluene is added to the suspension and vortexed for 30 sec at the highest rpm.

100 µl aliquot toluene-added suspension is separated for protein content determination and stored at 4°C.

200 µl of the rest of the toluene-added suspension is added with 20 µl 0.5 mol ACC solution, vortexed and and incubated at 30°C for 15min. 1ml 0.56 mol HCl is added and the suspension is centrifuged at 16000g for 5 min at room temperature.

1.0 ml of the supernatant is added with 800 µl of 0.56 mol HCl and 300 µl 2,4- dinitrophenyl-hydrazine reagent (0.2% 2,4-dinitrophenyl-hydrazine in 2 mol HCl) and incubated at 30°C for 30 min.2.0 ml 2 mol NaOH is added, and absorption is measured at 540nm.

Samples prepared without the addition of ACC serve as blind samples (control).

The following strains were chosen as ACC deaminase positive controls:
*Enterobacter cloacea* NF3 diazotroph strain- ACC deaminase activity: 1-3mM alpha-ketobutyrate/10¹⁰ CFU
*Pseudomonas fluorescens* NCAIM B01677 - ACC deaminase activity: 3-5 mM alpha-ketobutyrate/10¹⁰ CFU

The following strains were measured for ACC-deaminase activity
13/4 *Pseudomonas chlororaphis* - 5 mM alpha-ketobutyrate/mg/h, intense colour reaction
S177 *P.brassicacearum* - no detectable enyzme activity
NF6 *Azospirillum irakense* - < 1 mM alpha-ketobutyrate/mg/h
NF10 *Azospirillum brasilense* - < 1 mM alpha-ketobutyrate/mg/h
NF11 *Azospirillum brasilense* - < 1 mM alpha-ketobutyrate/mg/h
S125 *Pseudomonas jessenii* - < 1 mM alpha-ketobutyrate/mg/h
S33 *Pseudomonas frederiksbergensis* - < 1 mM alpha-ketobutyrate/mg/h
LU44 *Exiguobacterium acetylicum* - < 1 mM alpha-ketobutyrate/mg/h

The ACC deaminase activity of PGPR is highly variable. Even a very low - approx. 20 nmol alpha-ketobutyrate/mg/h - ACC deaminase activity can promote growth and metabolic activity in the presence of ACC as a source of nitrogen. Strains with a higher enyzme activity do not necessarily exert a higher growth promoting effect on e.g root elongation, than strains with a lower ACC deaminase activity. Besides IAA production, strains NF 6, NF 10, NF11, S125, S33 and LU44 also exert measurable ACC deaminase activity which improves their efficiency in plant-growth promotion.

### Example No.16.: Classification of potential soil inoculant strains optimal in plant growth promotion, stress-tolerance and soil formation

Strains were classified according abiotic stress-tolerance (ST), plant growth promoting (PGPR) and soil aggregation forming (AP) potential, with potentially human pathogen strains excluded from classification.

The strains with the widest ranges of abiotic stress tolerance (STB: Stress Tolerant Bacteriaceae) were selected (60 strains). 14 of them proved to be potentially human pathogen. 43 strains had optimal salt-, acidic pH- and cold tolerance. Some of them have a wide range of pH tolerance, showing maximal growth at all pH levels and excellent salt tolerance, growing at 7% and 8% NaCl concentrations.

**Table 21: STB strains with optimal stress tolerance accoring to the SBSS screening system 92 strains with optimal plant growth promoting properties were selected (PGPR-AP-B:Plant Growth Promotion Rhizobacteriaceae and Aggregate Promotion Bacteriaceae). Some of these strains are diazotroph, produce exopolysaccharide, capable of phosphate solubilization and indole-3-acetic acid production.**

| strain | | | |
|---|---|---|---|
| /4B | alkaline pH optimum | -2 % NaCl | |
| /9 | alkaline pH optimum | -2 % NaCl | |
| B41 | alkaline pH optimum | | |
| ES202 | acidic pH optimum | -8 % NaCl | cold tolerant |
| ES204 | acidic pH optimum | -8 % NaCl | cold tolerant |
| ES207 | alkaline pH optimum | -8 % NaCl | cold tolerant |
| ES209 | acidic pH optimum | -8 % NaCl | cold tolerant |
| ES211 | acidic pH optimum | -8 % NaCl | cold tolerant |
| ES216 | alkaline pH optimum | -8 % NaCl | cold tolerant |
| ES228 | neutral pH optimum | -8 % NaCl | cold tolerant |
| ES231 | acidic pH optimum | -8 % NaCl | cold tolerant |
| ES232 | alkaline pH optimum | -8 % NaCl | cold tolerant |
| ES233 | neutral pH optimum | -8 % NaCl | cold tolerant |
| ES235 | neutral pH optimum | -8 % NaCl | cold tolerant |
| Es236 | acidic pH optimum | -8 % NaCl | cold tolerant |
| ES249 | alkaline pH optimum | -8 % NaCl | cold tolerant |
| ES253 | acidic pH optimum | -8 % NaCl | cold tolerant |
| ES266 | acidic pH optimum | -8 % NaCl | cold tolerant |
| ES272 | alkaline pH optimum | -8 % NaCl | cold tolerant |
| ES274 | alkaline pH optimum | -8 % NaCl | cold tolerant |
| ES275 | alkaline pH optimum | -8 % NaCl | cold tolerant |
| ES283 | acidic pH optimum | -8 % NaCl | cold tolerant |
| ES289 | neutral pH optimum | -8 % NaCl | cold tolerant |
| ES297 | acidic pH optimum | -8 % NaCl | cold tolerant |
| ES305 | acidic pH optimum | -8 % NaCl | cold tolerant |
| LU19 | acidic pH optimum | -8 % NaCl | cold tolerant |
| LU22 | acidic pH optimum | -8 % NaCl | cold tolerant |
| LU25 | acidic pH optimum | -8 % NaCl | cold tolerant |
| LU44 | wide pH tolerance | -8 % NaCl | cold tolerant |
| LU5 | neutral pH optimum | -8 % NaCl | cold tolerant |
| LU56 | neutral pH optimum | -8 % NaCl | cold tolerant |
| NF10 | alkaline pH optimum | -5 % NaCl | cold-selected |
| NF11 | alkaline pH optimum | -2 % NaCl | |
| NF6 | neutral pH optimum | -5 % NaCl | cold-selected |
| NF7 | acidic and neutral pH optimum | -5 % NaCl | cold tolerant |
| S125 | neutral pH optimum | -5 % NaCl | cold tolerant |
| S153 | alkaline pH optimum | | |
| S225 | neutral pH optimum | | |
| S28 | acidic pH optimum | | |
| S284 | acidic pH optimum | -2 % NaCl | |
| S314 | neutral pH optimum | -8 % NaCl | cold tolerant |
| S33 | acidic pH optimum | -8 % NaCl | cold tolerant |
| S47 | acidic pH optimum | -2 % NaCl | cold tolerant |

**Table 22: Strains with optimal plant growth promoting (PGPR) and soil aggregate promoting (AP) properties according to the SBSS screening system (Plant Growth Promotion Rhizobacteriaceae and Aggregate Promotion Bacteriaceae)**

| strain | nitrogen-fixation | phosphate solubilization | indole-3-acetic acid production | exopolysaccharide (EPS) production | siderophore production |
|---|---|---|---|---|---|
| 13/4B | | phosphate solubilizing | | polysacharide production | siderophore production |
| 9/4 | | phosphate solubilizing | | polysacharide production | siderophore production |
| NF7 | nitrogen-fixing | | IAA producing | | |
| 242/9 | nitrogen-fixing | | IAA producing | | |
| B41 | nitrogen-fixing | | IAA producing | | |
| ES1 | | phosphate solubilizing | | | |
| ES10 | | phosphate solubilizing | | | |
| ES11 | | phosphate solubilizing | | | |
| ES17 | | phosphate solubilizing | | polysacharide production | |
| Es176 | | phosphate solubilizing | | | |
| ES18 | | phosphate solubilizing | | | |
| Es187 | | phosphate solubilizing | | | |
| ES19 | | phosphate solubilizing | | | |
| ES2 | | phosphate solubilizing | | | |
| ES200 | | phosphate solubilizing | IAA producing | | |
| ES217 | | phosphate solubilizing | IAA producing | | siderophore production |
| ES218 | | phosphate solubilizing | IAA producing | | |
| ES22 | | phosphate solubilizing | | | |
| ES222 | | phosphate solubilizing | IAA producing | | |
| ES223 | | phosphate solubilizing | IAA producing | | |
| ES23 | | phosphate solubilizing | | | |
| ES231 | | phosphate solubilizing | IAA producing | | |
| ES232 | | phosphate solubilizing | IAA producing | | |
| ES24 | | phosphate solubilizing | | | |
| ES25 | | phosphate solubilizing | | | |
| ES26 | | phosphate solubilizing | | | |
| ES27 | | phosphate solubilizing | | | |
| ES28 | | phosphate solubilizing | | | |
| ES282 | | phosphate solubilizing | | | |
| ES283 | | phosphate solubilizing | | | siderophore production |
| Es29 | | phosphate solubilizing | | | |
| ES3 | | phosphate solubilizing | | | |
| ES30 | | phosphate solubilizing | | | |
| ES305 | | phosphate solubilizing | IAA producing | | siderophore production |
| ES307 | | | IAA producing | | |
| ES308 | | phosphate solubilizing | IAA producing | | siderophore production |
| ES31 | | phosphate solubilizing | | | |
| ES33 | | phosphate solubilizing | | | |
| Es35 | | phosphate solubilizing | | | |
| ES36 | | phosphate solubilizing | | | |
| Es41 | | phosphate solubilizing | | | |
| LU16 | | phosphate solubilizing | IAA producing | | |
| LU36 | | phosphate solubilizing | | | |
| LU44 | | phosphate solubilizing | IAA producing | | |
| LU45 | | phosphate solubilizing | IAA producing | | |
| LU47 | | phosphate solubilizing | | | |
| NF10 | nitrogen-fixing | | IAA producing | | |
| NF11 | nitrogen-fixing | | IAA producing | | |
| NF6 | nitrogen-fixing | | | | |
| NF7 | nitrogen-fixing | | IAA producing | | |
| S1 | | phosphate solubilizing | IAA producing | | |
| S102 | | phosphate solubilizing | IAA producing | | |
| S116 | | | IAA producing | | |
| S120 | | phosphate solubilizing | IAA producing | polysacharide production | |
| S123 | | phosphate solubilizing | IAA producing | | |
| S125 | | phosphate solubilizing | IAA producing | | |
| S127 | | | | polysacharide production | |
| S 114 | | | | | siderophore production |
| S142 | | phosphate solubilizing | IAA producing | | |
| S 202 | | | | | siderophore production |
| S145 | | phosphate solubilizing | IAA producing | | |
| S153 | | phosphate solubilizing | IAA producing | | siderophore production |
| S158 | | | IAA producing | | |
| S162 | | | IAA producing | | |
| S17 | | phosphate solubilizing | IAA producing | polysacharide production | |
| S172 | | phosphate solubilizing | IAA producing | | |
| S177 | | | IAA producing | | siderophore production |
| S182 | | phosphate solubilizing | IAA producing | | |
| S187 | | phosphate solubilizing | IAA producing | | |
| S197 | | | | | |
| S22 | | phosphate solubilizing | IAA producing | | |
| S225 | | | IAA producing | polysacharide production | |
| S25 | | phosphate solubilizing | | | |
| S273 | | | IAA producing | | |
| S275 | | phosphate solubilizing | | | |
| S47 | | phosphate solubilizing | IAA producing | | siderophore |
| | | | | | production |
| S28 | | | | polysacharide production | |
| S284 | | | | polysacharide production | siderophore production |
| S30 | | phosphate solubilizing | | | |
| S301 | | phosphate solubilizing | | | |
| S33 | | phosphate solubilizing | IAA producing | | siderophore production |
| S34 | | phosphate solubilizing | IAA producing | | |
| S37 | | phosphate solubilizing | IAA producing | | |
| S41 | | phosphate solubilizing | IAA producing | | siderophore production |
| S46 | | phosphate solubilizing | IAA producing | | |
| S62 | | | | polysacharide production | |
| S85 | | phosphate solubilizing | IAA producing | | |
| S93 | | phosphate solubilizing | IAA producing | | |
| S96 | | phosphate solubilizing | IAA producing | | |

192 of the studied 636 strains are capable of phosphate solubilization, 36 of them are not pathogen and well solubilizes insoluble hydroxylapatite (HP). 89 of the p1 pathogen starins are phosphate solubilizing.

**127 of 631 strains are capable indole-3-acetic acid production, 37 of which is not pathogen and has a good hormone-producing capacity. All 91 of the 91 pathogen strains produce indole-3-acetic acid and 21 strains out of the 630 are producing siderophores.**

**16 of 100 strains are capable of nitrogen-fixation (acetylene reduction test).**

**The selected strains were scored according to the scoring protocol described in Example 2. and the 26 strains with the highest scores (Stress Tolerant - Plant Growth Promotion Rhizobacteriaceae and Aggregate Promotion Bacteriaceae) were identified by 16 rRNA sequence analysis and The BLAST database.**

**Table 23: The optimal Stress Tolerant - Plant Growth Promotion Rhizobacteriaceae and Aggregate Promoting Bacteriaceae strains**

| Strain | Stress tolerance | Score | PGPR and AP | Score | Name | In total |
|---|---|---|---|---|---|---|
| ES113 | alkaline pH optimum (over 8) | 2 | low-rate IAA production | 0 | *Lysinibacillus xylanilyticus* | 2 |
| ES4 | alkaline | 2 | - | 0 | *Brevibacterium frigoritolerans* | 2 |
| S23 | neutral | 2 | - | 0 | *Bacillus simplex* | 2 |
| S7 | wide range of tolerance | 2 | - | 0 | *Bacillus pumilus* | 2 |
| ES209 | salt tolerant, cold tolerant, pH tolerant | 4 | good phosphate solubilization | 2 | *Bacillus megaterium* | 6 |
| NF5 | neutral | 0 | nitrogen-fixation | 6 | *Azospirillum lipoferum* | 6 |
| ES215 | neutral | 2 | moderate phosphate | 5 | *Pseudomonas chlororaphis* | 7 |
| | | | solubilization | | | |
| ES266 | salt-, cold-tolerant, wide range of pH tolerance | 4 | low rate IAA production | 3 | *Bacillus megaterium* | 7 |
| ES232 | salt-, cold-tolerant, wide range of pH tolerance | 3 | low rate phosphate solub., low rate IAA prod. | 4 | *Bacillus megaterium* | 7 |
| S123 | acidic | 2 | low rate phosphate solub., low rate IAA prod. | 5 | *Agreia pratensis* | 7 |
| S34 | acidic | 2 | phosphate solub., low-rate IAA | 5 | *Pseudomonas frederiksbergensis* | 7 |
| S 225 | neutral pH | -4 | IAA, polysacharide prod | 12 | *Kocuria rosea* | 8 |
| S28 | acidic pH | 2 | polysacharide, biocontrol | 6 | *Bacillus simplex* | 8 |
| 242/9 | neutral pH | -3 | N-fix., IAA | 12 | *A. brasilense* | 9 |
| NF10 | cold-selected, neutral - alkaline pH, | -2 | N-fix., IAA | 11 | *A. brasilense* | 9 |
| NF6 | cold selected, cold tolerant, neutral-alkaline pH, | -2 | N-fix., IAA | 11 | *A. irakense* | 9 |
| NF7 | cold tolerant, acidic neutral pH, | 0 | N-fix. | 9 | *Azospirillum brailense* | 9 |
| S 125 | cold tolerant, moderate salt tolerance, neutral pH | 2 | phosphate solub., low rate IAA | 7 | *Pseudomonas jessenii* | 9 |
| B 41 | neutral-alkaline pH | -1 | N-fix., IAA. | 12 | *A. largimobile* | 11 |
| LU 44 | cold tolerant, high salt tolerance, wide range of pH tolerance | 4 | IAA prod., phosphate solub. | 8 | *Exiguobacterium acetylicum* | 12 |
| NF 11 | slightly acidic -neutral pH | -3 | N-fix., IAA | 16 | *A. brasilense* | 13 |
| S 153 | alkaline pH | 2 | siderophore, IAA, phosphate solub, | 12 | *Arthrobacter crystallopoietes* | 14 |
| S 33 | cold tolerant, high salt tolerance, acidic pH | 2 | phosphate solub, low rate IAA | 12 | *Pseudomonas. frederiksbergensis* | 14 |
| S 47 | cold tolerant, acidic pH | 2 | phosphate solub, IAA, siderophore | 13 | *Agreia pratensis* | 15 |
| 13/4B | alkaline pH | 1 | phosphate solub, siderophore prod., polysacharide pr. | 18 | *Pseudomonas. chlororaphis* | 19 |
| S 284 | strongly acidic pH | -1 | siderophore, polysacharide pr. | 20 | *Paenibacillus peoriae* | 19 |

**According to the SBBS screening system classification, 26 strains were found suitable for inoculation on low fertility deteriorated soils due to abiotic stress tolerance (ST), plant growth promoting (PGPR), nitrogen-fixing, phosphate solubilizing, siderophore and polysacharide producing (aggregation promoting AP) properties.**

**The identification protocol of the strains was performed as follows:**
The 16S rRNA sequencing is a commonly used method for taxonomical identification of microbes. It is based on matching the 16S rRNA sequence of the unidentified isolate with the sequences in the NCBI nucleotide-nucleotide BLAST (http://www.ncbi.nlm.nih.gov/blast/) database. Sequence similarities above 98-99% are considered identification.

Brief description of the method: extraction and purification of genomic DNA, PCR-amplification of the 16S rRNA region - primers applied: 27f (5' **GAGTTGATCCTGGTCAG** 3'), 1525r (5' **AGAAAGGAGGTGATCCAGCC** 3'), direct sequencing of the PCR product by using a BigDye Terminator Cycle Sequencing Ready Reaction Kit (Applied-Biosystem / Perkin-Elmer ) and an ABI Prism 310 Genetic Analyser, primer applied: 519r(5' **G(T/A)ATTACCGCGGC(T/G)GCTG** 3'), matching of the obtained sequence with the BLAST database.

### Example No.17.: Colonizing-competition - CoCoHelp - studies

The identified strains were tested for their interactions in dual cultures to in order to find the optimal components for the multi-strain inoculants. Siderophore and exoploysacharide production were considered as factors of potential inhibition of bacterial growth and colonizing ability, respectively. The strains were screened for their possible antibiotic effects (growth inhibiton) against soil-living Gram negative *Pseudomonas fluorescens* NCAIM B. 01102 and diazotroph *Raoultella terrigena* NCAIM B. 02149 and Gram positive *Bacillus circulans* NCAIM B. 01115.

The strains were classified as invasive, resistent or sensitive based on the results of antibiosis studies.

**Table 24: Antibiotic effects of invasive strains in Pseudomonas, Bacillus and Raoultella growth inhibition biotests**

| Strain | *Species* | siderophore | zone of inhibition | Gram | exopolysaccharide production | biosensitivity |
|---|---|---|---|---|---|---|
| ES113 | *Lysinibacillus xylanilyticus* | | + | + | | invasive |
| ES215 | *Pseudomonas chlororaphis subsp. Aurantica* | + | + | - | | invasive |
| S125 | *Pseudomonas jessenii* | | | - | | invasive |
| **S34** | *Pseudomonas frederiksbergensis* | | | - | | invasive |
| S153 | *Arthrobacter crystallopoietes* | + | | + | | invasive |
| S28 | *Bacillus simplex* | | + | + | + | invasive |
| S33 | *Pseudomonas frederiksbergensis* | + | | - | | invasive |
| 13/4 B | *Pseudomonas chlororaphis* | + | + | - | + | invasive |
| 25/4 | *Leuconostoc mesenteroides* | | + | + | + | invasive |
| 9/4B | *Pserudomonas chlororaphis* | + | + | - | + | invasive |

Siderophore producing or in other way antibiotic strains were considered as invasive. Strains 13/4 B and ES 225 inhibited the growth of *R.terrigena,* the ES 215, ES 317, 25/4, 13/4 B, S 28, 9/4 B, ES 113 and S 30 strains inhibited the growth of *B.circulans.* The growth of Ps fluorescens was not inhibited by any of the strains.

**Table 25: Gram positive resistant strains that did not inhibit growth in Pseudomonas, Bacillus and Routella growth inhibition biotests**

| **strain** | *species* | Gram | exopolysaccharide prod. | biosensitivity |
|---|---|---|---|---|
| **ES209** | *Bacillus megaterium* | + | | resistent |
| **ES232** | *Bacillus megaterium* | + | | resistent |
| **ES266** | *Bacillus megaterium* | + | | resistent |
| **S7** | *Bacillus pumilus* | + | | resistent |
| **ES4** | *Brevibacterium frigoritolerans* | + | | resistent |
| **LU44** | *Exiguobacterium acetylicum* | + | | resistent |
| **S123** | *Agreia pratensis* | + | | resistent |
| **S225** | *Kocuria rosea* | + | | resistent |
| **S23** | *Bacillus simplex* | + | | resistent |
| **S284** | *Paenibacillus peoriae* | + | | resistent |
| **S47** | *Agreia pratensis* | + | | resistent |

Gram positive, capsule producing, polysacharide producing and / or antibiotic resitent strains were found resistent in biotests.

### Sensitive strains

**Table 26: Strains proven to be sensitive in Pseudomonas, Bacillus és Routella growth inhibition biotests**

| strain | species | siderophore | zone of inhibition | Gram | polysacharide or capsule production | biosensitivity |
|---|---|---|---|---|---|---|
| NF5 | *Azospirillum lipoferum* | | | - | | sensitive |
| S47 | *Agreia pratensis* | | | - | | sensitive |
| NF6 | *Azospirillum irakense* | | | - | | sensitive |
| NF7 | *Azospirillum brasilense* | | | - | | sensitive |
| NF10 | *Azospirillum brasilense* | | | - | | sensitive |
| NF11 | *Azospirillum brasilense* | | | - | | sensitive |
| B 41 | *Azospirillum largimobile* | | | - | | sensitive |
| 242/9 | *Azospirillum brasilense* | | | - | | sensitive |

The *diazotroph Azospirillum brasilense* NF7, NF10, NF 11 and 242/9 strains, the *Azospirillum lipoferum* NF 5, the *Azospirillum largimobile* B41 and the *Azospirillum irakense* NF6 strains were classified as non-inhibitory, non-resitent sensitive strains.

Inhibitory, partial inhibitory or helper interactions were demonstrated by colonization-competition studies (CoCoHelp). 12h (28C) inoculum cultures (modified Nutrient Broth (NB) medium) were inoculated/streaked onto modified Nutrient Agar (NA) plates. Parallel inoculations were performed. Holes of 10mm in diameter were cut from the inoculated agar plates. 0.2 ml of the supernatant of centrifuged 12h cultures (in NB medium) of the studied strains were spotted in the wells of the inoculated agar plates. The presence of inhibitory or helper zones around the wells were examined after 168 hours of incubation and the diameter of the zones were registered. A lower rate of growth around the wells was recorded as partial inhibition, while a zone of intensive cell proliferation was considered as a helper, synergistic interaction.

The most invasive strains, that induce inhibition or partial inhibition regarding most of the strains are S33, S284 and 13/4B. The *Azospirillum brasilense* NF 10, NF 11, NF 7, 242/9, the *Azospirillum irakense* NF 6, the *Azospirillum largimobile* B 41 and the *Agreia pratensis* S47 strains are the most sensitive against the inhibitory effects of the invasive strains. The ***Exiguobacterium acetylicum Lu 44* has been proven to be a helper strain that promotes the growth of *Azospirillum brasilense* strains (NF7, NF10, NF11 and 242/9).** Strains with a good growth rate, no or partial inhibitory effects and low sensitivity are considered as good options for inoculation. In case of a sensitive strain, the presence of a helper strain is required.

Results of the 15 strains most suitable for inoculation, based on CoCoHelp studies:

**Table 27: Inhibitory and helper interactions of the strains selected in CoCoHelp studies**

| **Diameter of zone of inhibition (mm), the presence of partial inhibition (PI) or synergistic-helper (SH) effect between strains** | | | | | | | | | | | | | | | |
|---|---|---|---|---|---|---|---|---|---|---|---|---|---|---|---|
| strain | S 28 | S 33 | S 47 | S 125 | S 153 | S 225 | S 284 | NF 6 | NF 10 | NF 11 | 13/4 B | 242/9 | Lu 44 | NF7 | B41 |
| S 28 | | - | - | - | - | - | **2,5** | - | - | - | 4,5 | - | - | - | **PI** |
| S 33 | - | | - | - | - | - | **2** | - | - | - | - | - | - | - | - |
| S 47 | - | **PI** | | PI | - | SH | **PI** | - | SH | - | PI | - | **PI** | **SH** | **PI** |
| S 125 | - | - | - | | - | - | **PI** | - | - | - | - | - | - | - | **PI** |
| S 153 | - | - | - | - | | - | **PI** | - | - | - | - | - | **1,5** | - | **PI** |
| S 225 | - | - | - | - | - | | **6,5** | - | - | - | PI | - | 2 | - | **PI** |
| S 284 | - | - | - | - | - | - | | - | - | - | 6,5 | - | - | - | - |
| NF 6 | - | - | - | - | - | - | **2** | | - | - | PI | PI | - | - | - |
| NF 10 | PI | **6,5** | - | - | - | - | - | - | | - | PI | - | **SH** | - | **2,5** |
| NF 7 | PI | **6,5** | - | - | - | - | - | - | - | - | PI | - | **SH** | - | - |
| NF 11 | PI | **PI** | - | - | - | - | **PI** | - | - | | PI | - | **SH** | - | - |
| 13/4 B | - | - | - | - | - | - | - | - | - | - | | - | - | - | - |
| 242/9 | - | **PI** | - | - | - | - | **PI** | - | - | - | PI | | **SH** | - | - |
| Lu 44 | - | - | - | - | - | - | **6,5** | - | - | - | 6,5 | - | - | - | - |
| B 41 | - | - | - | - | - | - | **PI** | - | - | - | PI | - | - | - | - |

**Table 28: Description of the strains selected as suitable soil inoculants according to the SBSS screening system and colonization-competition (CoCoHelp) studies**

| strain | identification | abiotic stress tolerance ecology | plant growth promotion and soil ecology improvement | biological tol-erance |
|---|---|---|---|---|
| **S125** | *Pseudomonas jessenii* | cold tolerant, salt tolerant, neutral | phosphate solubilization, low rate IAA prod., siderophore prod. | invasive |
| **S153** | *Arthrobacter crystallopoietes* | draught tolerant, alkaline | phosphate solubilization, IAA prod., siderophore prod. | invasive |
| **S225** | *Kocuria rosea* | neutral | IAA production | resistent |
| S28 | *Bacillus simplex* | acidic | polysacharide prod. | invasive |
| **S284** | *Paenibacillus peoriae* | acidic | polysacharide and siderophore prod. | resistent |
| **S33** | *Pseudomonas frederiksbergensis* | cold tolerant, salt tolerant (7%) | phosphate solub., siderophore prod. | invasive |
| **S47** | *Agreia pratensis* | cold tolerant, salt tolerant (2%), acidic | phosphate solub. | resistent |
| NF6 | *Azospirillum irakense* | salt tolerant, cold selection tolerant | nitrogen-fixation | sensitive |
| NF7 | *Azospirillum brasilense* | salt tolerant, cold tolerant, wide range of pH tolerance | nitrogen-fixation, IAA prod. | sensitive |
| NF10 | *Azospirillum brasilense* | salt tolerant, cold selection tolerant | nitrogen-fixation, IAA prod. | sensitive |
| NF11 | *Azospirillum brasilense* | alkaline | nitrogen-fixation, IAA prod. | sensitive |
| B 41 | *Azospirillum largimobile* | alkaline | nitrogen-fixation, IAA prod. | sensitive |
| 242/9 | *Azospirillum brasilense* | neutral | nitrogen-fixation, IAA prod. | sensitive |
| 13/4 B | *Pseudomonas chlororaphis* | alkaline | siderophore prod. | invasive |

### Example No.18.: Population genetic studies

### 1. Development and validation of an in silico model system

The concept of developing a model system was to distinguish individual strains in multistrain inoculants when monitoring the presence and cell count of inoculant strains in the microbial community of the soil. The model system is based on T-RFLP and *in silico* restriction analysis of different variable regions of the 16S rRNS gene. We have searched for a combination of a restriction endonuclease and a variable region that is able to differentiate the three inoculant strains on the T-RFLP electrophoregram. The reliability and practical applicability of the selected system was to be tested in regard to all of the identified strains.

### 2. Bacterial diversity of soils and the effects of inoculation

Suggesting that the T-RFLP based differentiation of strains is successfully put in practice, the next step is to map the microbiota of target soil types. We aimed to compare the microbial population of control and inoculated soils by T-RFLP fingerprinting. We aimed to monitor the increase of the relative abundance of inoculated strains and any other changes in the soil microbiota in given times following inoculation.

### 3. Soil samples and DNA isolation

Samples from the studied agricultural lands were stored frozen at -20C until further analysis. Samples, their identification codes and sampling dates are displayed in Table 29. Soil samples that have not been inoculated yet were signed 'W0', serving as control samples. Samples deriving from further sampling dates are signed W1-W2-W3-W4-W5, respectively. Different combinations of inoculants are coded with capitals and numbers, as displayed in Table 29. Genomic DNA was isolated from altogether 26 samples.

**Table 29.: Identification and characteristics of soil samples from Zimány (Z) from control and inoculated corn and sugar beet fields**

| **Sample No.** | **Lab code** | **Sampling site** | **Crop** | **Soil pH** | **Time of sampling** | **Quantity of sample for DNA isolation** |
|---|---|---|---|---|---|---|
| **Control samples** | | | | | | |
| **1** | SZG-W0 | SzTG | maize | silghtly acidic | 2010.04.13. | 0.45g |
| **2** | 16-W0 | 6Igal | maize | strongly acidic | 2010.04.13. | 0.53g |
| **3** | 14-W0 | 6 Igal | maize | neutral | 2010.04.13. | 0.46g |
| **4** | Z62S-W0 | 3 Z62 | maize | strongly acidic | 2010.04.20. | 0.42g |
| **5** | Z62-W0 | 3 Z62 | maize | neutral | 2010.04.20. | 0.40g |
| **6** | Z1-W0 | Z33 | sugar beet | neutral | 2010.03.25. | 0.57g |
| 7 | Z3-W0 | Z33 | sugar beet | alkaline | 2010.03.25. | 0.45g |
| **8** | Z4-W0 | Z33 | sugar beet | acidic | 2010.03.25. | 0.50g |
| | | | | | | |

| **Inoculated samples** | | | | | | |
|---|---|---|---|---|---|---|
| **9** | SZG-W1-A | SzTG | maize | slightly acidic | 2010.04.20. | 0.44g |
| **10** | SZG-W1-G | SzTG | maize | slightly acidic | 2010.04.20. | 0.49g |
| **11** | SZG-W1-H | SzTG | maize | slightly acidic | 2010.04.20 | 0.45g |
| **12** | SZG-W3-H | SzTG | maize | slightly acidic | 2010.05.04. | 0.43g |
| **13** | SZG-W5-H | SzTG | maize | slightly acidic | 2010.05.26. | 0.47g |
| **14** | SZG-W1-I | SzTG | maize | slightly acidic | 2010.04.20. | 0.39g |
| **15** | 14-W1-G | 6Igal | maize | neutral | 2010.04.20. | 0.52g |
| **16** | 14-W3-G | 6Igal | maize | neutral | 2010.05.04. | 0.49g |
| **17** | 14-W5-G | 6Igal | maize | neutral | 2010.05.26. | 0.52g |
| **18** | 14-W1-I | 6Igal | maize | neutral | 2010.04.20. | 0.48g |
| **19** | Z62-W1-E | 3Z62 | maize | neutral | 2010.04.26. | 0.41g |
| **20** | Z62-W3-E | 3Z62 | maize | neutral | 2010.05.10. | 0.39g |
| **21** | Z62-W5-E | 3Z62 | maize | neutral | 2010.06.08. | 0.42g |
| **22** | Z62-W1-F | 3Z62 | maize | neutral | 2010.04.26. | 0.41g |
| **23** | Z1-W1-1 | Z33 | sugar beet | neutral | 2010.03.31. | 0.41g |
| **24** | Z1-W3-1 | Z33 | sugar beet | neutral | 2010.04.20. | 0.40g |
| **25** | Z1-W5-1 | Z33 | sugar beet | neutral | 2010.05.04. | 0.44g |
| **26** | Z1-W1-5 | Z33 | sugar beet | neutral | 2010.03.31. | 0.44g |

**Table 30: Identification of different stress tolerant soil inoculant strain combinations besides positive control Bactofil and Bactofil H**

| **Inoculation type** | **Szentgáloskér** | **Igal** | **Zimány** | **Inoculation type** | **Zimány** |
|---|---|---|---|---|---|
| | **slightly acidic** | **neutral** | **neutral** | | **neutral** |
| | **SZG** | **I4** | **Z62** | | **Z1** |
| **A** | Bactofil | 9/4B | 9/4B, 13/4B | **1** | Bactofil |
| **B** | 9/4B, 13/4B | 9/4B, 13/4B | 9/4B | **5** | BactofilH |
| **C** | KE9, 9/4B, 13/4B | KE9, 9/4B, 13/4B | KE9, 9/4B, 13/4B | | |
| **D** | KE9, 9/4B, 13/4B, Str | KE9, 9/4B, 13/4B, Str | KE9, 9/4B, 13/4B, Str | | |
| **E** | SW51H, Bactofil | 25/4, KE9, 9/4B, 13/4B | BcirH, M326H, Bactof | | |
| **F** | M657H, Bactofil | SW51H, M657H, A21H, Bactofil | BcirH, SW11H, M326H, ALH, Bactofil | | |
| **G** | A21H, Bactofil | BcirH, M326H, Bactofil | H(all) | | |
| **H** | M326H, Bactofil | H(all) | Bactofil | | |
| **I** | H(all) | BcirH, SW11H, M326H, ALH, Bactofil | 25/4, KE9, 9/4B, 13/4B | | |
| **J** | | Bactofil | | | |

Genomic DNA were isolated form samples by using an *UltraClean^{™} Soil DNA Isolation Kit* (MoBio) system, in a laminar chamber. 0.5 g of the frozen samples were used. Purified genomic DNA samples were eluted in 40 µl buffer. The success of isolation and quantity of DNA siolates were determined by agarose-gel (1%) electrophoresis. Electrophoresis was perfomed at 100V for 20 min in 1 X TBE buffer with 2 µl molecular weight standard (Lambda DNA/EcoRI+HindIII Marker-3, Fermentas, Vilnius, Lithuania [125-21226 bp range]) as reference.

### 3.T-RFLP analysis

The T-RFLP method (terminal restriction fragment length polymorphism) is based on separation of the restricion enzyme hydrolysed PCR products, according to length. During PCR, the amplification of the template is done with a special primer, fluorescent in the 5' terminus that results in fluorescent marked PCR products. The number and location of cleavage sites for restriction enzymes are varying on the PCR products. Hydrolysis with restriction endonuclease thus result in the different length of terminally-marked fragments (Liu et al., 1997). Hydrolysed amplicons are separated via high-performance capillary gel electrophoresis, being able to detect on the fluorescent terminals. Based on T-RF lengths, the peaks provide phylogenetic reference and the area under the curve is a basis of assessing relative quantities.

DNA samples were isolated from pure cultures of the studied strains and the nucleotide sequence of the small-subunit ribosomal RNA gene (16SrDNA) were determined in regard to every strain. In view of the obtained nucleotide sequences, the recognition sites of available universal eubacterial primers and the cleavage sites of restriction endonucleases the restriction fragment lengths were determined theoretically by the *in silico* method. Primer pair-enzyme combinations were selected on the basis of results, and tested *in vitro,* performing polymerase chain reaction (PCR), restricion hydrolysis and capillary electrophoretic detection regarding every strain and primer-enzyme combination. To increase reliability, two different primer pair - enzyme combinations were selected from the potential combinations. Terminal restriction fragment lengths (TRF) of stress-tolerant strains obtained with different primer pair-enzyme combinations are summed up in Table 31. The combination of 341F-907R primers and the *Alu*I restriction enzyme is marked by "A", the combination of 27F-534R primers and *Hin*6I enzyme are marked by "B" hereinafter.

**Table 31.: TRF lengths of stress tolerant strains obtained with different primer-enyzme combinations**

| | "A" T-RFLP | "B" T-RFLP |
|---|---|---|
| Strain ID | TRF lengths using 341F-907R primers and *Alu*I enzyme | TRF lengths using 27F-534R primers and *Hin*6I enzyme |
| 25/4K | 118 bp | 204 bp |
| 9/4B | 313 bp | 203 bp |
| 13/4B | the 16S rDNA is identical with 9/4B | |

Electrophoretic separation (CGE: capillary gel electrophoresis) of fluorescently marked terminal fragments was performed by using an *ABI PRISM 310 Genetic Analyzer* (Applied Biosystems, Foster City, CA, USA). The adjusted parameters were as follows: 8 sec injection, 30min running time on POP-4^{™} polymer (*Performance Optimized Polymer 4,* Applied Biosystems) at 60°C and 15kV. At least 3 parallel runs were launched for each sample. Samples were added with formamide and TAMRA 500 standard. The prepared samples were denaturated on 98°C and placed on ice.

The GeneMapper software (Applied Biosystems, version 3.7) was used for the analysis of T-RFLP chromatograms. Exact determination of fregment lengths of separated peaks is based on the the TAMRA 500 length standard, marked with a different fluorescent. Fragments of the length standard compensate for possible differences in injections as being under identical electrophoretic forces as the sample. The fragments of the standard are evenly distributed in the given size range which makes the exact determination of peaks. Running the standard helps in evaluating he quality of the electrophoregram, as the software cannot detect the length standards properly in case of partial separation. The GeneMapper software associates the following data to standard peaks: i) time of detection (in time and data points), ii) peak height (given in fluorescence intensity units) and iii) areaunder-the-curve (given in fluorence intensitiy units).

Statistical evaluation of results was carried out by the T-REX *(T-RFLP Analysis Expedited)* online software (Culman et al., 2009). Hierarchic classification from the obtained data matrix was performed by the PAST software (Hammer et al., 2001) the Bray Curtis index method and UPGMA (Unweighted Pair Group Method of Average).

### 4. DNA T-RFLP studies of stress tolerant Pseudomonas chlororaphis 13/4B and 9/4B and Leuconostoc mesenteroides 25/4K strains in neutral soil

### Sampling method:

By means of a spiral soil sampler, marking sampling sites in the plot, from rows, on inoculated plots: in comparison with soil samples taken prior to inoculation, storage at -20°C.

**Table 32.: Presence of Pseudomonas chlororaphis 9/4B and 13/4B and Leuconostoc mesenteroides 25/4K stress tolerant strains in inoculated neutral soil given in percentage of microbial population at the time of samplings in the 7 weeks following inoculation**

| **Time of sampling** | **Prevalence of the strain in the soil microbial popualtion (%)** | |
|---|---|---|
| | ***Ps. chlororaphis*** | ***L. mesenteroides*** |
| | **13/4 - 9/4** | **25/4** |
| **neutral soil (Zimány)** | | |
| prior to inoculation | 0.390 | 0 |
| 1 wk after inoculation | 1.59 | 0 |
| 3 wks after inoculation | 0.8 | 0.426 |
| 7 wks after inoculation | 1.58 | 1.44 |
| | | |

| **neutral soil (Igal)** | | |
|---|---|---|
| prior to inoculation | 0.28 | 0 |
| 1 wk after inoculation | 3.78 | 1.166 |
| 3 wks after inoculation | 3.85 | 1.22 |
| 7 wks after inoculation | 1.25 | 1.66 |

**DNA fragments of *P. chlororaphis* strains were detected. 1-3 weeks after inoculation the proportion of Ps. chlororaphis DNA fragments was considerable, approximately 4% of the total microbial population. The L. mesenteroides strains was also detectable, making up 1-1.5% of the total microbial population even 7 weeks after inoculation.** The stress tolerant *Pseudomonas* 9/4B, 13/4B and Leuconostoc 25/4K strains were persistently present in neutral soils.

### 5. DNA T-RFLP studies of stress tolerant Pseudomonas chlororaphis 13/4B and 9/4B and Leuconostoc mesenteroides 25/4K strains in acidic soil

Figure 4 displays the parameters of the sampling plot (Orczy 42, Zimány): strongly acidic: pH 5.0, with low-phosphate areas, contains loam, clayey loam, with medium humus content

Figure 5. displays sampling technique: by means of a spiral soil sampler, marking sampling sites in the plot, sampling form rows and inter-row spaces, from inoculated and uninoculated plots, from soil, rizosphere and roots. Samples stored at -20 °C.

**Table 33: Presence of Pseudomonas chlororaphis 9/4B and 13/4B and Leuconostoc mesenteroides 25/4K stress tolerant in inoculated acidic (pH 5.5) soil given in percentage of microbial population at the time of samplings in the 6 months following inoculation, based on DNA T-RFLP results**

| | **Prevalence of DNA fragments of the studied strains in the soil(%)** | | | | | | | |
|---|---|---|---|---|---|---|---|---|
| **Minta** | ***P. chlororaphis* 13/4 és 9/4** | | | | ***L. mesenteroides 25*/*4*** | | | |
| | **uninoculated** | **Rows. inoculated** | **unioculated** | **Interrow space, inoculated** | **uninoculated** | **Rows** | **unninoculated** | **interrow space** |
| | | | | | | | | |
| **prior to inoculation** | 0.726 | 0.268 | 0.726 | 0.268 | 0 | 0.081 | 0 | 0.081 |
| **immediatelv after inoculation** | 0.,503 | 1.39 | 0.503 | 1.39 | 0 | 0.328 | 0 | 0.328 |
| **1wk after inocula-tion** | 0.741 | 1.04 | 0.704 | 1.16 | 0 | 0 | 0 | 0.258 |
| **1month after inoc-ulation** | 1.19 | 2.63 | 1.58 | 2.48 | 0 | 0 | 0 | 0.255 |
| **6 months after inoculation** | 0 | 0 | 0 | 3.34 | 0 | 0.381 | 0 | 0 |

**The relative prevalence of Ps. chlororaphis 9/4 and 13/4 is higher in inoculated than unioculated acidic soil. 1 month after inoculation, the relative proportion is 2.5% in rows and interrow spaces, and 3.5% in interrow spaces 6 months after inoculation. L. mesenteroides is not present or detectable in uninoculated soil, while it is present in a low relative proportion 1 month after inoculation, in interrow spaces.**

**T-RFLP peaks referring to strains 13/4B, 9/4B and 25/4K were detected is a significantly higher rate in inoculated acidic soil samples from inter-row spaces and rizospheric soil than in uninoculated soil. T-RFLP tracing is suitable to monitor the persistence of soil inoculant strains in the soil, however, certain natural and methodological factors can highly influence reliability of results.**

### Example 19. Fermentation culturing of soil inoculant strains

### Preparation of agar slants

**NA agar slants are prepared as follows:** Nutrient Agar (Oxoid) is prepared according to the manufacturer's prescriptions and supplemented with 0.5% yeast extract, 0.5% glucose and 0.5% sacharose before sterilization at 121°C for 25min.

Agar slant are inoculated with the following strains:
***Pseudomonas chlororaphis*** K2009 13/4 B NCAIM (P) B 001370
***Pseudomonas chlororaphis*** K2009 9/4-B NCAIM (P) B 001371
***Leuconostoc mesenteroides*** K2009 25/4 NCAIM (P) B 001372
*Azospirillum brasilense* **NF 11 NCAIM** (P) B **001428**
***Azospirillum brasilense* 242/9** NCAIM (P) B 001403
***Azospirillum brasilense* NF 10 NCAIM** (P) B **001427**
***Azospirillum largimobile,* B41 NCAIM** (P) B 001402
***Azospirillum brasilense* NF 7** NCAIM (P) B 001433
***Azospirillum irakense* NF 6 NCAIM** (P) B **001425**
***Exiguobacterium acetylicum LU44* NCAIM** (P) B *001423*
***Paenibacillus peoriae S* 284 NCAIM** (P) B **001430**
***Agreia pratensis S* 47 NCAIM** (P) B ***001431***
***Arthrobacter crystallopoietes** S 153* (P) **NCAIM** B 001424
***Pseudomonas frederiksbergensis** S33* NCAIM (P) B ***001429***
***Pseudomonas jessenii S 125* NCAIM** (P) B ***001422***
***Bacillus simplex S 28* NCAIM** (P) B **001432**
***Azospirillum brasilense* NF 7 NCAIM**
***Kocuria rosea S* 225 NCAIM** (P) B 001426
and incubated at 28°C for 72-96h. Stored at 4°C, the cultures are viable for 3 months. The suspension of bacteria washed off the agar slant with sterile saline - added with 10 dimethyl -sulphoxide or 20% glycerol and stored at -40°C- is viable for a year.

### Preparation of starter cultures

**Strains are transferred from the agar slants to 100ml NB sterilized medium, prepared as follows:** Nutrient Broth (Oxoid) is supplemented with 0.5% yeast extract, 0.5% glucose, 0.5% sacharose and adjusted to pH 7.0-7.5 before sterilization at 121°C for 25min. The strains are grown at30°C, in shaken cultures at 250rpm, for 24h. Growth is monitored by microscopy. Cell count in 24h cultures reaches approx. 1.0-2.0 x 10⁹ CFU/ml, except for
***Exiguobacterium acetylicum LU44* NCAIM** (P) B ***001423***
***Paenibacillus peoriae S* 284 NCAIM** (P) B **001430**
***Bacillus simplex S 28* NCAIM** (P) B **001432**
***Kocuria rosea S 225* NCAIM** (P) B 001426
***Azospirillum largimobile,* B41 NCAIM** (P) B 001402
***Azospirillum brasilense* 242/9** NCAIM (P) B 001403
***Azospirillum brasilense* NF** 7 NCAIM (P) B 001433
cultures, which have a cell count of 4.0 - 5.0 × 10⁸ CFU/ ml.

### Preparation of inoculums for 100 l fermentation

30ml of the starter cultures were inoculated into 1000 ml NB sterilized broth in 3000 ml Erlenmeyer flasks. The Nutrient Broth is added with 0.5 yeast extract, 0.5% glucose and 0.5% saccharose and adjusted to pH 6.7-7.0 before sterilization at 121°C for 25 min. The strains are grown at 28-30°C, in shaken cultures at 250 rpm, form 24-48h. Growth is monitored by microscopy. Cell count in 24h cultures reaches 1.0-2.0 × 10⁹ CFU/ml, except for
***Exiguobacterium acetylicum** LU44* **NCAIM** (P) B ***001423***
***Paenibacillus peoriae S* 284 NCAIM** (P) B **001430**
***Bacillus simplex S 28* NCAIM** (P) B **001432**
***Kocuria rosea S 225* NCAIM** (P) B 001426
***Azospirillum largimobile,* B41 NCAIM** (P) B 001402
***Azospirillum brasilense* 242/9** NCAIM (P) B 001403
***Azospirillum brasilense* NF** 7 NCAIM (P) B 001433
cultures, which have a cell count of 4.0 - 5.0 × 10⁸ CFU/ ml.

### Main fermentation in 100 l volume (FŐ 1)

**Sterilized Main 1 medium in 100 l working volume stirred tank bioreactors are inoculated with the 1000 ml cultures.**

Main 1 medium: corn steep liquor 0.5%, molasses 0.5%, glucose 0.5%, KH₂PO₄ 0.5%, K₂HPO₄ 0.1%, CaCO₃ 0.2%, MgSO₄ 0.02%, yeast extract 0.2%, isoglucose 0.1%.

PH is adjusted to 7.2-7.5 before sterilization at 121°C, at 1.2 atm for 25 min.

0.5% sterilized dextrose solution (50%) is added after sterilization.

Air flow rate is adjusted to 300 l/h, the speed of the impeller is 300rpm (1 v/v/m). Cultures are grown at 28-30°C for 24h. End pH: 6.5-7.5.

Oxygen saturation rapidly decreases and reaches 50% after 5-6 h, following a short lag phase, then intensive cell proliferation starts. The impeller speed is set at 250-300 rpm, the air flow rate is on average 5-6 m³/h/100 1 (0.8-1 v/v/m). Cell count after 24 h reaches 3.0-6.0 x 10⁹ CFU/ml, except for
***Exiguobacterium acetylicum LU44* NCAIM** (P) B ***001423***
***Paenibacillus peoriae S* 284 NCAIM** (P) B **001430**
***Bacillus simplex S 28* NCAIM** (P) B **001432**
***Kocuria rosea S 225* NCAIM** (P) B 001426
***Azospirillum largimobile,* B41 NCAIM** (P) B 001402
***Azospirillum brasilense* 242/9** NCAIM (P) B 001403
***Azospirillum brasilense* NF** 7 NCAIM (P) B 001433
cultures, which have a cell count of 4.0 - 8.0 × 10⁸ CFU/ ml..

The pH of the cultures slightly decreases at the start of the fermentation (pH 6.2-7.0) and increases after 15-20h (pH 6.6-7.0). No buffering is required.

End product samples are stored frozen for any future checkups.

### Main fermentation in 1000 l volume (Main 2)

**50 l of the microscopically checked 100 l inoculum was transferred to 1000 l K3 culture medium and fermented at 28-30°C.**

K3 culture medium: corn steep liquor 1.5%, molasses 0.5%, glucose 0.5%, KH₂PO₄ 0.1%, K₂HPO₄ 0.1%, CaCO₃ 0.2%, (NH₄)₂SO₄ 0.1%, MgSO₄ 0.02%, trace element solution 1ml/100ml

Trace element solution: FeSO4 x 7H2O 300 mg, CoCl2 x 6H2O 10mg, Na2MoO4 x 2H2O 10mg, Na2SeO3 0.01 mg, MnSo4 x 7H20 1mg, Na2B4O7 x 10H2O 1mg, SnCl2 0.01mg, Bi(NO3)3 x 5H2O 0.1 mg, TiCl3 0.01mg, KI 1mg, phosphotungstic acid 1mg, distilled water 1000ml.

The pH was adjusted to 7.2-7.5 before sterilization at 121C, on 1.2 bar for 25 min.

Air flow rate is adjusted to 300 1/h, the speed of the impeller is 300rpm (1 v/v/m). Cultures are grown for 24h. End pH: 6.5-7.5. The rate of growth is monitored as described earlier. Oxygen saturation rapidly decreases and reaches 50% after 4-5 h, following a short lag phase, then intensive cell proliferation starts. Stirring speed is 200rpm at minimum, a more effective aeriation system requires less air flow than 100 1 fermentors. Average air flow rate is 30-60 m3/h/10001 (0.5 v/v/m). Cell count after 24 h reaches 3.0-6.0 x 10⁹ CFU/ml, except for
***Exiguobacterium acetylicum LU44* NCAIM** (P) B ***001423***
***Paenibacillus peoriae S* 284 NCAIM** (P) B **001430**
***Bacillus simplex S 28* NCAIM** (P) B **001432**
***Kocuria rosea S 225* NCAIM** (P) B 001426
***Azospirillum largimobile,* B41 NCAIM** (P) B 001402
***Azospirillum brasilense* 242/9** NCAIM (P) B 001403
***Azospirillum brasilense* NF 7** NCAIM (P) B 001433
cultures, which have a cell count of 4.0 - 8.0 × 10⁸ CFU/ ml.

Proper oxygen stauration is required for optimal growth rate (agitator rpm and sterile air inflow).

The pH of the culture changes tp 6.8-7.5, unlike the 100 1 fermentation.

End product samples are stored frozen for any future checkups.

### Example No.20. Plant tests on maize inoculated with model cultures of stress tolerant soil bacterial strains.

The effect of isolates - selected as potential inoculants - on plant biomass production was studied on maize test plant in greenhouse pot trials. Plants were inoculated with individual, or mixed cultures of the isolates, respectively.

Effect on plant biomass production on hybrid corn test plant in greenhouse pot trials:
Corn seeds were surface disinfected and germinated in liquid agar medium at room temperature. Threeday old seedlings were inoculated with fermented cultures (main fermentation).

The inoculum had a cell count of 2-3 x 10⁷ CFU. Treatments were carried out in 4 repetitions (3plant/treatment) to calculate an average. The plants were grown in pots, in sand medium, with controlled temperature, light and humidity conditions. Shoot and root dry mass of 4-week old plants were measured and given in percentage to that of control (uninoculated) plants.

### Inoculations:

**Table 34: Combination of strains for model cultures**

| 1 | 25/4 (50x dilution) |
|---|---|
| 2 | 9/4 |
| 3 | 13/4 |
| 4 | Control: PBS |
| 5 | 25/4 + 9/4 + 13/4 |
| 6 | 25/4(50x) + 9/4 + 13/4 |
| 7 | 9/4 + 13/4 |
| 8 | 25/4 + 13/4 |
| 9 | 25/4(50x) + 13/4 |

The effect of single strain and mixed inoculations on the shoot and root dry mass of 4-week-old plants are summed up in Table 35.

**Table 35. The effect of single strain and mixed inoculations on the shoot and root dry mass of 4-week-old maize in pot trials.**

| | Dry matter/specimen (g) in percentage of the control | |
|---|---|---|
| Inoculation type | root | shoots |
| 1. | 105.0 | 110.9 |
| 2 | 128.6 | 108.2 |
| 3 | 130.0 | 117.8 |
| 4 | 100.0 | 100.0 |
| 5 | 106.4 | 110.9 |
| 6 | 102.1 | 105.4 |
| 7 | 136.4 | 126.0 |
| 8 | 116.4 | 113.7 |
| 9 | 101.4 | 110.9 |

*L. mesenteroides* 25/4(50× dilution) - Inoculation type 1:
- moderate improvement in root and stem elongation,
- abundant extracellular polysacharide production,
- tolerates high temperatures,
- optional for the inoculation of arenosol, alkaline-saline areas and desert soil.

*Ps. chlororaphis* 9/4 - Inoculation type 2:
- definite improvement in root elongation,
- moderate enhancement of stem elongation,
- enriches phosphorus supply in arenosol due to phosphate solubilization,
- tolerates high temperatures
- optional for the inoculation of arenosol, alkaline-saline areas and desert soil.

*Ps. chlororaphis* 13/4 - Inoculation type 3:
- excellent root and stem growth promoting effect
- optional for the inoculation of arenosol, alkaline-saline areas and desert soil.

Dual combination: *Ps. chlororaphis* 13/4 + *Ps. chlororaphis 9*/*4* - Inoculation type 7.
- excellent root and stem growth promoting effect
- polysacharide production and soil aggregate promotion
- the exopolysaccharide is a potential carbon source for *Ps. chlororaphis*
- phosphate solubilization
- optional for arenosol, draughty areas, salinized soils

Dual combination: *L. mesenteroides* 25/4+ *Ps. chlororaphis* 9/4 - Inoculation type 8.
- balanced root and shoot growth promoting effect
- polysacharide production and phosphate mobilization (P-supply enrichment)
- microbial degradation of the polysacharide enriches soil in carbon available for plants, the soil carbon/nitrogen ratio improves
- optional for arenosol, draughty areas, salinized soils

Dual combination: *L. mesenteroides* 25/4(50× dil.) +*Ps. chlororaphis*13/4 - Inoculation type 9.
- no growth promoting effect on roots, moderate for stem
- not suitable for inoculation

Triple combination: *L. mesenteroides 25*/*4* + *Ps. chlororaphis*13/4 + *Ps. chlororaphis* 9/4 (Inoculation type 5.)
- moderate but balanced root and stem growth promotion
- polysacharide production
- phosphate solubilization (increased P-supply)
- polysacharide as a carbon and energy source for the other two strains
- microbial degradation of the polysacharide enriches soil in carbon available for plants, the soil carbon/nitrogen ratio improves
- optional for arenosol, draughty areas, sodic and salinized soils
   Triple combination: *L. mesenteroides* 25/4(50× dil.) + *Ps. chlororaphis*13/4 + *Ps. chlororaphis* 9/4 (Inoculation type 6.)
- no growth promoting effect on roots, moderate for stem
- not suitable for inoculation

### Example No. 21.: Maize pot trials on the effect of diazotroph stress-tolerant Azospirillum strains

The effect of the Azospirillum strains on plant biomass production was compared with a commercially available Azospirillum inoculant in greenhouse pot trials, on maize test plants

According to the given inoculation cell count, the cultures were diluted with potassium phosphate buffered saline or sterile water.

Corn seeds are surface disinfected and germinated in liquid agar, or filter paper, at room temperature. 2-3 day old seedlings are planted in pots (3 seedlings/pot) in loose structured sandy soil. The soil is inoculated with 1 ml of 2-3 x 10⁷ CFU/ml concentration soil inoculant / test plant. The plants are grown in controlled temperature and lighting conditions and regulated irrigation with tap water. Four parallel inoculations are performed per strain.

Germination rate and root and shoot dry mass of 8-week-old plants were compared with that of the uninoculated control.

**Table 36: Effect of stress-tolerant Azospirillum strains on the root and shoot mass of maize test plant in comparison with commercially available Azospirillum inoculant**

| Strain | Results | |
|---|---|---|
| | biomass production of inoculated maize in pot trials (in % of control) | |
| | **root mass increase** | **shoot mass increase** |
| *Azospirillum brasilense A. brasilense KE1 NCAIM (P), B 001324 positive control* | **130-150 %** | **110-115 %** |
| *Azospirillum largimobile B41* | **175 (SZD5%); high** | **110-115 %** |
| ***Azospirillum brasilense NF10*** | **310 (SZD5%); prominently high** | **110-115 %** |
| *Azospirillum irakense NF6* | **135 (SZD5%) medium** | **110-115 %** |
| ***Azospirillum brasilense 242*/*9*** | **178 (SZD5%); high** | **110-115 %** |
| *Azospirillum brasilense NF11* | **130; not significant medium** | **110-115 %** |

Root growth promoting effect of strain NF10 highly exceeds that of the two commercially available Azospirillum strains. Root growth enhancement by strains 242/9 and B41 slightly exceeds that of the positive control.

Strains NF6 and NF11 have the same growth promoting effect as the positive control *Azospirillum* strains. Growth promoting effect regarding the stem and shoots were moderate and not significant regarding all treatments.

### Example No.22. Soil specific stress-tolerant soil bacterial combinations for inoculation

The cultures of the following strains are mixed in the ratios as displayed in Table 37:
**For neutral soil types:**
   ***Pseudomonas jessenii S 125* NCAIM** (P) B ***001422***
   ***Arthrobacter crystallopoietes** S 153* (P) **NCAIM** B 001424
   ***Kocuria rosea S 225* NCAIM** (P) B 001426
   ***Azospirillum brasilense* NF 11 NCAIM** (P) B **001428**
   ***Azospirillum brasilense* NF 10 NCAIM** (P) B **001427**
   ***Azospirillum largimobile,* B41 NCAIM** (P) B 001402
   ***Azospirillum brasilense* 242/9** NCAIM (P) B 001403
**For acidic soil types:**
   ***Bacillus simplex S 28* NCAIM** (P) B **001432**
   ***Pseudomonas frederiksbergensis** S33* **NCAIM** (P) B ***001429***
   ***Agreia pratensis S* 47 NCAIM** (P) B ***001431***
   ***Azospirillum brasilense* NF 11 NCAIM** (P) B **001428**
   ***Exiguobacterium acetylicum LU44* NCAIM** (P) B *001423*
   ***Azospirillum largimobile,* B41 NCAIM** (P) B 001402
   ***Paenibacillus peoriae S* 284 NCAIM** (P) B **001430**
**For alkaline soil types:**
   ***Exiguobacterium acetylicum LU44* NCAIM** (P) B ***001423***
   ***Pseudomonas jessenii S 125* NCAIM** (P) B *001422*
   ***Arthrobacter crystallopoietes** S 153* (P) **NCAIM** B 001424
   ***Pseudomonas chlororaphis*** K2009 13/4 B NCAIM (P) B 001370
   ***Azospirillum brasilense* 242/9** NCAIM (P) B 001403
   ***Azospirillum largimobile,* B41 NCAIM** (P) B 001402
   ***Azospirillum irakense* NF 6 NCAIM** (P) B **001425**

**Table 37.: Combination (mixing ratios) of soil-specific SBSS strains for acidic, neutral and alkaline soil types**

| Strains | **acidic soil types** | **alkaline soil types** | **neutral soil types** |
|---|---|---|---|
| **13/4 B** | 0 | 1 | 0 |
| **242/9** | 0 | 2 | 1 |
| **B 41** | 2 | 2 | 1 |
| **LU 44** | 1.5 | 1 | 2 |
| **NF 10** | 0 | 0 | 0 |
| **NF 11** | 2 | 0 | 1 |
| **NF 6** | 0 | 2 | 0 |
| ***S 125*** | 0 | 1 | 1 |
| **S 153** | 0 | 1 | 1 |
| **S 225** | 0 | 0 | 2 |
| **S 28** | 1.5 | 0 | 0 |
| **S 284** | 1 | 0 | 0 |
| **S 33** | 1 | 0 | 0 |
| **S 47** | 1 | 0 | 0 |
| **in total** | 10 | 10 | 10 |

### Example No.23: Growth promoting effect of combinant soil specific stress tolerant soil bacterial inoculants on white mustard in calcaric chernozem and sandy brown forest soil

1. The soil pH-specific strain combinations - for the inoculation of acidic, neutral and alkaline soils, as described in Example No.22 - were applied.
2. Test plant parameters
2.1. Species: White mustard *(Sinapis alba L.* )
2.2. Time of sowing: 2013.04.12.
2.3. Seed quantity: white mustard: 60 seeds/pot
2.4. Time of inoculation: 2013. 04. 12.
2.5. Time of harvest: 2013. 05. 13.

3.2. Soil parameters
**3.2.1.calcaric chernozem** (Dunaszekcső)
pH_{KCl}= 6.93
K_{A}= 43
Humus= 2.10 %
Ca, Mg, K salts: 0,04 m/m% CaCO₃= 6 %
Minerals:

| | |
|---|---|
| P₂O₅= 544 mg/kg | K₂O= 203 mg/kg |
| Mg= 181 mg/kg | Na= 97.7 mg/kg |

N(nitrite and nitrate)= 36 mg/kg

| | |
|---|---|
| Cu= 2.48 mg/kg | Zn= 2.4 mg/kg |
| Mn= 74.8 mg/kg | SO₄-S= 16.1 mg/kg |

**3.2.2.Brown forest soil (with sand)** - (Vajszló)
pH_{KCl}= 7.08

| | |
|---|---|
| humusz= 0.87 % | K_{A}= 30 |
| Ca, Mg, K salts: < 0.02 % | CaCO₃= 1,3% |

Minerals:

| | |
|---|---|
| P₂O₅= 212 mg/kg | K₂O= 170 mg/kg |
| Mg= 44.8 mg/kg | Na= 85.4 mg/kg |

N(nitrite and nitrate)= 19.4 mg/kg

| | |
|---|---|
| Cu= 1.42 mg/kg | Zn= 0.838 mg/kg |
| Mn= 83.7 mg/kg | SO4-S= 4.47 mg/kg |

4. Experimental layout
4.1. Plot size: 160 cm²/pot
4.2. Repetitions : 4
4.3. Design (with schematic view):

| | | | | |
|---|---|---|---|---|
| 1 | 2 | 3 | 4 | - 8. Soil bacterial inoculant for **alkaline** soil, **2 l**/ha |
| 1 | 2 | 3 | 4 | - 7. Soil bacterial inoculant for **alkaline** soil, **1 l**/ha |
| 1 | 2 | 3 | 4 | - 6. Soil bacterial inoculant for **acidic** soil, 2 **l**/ha |
| 1 | 2 | 3 | 4 | - 5. Soil bacterial inoculant for **acidic** soil, **1 l**/ha |
| 1 | 2 | 3 | 4 | - 4. Soil bacterial inoculant for **neutral** soil, 2 **l**/ha |
| 1 | 2 | 3 | 4 | - 3. Soil bacterial inoculant for **neutral** soil, **1 l**/ha |
| 1 | 2 | 3 | 4 | - 2. Standard: **BactoFil A10, BactoFil B10 1 l**/ha |
| 1 | 2 | 3 | 4 | - 1. **Control** |

4.4. Inoculation parameters:

**Table 38.: Inoculation on white mustard in calcaric chernozem and sandy brown forest soil in pot trials**

| | **Treatments** | **Dose l/ha** | **Phenophase** | **Method** | **Irrigation l/ha** |
|---|---|---|---|---|---|
| **1.** | Control | - | - | - | as required |
| **2.** | Standard: Bactofil A (for monocotyledons) Bactofil B (for dicotyledons) | 1 | prior to sowing | spraying, ploughed in | 200-400 |
| **3.** | Soil bacterial inoculant for **neutral soil** | 1 | prior to sowing | spraying, ploughed in | 200-400 |
| **4.** | Soil bacterial inoculant for **neutral soil** | 2 | prior to sowing | spraying, ploughed in | 200-400 |
| **5.** | Soil bacterial inoculant for **acidic soil** | 1 | prior to sowing | spraying, ploughed in | 200-400 |
| **6.** | Soil bacterial inoculant for **acidic soil** | 2 | prior to sowing | spraying, ploughed in | 200-400 |
| **7.** | Soil bacterial inoculant for **alkaline soil** | 1 | prior to sowing | spraying, ploughed in | 200-400 |
| **8.** | Soil bacterial inoculant for **alkaline soil** | 2 | prior to sowing | spraying, ploughed in | 200-400 |

Temperature at the time of inoculation: 25,4°C
Relative humidity: 65,3%

### Phytotoxic effect: not observed in any of the treatments

5. Parameters monitored and method, time and phenophase of sampling/measurement
5.1. Temperature and relative humidity in the greenhouse unit - weekly average (Appendix 1.)

### Data collection from the computer database

5.2. Germination rate (number of germinated seeds/pot on the 6th day after sowing) (Appendix: 2)
   Counting all mono- and germinated mono- and dicotyledon specimens
5.3. Average plant height (cm)/pot (Appendix: 3.)
   Average of the height of plants measured at harvesting
5.4. Shoot mass g/pot (Appendix: 4.)
   Plant biomass except the root mass measured at harvesting

### 6. Effects on alisol (brown forest soil)

### Neutral soil bacterial inoculant for brown forest soil: the inoculant significantly increased the average plant height of white mustard in both concentrations applied (P=0.01)

**Both treatments** with the inoculant **have significantly increased shoot mass of white mustard** in **brown forest soil** (P=0.05) compared to control.

*When applied in the **lower concentration, the** inoculant has significantly increased shoot mass of spring **barley** (P=0.01) **on brown forest soil** compared to control.*

### Alkaline soil bacterial inoculant for alisol (brown forest soil)

The inoculant has **significantly** (P=0.01) **increased plant height in 1 l**/ha **concentration** on brown forest soil. Treatment with **2 l/ha increased plant height by 13%.**

Inoculation has significantly (P=0.01) **increased shoot mass of white mustard** compared to control.

### 7. Effects on chernozem soil

**Neutral soil inoculant:** Treatment in a **concentration of 2 l/ha increased plant height by 7%** (Figure 6.) The same concentration has **increased shoot mass of white mustard by 12%.** (Figure 7.)

**Alkaline soil inoculant:** Applied in a higher concentration, the inoculant has **increased average plant height of white mustard (P=0.01),** the lower concentration has increased growth by 9%. **Shoot mass of white mustard has increased by 7%** compared to control.

**Acidic soil inoculant:** inoculation in 2 l/ha concentration has significantly (P=0.05) increased average plant height of white mustard and **increased shoot mass by 8%.**

### Example No 24.: Growth promoting effect of combinant soil specific stress tolerant soil bacterial inoculants on spring barley pot trials in solonchak soil

1. The soil pH-specific strain combinations for the inoculation of alkaline soils, as described in Example No.22 - were applied.
2. Test plant parameters
2.1. Species: Spring barley *(Hordeum vulgare* L.) 'Maresi'
2.2. Time of sowing: 2013.04.12.
2.3. Seed quantity: 60 seeds/pot
2.4. Time of inoculation: 2013.04.12.
2.5. Time of harvest: 2013.06.20.

3.2. Soil parameters
**3.2.1.Calcic solonchak soil (Csongrád county, Kistelek)**
pH_{KCl}= 8,04

| | |
|---|---|
| K_{A}= 29 | Humus= 1.65 % |
| Ca, Mg, K salts: <0,02 | CaCO₃= 12 % |

Minerals:

| | |
|---|---|
| P₂O₅= 232 mg/kg | K₂O= 108 mg/kg |
| Mg= 240 mg/kg | Na= 75.6 mg/kg |

N(nitrite + nitrate)= 28.8 mg/kg
4.4. Inoculation parameters:

**Table 39: Inoculation protocol in spring barley pot trials on solonchak soil**

| | Treatments | Dose | Phenophase | Method | Irrigation l/ha |
|---|---|---|---|---|---|
| 1. | Control | - | - | - | as required |
| 2. | BactoFil A10 | 1.0 l/ha | prior to sowing | spraying, ploughed in | 200-400 |
| 3. | Soil bacterial inoculant for alkaline soil | 1.0 l/ha | prior to sowing | spraying, ploughed in | 200-400 |
| 4. | Soil bacterial inoculant for alkaline soil | 2.0 l/ha | prior to sowing | spraying, ploughed in | 200-400 |

5. Parameters monitored and method, time and phenophase of sampling/measurement
5.5. Protein content determination
N-content of total plant biomass was determined per treatment. The results obtained in percentage were calculated to crude protein (f=6.25). N-determination was performed by the Plant Protection and Soil Conservation Laboratory (NFCSO) at Velence.
6.5. Protein content of shoots

Inoculation in 1 l/ha concentration has significantly (P=0.05) increased protein content of spring barley, compared to control. Figure 8. displays that as an effect of the alkaline soil inoculant the protein content of spring barley has significantly increased.

### Example No. 25: Small-plot field study in sunflower on acidic soil

1.2. Inoculant: The soil pH-specific strain combinations for the inoculation of acidic soils, as described in Example No.22 - were applied.
2. Test plant parameters
2.1. Species: Sunflower *(Helianthus annuus*) 'NK Neoma'
2.2. Time of sowing: 2013.04.15.
2.3. Sowing density: 55,000 plants/ha
2.4. Sowing depth: 5 cm
2.5. Previous crop: maize
2.6. Row and stem distance: 76x24 cm
2.7. Other parameters, homogenity, infections:
Dry weather in the period after sowing has slowed the early development of plants. Rains in May have improved the rate of growth and development. A warm spring has favoured the prolification of pests. Pathogens or pests caused no problem in the course of the study.
2.8. Time and method of harvesting: 2013.09.11. 3 rows/plot, manual harvesting with pruning shears
3.1.Soil type: chernozem
3.2. Soil parameters
K_{A}: 30
pH_{KCl}: 5,4

4. Experimental layout
4.1. Plot size: 50 m²
4.2. Repetitions: 6
4.3. Inoculation parameters

**Table 40: Concentration, time and method of inoculation in small-plot field study in sunflower on acidic soil**

| Inoculations | Dose | Time | Phenophase | Method | Irrigation |
|---|---|---|---|---|---|
| 1. Control | - | | | | as required |
| 2.Standard: Bactofil B10 | 1 l/ha | 2013.04.15. | prior to sowing | spraying, plowed in | 300 l/ha |
| 3.Soil bacterial inoculant for acidic soil | 1 l/ha | 2013.04.15. | prior to sowing | spraying, plowed in | 300 l/ha |
| 4..Soil bacterial inoculant for acidic soil | 2 l/ha | 2013.04.15. | prior to sowing | spraying, plowed in | 300 l/ha |

**5. Agrotechnical measures**
After harvesting corn, the following tilling procedures have taken place:

| | |
|---|---|
| 2012.11.25. | harrowing |
| 2012.12.10. | fall plow (22-25 cm) |
| 2013.03.26. | smoothing by cultivator |
| 2013.04.08. | fertilization |
| 2013.04.12. | combinator tillage |
| 2013.04.15. | sowing |

**6. Plant protection measures**

| | |
|---|---|
| 2013.05.04. | Pulsar 40 SL 1,2l/ha |
| 2013.06.26. | mechanical weeding with inter-row cultivator |
| 2013.07.05. | Alert Sun treatment( Alert Solo+Tanos 50 DF) |
| 2013.08.06. | Air-One 2 l/ha |

**7. Time of occurrence of main phenophases:**

| | | |
|---|---|---|
| 4-leaf stage | DC14 | 2013.05.09 |
| R-1 stage | DC 51 | 2013.06.20 |
| Flowering | DC 65 | 2013.06.27 |
| End of flowering | DC 69 | 2013.08.03 |
| R-9 | DC 89 | 2013.08.30 |
| Harvested crop | DC 99 | 2013.09.11 |

**8. Studied parameters**

| | |
|---|---|
| 1. Average weight of sunflower heads | (g) |
| 2. Crop yield | (t/ha) |

Inoculation in a concentration of 1 l/ha has increased average sunflower head weight by 16.5% compared to control (Figure 9.)

### Example No. 26: Small-plot field trial in soybean on slightly-acidic/neutral soil

1.2. Inoculant: The soil pH-specific strain combinations for the inoculation of neutral and acidic soils, as described in Example No.22 - were applied, supplemented with the inoculant culture of soybean symbiont diazotroph *Bradyrhizobium japonicum.*
2. Test plant parameters
2.1. Species: Soybean (*Glycine max)* 'Izidor'
2.2. Time of sowing: 2013.05.08.
2.3. Previous crop: maize
3.1.Soil type: calcaric chernozem
3.2. Soil parameters
K_{A}: 42
pH_{KCl}: 6,6
Humus: 2,4 m/m%

4. Experimental layout
4.1. Plot size: 50 m²
4.2. Repetitions: 6
4.3. Inoculation parameters:

**Table 41: Concentration, time and method of inoculation in small-plot field trail in soybean on neutral/slightly acidic soil**

| Inoculations | Concentration | Time | Phenophase | Method | Irrigation |
|---|---|---|---|---|---|
| 1. Control | - | | | | |
| 2. Standard: Bactofil B 10 | 1 l/ha | 2013.04.15. | Prior to sowing | Spraying, plowed in | 300 l/ha |
| 3. Soil bacterial inoculant for neutral soil + soy-specific inoc. | 1 l/ha + 0,11 soy-spec. inoc. | 2013.04.15. | Prior to sowing | Spraying, plowed in | 300 l/ha |
| 4. Soil bacterial inoculant for neutral soil + soy-specific inoc. | 2 l/ha + 3,2 l soy-spec. inoc. | 2013.04.15. | Prior to sowing | Spraying, plowed in | 300 l/ha |
| 5. Soy-specific inoculant | 0,1 l soy-spec. inoc. | 2013.04.15. | Prior to sowing | Spraying, plowed in | 300 l/ha |
| 6. Soy-specific inoculant | 3,2 l soy-spec. inoc. | 2013.04.15. | Prior to sowing | Spraying, plowed in | 300 l/ha |

5. Plant protection and agrotechnical measures:
Plant protection treatments:

| | |
|---|---|
| 2013.05.09. | Pledge 50WP |
| 2013.05.09. | spectrum 720 EC |
| 2013.06.18. | Pulsar 40 SL 1,2l/ha |
| 2013.06.18. | Refine 50 Sx |

6. Studied parameter: number of pods

The neutral soil inoculant in a concentration of 2 l/ha has resulted in the highest crop yield (106.9%) comapred to control. Inoculation in a concentration of 1 l/ha and the soy-specific inoculant have also significantly increased crop yield compared to control and standard control (103.5%, 103.3% and 102.6%, respectively).

**Table 42: Number of pods on soybean plants on neutral/slightly acidic soil**

| **Data set** | | | | | | | | | | |
|---|---|---|---|---|---|---|---|---|---|---|
| No. | Inoculation type | Dose (l/ha) | Repetitions | | | | | | Mean | In % of control |
| | | | 1 | 2 | 3 | 4 | 5 | 6 | | |
| 1 | Control | | 67.42 | 74.12 | 74.46 | 72.46 | 73.35 | 73.27 | 72.51 | 100.0 |
| 2 | Standard control | 1 l/ha | 72.69 | 72.27 | 72.31 | 75.00 | 73.31 | 77.73 | 73.88 | 101.9 |
| 3 | Soil inoc. + soy-spec. | 1 l/ha + 0.1 l/ha | 73.38 | 75.77 | 78.50 | 72.92 | 75.35 | 74.58 | 75.08 | 103.5 |
| 4 | Soil inoc. + soy-spec. | 2 l/ha + 0.2 l/ha | 78.35 | 76.54 | 78.04 | 77.81 | 77.15 | 77.35 | 77.54 | 106.9 |
| 5 | Soy-spec | 0.1 l/ha | 74.81 | 75.73 | 72.58 | 72.46 | 74.23 | 76.50 | 74.38 | 102.6 |
| 6 | Soy-spec. | 0.2 l/ha | 73.00 | 76.00 | 74.00 | 75.38 | 75.08 | 76.00 | 74.91 | 103.3 |
| **Statistics** | | | | | | | | | | |

| Factor | | SQ | | FG | MQ | F -value | | P | SD | |
|---|---|---|---|---|---|---|---|---|---|---|
| | | | | | | calculated | table | | | |
| All | | 181.934 | | | | | | | | |
| Repetitions | | 22.942 | | 5 | | | 3.85 | 1% | 2.81 | |
| Treatment | | 82.764 | | 5 | 16.5527 | 5.43 | 2.6 | 5% | 2.08 | |
| Error | | 76.229 | | 25 | 3.0492 | | 2.09 | 10% | 1.72 | |
| F-test : the level of significance was set at p=0.01. | | | | | | | | | | |
| **Results** | | | | | | | | | | |

| No. | Inoculation type | Dose (l/ha) | Time of treatment | | | Phenophase (main phase of pod formation) | | | Mean | In % of control |
|---|---|---|---|---|---|---|---|---|---|---|
| 1 | Control | | 2013.05.08. | | | BBCH0 | | | 72.51 | 100.0 |
| 2 | Standard control | 1 l/ha | 2013.05.08. | | | BBCH0 | | | 73.88 | 101.9 |
| 3 | Soil inoc. + soy-spec. | 1 l/ha + 0.1 l/ha | 2013.05.08. | | | BBCH0 | | | 75.08 | 103.5 |
| 4 | Soil inoc. + soy-spec. | 2 l/ha + 0.2 l/ha | 2013.05.08. | | | BBCH0 | | | 77.54 | 106.9 |
| 5 | Soy-spec | 0.1 l/ha | 2013.05.08. | | | BBCH0 | | | 74.38 | 102.6 |
| 6 | Soy-spec. | 0.2 l/ha | | | | | | | 74.91 | 103.3 |
| SD10% | | | | | | | | | 1.72 | 2.37 |
| SD5% | | | | | | | | | 2.08 | 2.86 |
| SD1% | | | | | | | | | 2.81 | 3.88 |

### Example No. 27. Small-plot field trial in soybean on alisol (Rahmann-type brown forest soil)

1.2. Inoculant: The soil pH-specific strain combinations for the inoculation of acidic soils, as described in Example No.22 - were applied, supplemented with the inoculant culture of soybean symbiont diazotroph *Bradyrhizobium japonicum.*
2. Test plant parameters
2.1. Species: Soybean (*Glycine max)* 'Izidor'
2.2. Time of sowing: 2013.05.08.
2.3. Previous crop: maize
3.1. Soil type: meadow calcaric chernozem
3.2. Soil parameters
K_{A}: 41
pH_{KCl}: 4.37
Humus: 1.32 m/m%

4. Experimental layout
4.1. Plot size: 50 m²
4.2. Repetitions: 6
4.3. Inoculation parameters:

**Table 43: Concentration, time and method of inoculation in small-plot field trail in soybean on acidic soil**

| Inoculations | Concentration | Time | Phenophase | Method | Irrigation |
|---|---|---|---|---|---|
| 1. Control | - | | | | |
| 2. Standard: Bactofil B10 | 1 l/ha | 2013.04.15. | Prior to sowing | Spraying, plowed in | 300 l/ha |
| 3.Soil bacterial inoculant for neutral soil + soy-specific inoc. | 1 l/ha + 0,11 soy-spec. inoc. | 2013.04.15. | Prior to sowing | Spraying, plowed in | 300 l/ha |
| 4. .Soil bacterial inoculant for neutral soil + soy-specific inoc. | 2 l/ha + 3,2 l soy-spec. | 2013.04.15. | Prior to sowing | Spraying, plowed in | 300 l/ha |
| 5. Soy-specific inoculant | 0,1 l soy-spec. | 2013.04.15. | Prior to sowing | Spraying, plowed in | 300 l/ha |
| 6. Soy-specific inoculant | 3,2 l soy-spec. | 2013.04.15. | Prior to sowing | Spraying, plowed in | 300 l/ha |

5. Plant protection and agrotechnical measures:
Plant protection treatments:

| | |
|---|---|
| 2013.05.06. | Wing P |
| 2013.05.06. | Dual Gold 960 EC |

Inoculation with 1 l/ha dose of the neutral soil inoculant in combination with the soy-symbiont inoculant (0.1 l/ha) has resulted in the highest soy crop yield. compared to the 2 l/ha dose or only soy-symbiont inoculations (0.1 and 0.2 l/ha, respectively). (Figure 11.)

### Example No.28.: Large-plot field trial in pea on brown forest soil using soil specific inoculants for acidic soil

The trial was conducted in a pea-field used for seed production. Experimental plots were designated side by side in the whole length of the field. The same plant protective and fertilization protocol were applied in the whole area of the field. The soil pH-specific strain combinations for the inoculation of acidic soils, as described in Example No.22 - were applied in a concentration of 1 l/ha, besides untreated control. Inoculation was carried out in parallel with seedbed preparation.

Cultivation technology:
- Previous crop - autumn wheat
- Time of fall plow: 2012.11.8.
- Disc harrowing: 2013.04.13.

Average soil pH in the field was 6.4. The pH within the field varies between 5.8-7.11, distinct areas are thus strongly acidic (abiotic stress). Humus content is 1.8 %.

Chemical fertilization: 2013.04.14.
- Timac EP NPK 34 200kg/ha
- Potash 64 kg/ha
- MAP 50 kg/ha

Seedbed preparation: 2013.04.15

Sowing: 2013.04.16.

Cultivar: Susan 175 kg/ha

Weeding: 2013.04.18.
- Afalon Dispersion : 2 l/ha
- Dual Gold 960 EC :1.6 l/ha Plant protection treatment: 2013.05.30
- fungicide: Amistar XTRA: 0.8 l/ha
- insecticide: Karate Zeon 5CS: 0.2 l/ha

Foliar fertilization and insecticide treatment: 2013.06.14.
- Foliar fertilizer: Fertileader Vital: 1 l/ha
- insecticid: Karate Zeon 0.2 l/ha

Harvest: 2013.07.22.

**Table 44.: Effect of inoculation on crop yield in pea on soil with uneven pH (strongly acidic in part)**

| Treatment | Area | Yield. | Average yield |
|---|---|---|---|
| Control | 11,5 ha | 19510 kg | 1696 kg/ha |
| Biofil Acid | 5 ha | 9572 kg | 1914 kg/ha |

Crops showed an even rate of development throughout the vegetation period in the whole area of the field. No considerable economic loss occurred. The inoculated (BioFil Acid) area has yielded 12.8% more crops than the control. The amount of rain in the period of and after flowering is determinative in growth and crop yield. At the time of flowering and in the afterward vegetation period, suboptimal precipitation hindered seed development and plant growth. It should be noted that the annual crop yield in years with average precipitation is 3-4000 kg/ha, and reaches 5-6000 kg/ha in years with more favourable weather. Despite the lack of rain, soil inoculation has resulted in a significantly higher crop yield, compared to control.

### Example No. 29.: Large-plot field trial in soybean on brown forest soil inoculated with soil specific neutral soil inoculant

The trial was conducted in a soybean field for seed production. The same plant protective and fertilization protocol were applied in the whole area of the field. Experimental plots were designated side by side in the whole length of the field. The soil pH-specific strain combinations for the inoculation of neutral soils, as described in Example No.22 - were applied, supplemented with the inoculant culture of soybean symbiont diazotroph *Bradyrhizobium japonicum (BJ).* Besides untreated control, the experimental plots were inoculated with the neutral soil inoculant (1 l/ha) supplemented with the *Bradyrhizobium japonicum inoculant* (0.1 l/ha), and two commercially available inoculants - B10 (1 l/ha) + *BJ* (0.1 l/ha) and PZ (10 l/ha), respectively. Inoculation was carried out in parallel with seedbed preparation.

Cultivation technology:
- Previous crop - autumn wheat
- Time of fall plow: 2012.11.9.
- Disc harrowing: 2013.04.13.

Average soil pH in the field was 6.4. The pH within the field varies between 5.8-7.11, distinct areas are thus strongly acidic (abiotic stress). Humus content is 1.8 %.

Chemical fertilization: 2013.04.14.-15.
- Timac EP NPK 34 200kg/ha
- Potash 64 kg/ha
- MAP 50 kg/ha
- Ammonium nitrate based fertilizer: 300 kg/ha

Seedbed preparation: 2013.05.06.

Sowing: 2013.05.06.

Cultivar: Martina
- in parallel with sowing: Radistart Turbo 10 kg/ha

Weeding: 2013.05.10.
- Afalon Dispersion : 2 l/ha
- Dual Gold 960 EC : 1.6 l/ha

Foliar fertilization and plant protection treatment: 2013.06.14.
- Foliar fertilizer: Fertileader Vital: 1 l/ha
- Insecticide: Karate Zeon 0.2 l/ha
- Fungicide: Amistar XTRA 0.8 l/ha

Harvest: 2013.10.26.

**Table 45: Effect of inoculation on crop yield in large-plot field study in soybean on soil with uneven pH (strongly acidic in parts)**

| Treatment | Area | Crop yield | Average yield |
|---|---|---|---|
| Control | 1.68 ha | 1395 kg | 830.4 kg/ha |
| Soil inoc. for neutral soil+ BJ | 1.5 ha | 3847 kg | 2565 kg/ha |
| B10+RJ. | 2.1 ha | 3851 kg | 1834 kg/ha |
| PZ | 1.42 ha | 1847 kg | 1300 kg/ha |

The soybean field was weed-free during the whole vegetation period. Chemical pest control prevented the crops form any considerable economic loss. The effect of treatments was already apparent in the phase of early development. Based on a pre-harvest observation the number of pods/plant were on average 20-24, 12-15 and 8-10 on the neutral inoculant +BJ, the B10+BJ and the PZ treated plots, respectively. Number of pods/plant on the control plot was 4-6. The soil-specific inoculant in combination with the soy-symbiont has induced the highest rate of crop yield enhancement, 309% compared to control, and 139 and 197%, respectively, compared to two commercially available soil inoculant products.

### Example No. 30.: Large-plot field trial in sunflower on alisol -type brown forest soil inoculated with soil-specific acidic soil inoculant

The trial was conducted in a sunflower field used for seed production. The same plant protective and fertilization protocol were applied in the whole area of the field. Experimental plots were designated side by side in the whole length of the field. The soil pH-specific strain combinations for the inoculation of acidic soils, as described in Example No.22 - were applied in a concentration of 1 l/ha, besides untreated control and a commercially available soil inoculant product (B10, 1 l/ha). Inoculation was carried out in parallel with seedbed preparation.

Average soil pH in the field was 6.1. The pH within the field varied between 4.8-6.7, with distinct areas being strongly acidic (abiotic stress). Humus content is 1.5 %.

Cultivation technology:
Previous crop: maize
Fall plow: 2012.11.12.
Disc harrowing: 2013.04.26
Fertilization: 2013.04.27.
   - Potash 125 kg/ha
   - MAP 100 kg/ha
   - Ammonium nitrate based fertilizer 100 kg/ha
Seedbed preparation: 2013.04.28.
Sowing: NK Fortini
   - First male row: 2013.04.29.
Weeding: 2013.04.30.
   - Racer 2,6 l/ha
   - Gardoprin Plusz Gold 4 l/ha
Second male row and female rows: 2013.05.04.
In parallel with sowing:
   - Timac EP NPK 38 150 kg/ha
   - Radistart Turbo 15 kg/ha
Weeding: 2013.06.15
   - Pulsar 1 l/ha
Fertilization: 2013.06.18.
   - Ammonium nitrate based fertilizer 200 kg/ha
Pest control: 2013.06.28.
   - Fungicide: Amistar XTRA 0.8 l/ha
   - Insecticide: Karate Zeon 0.2 l/ha
Foliar fertilization and fungicide: 2013.07.14.
   - Fertileader Vital 1 l/ha
   - Fertileader Gold 1 l/ha
   - Fungicide: Amistar XTRA 0.8 l/ha
Rouring: 2013.07.20-2013.07.22.
Male row destruction: 2013.08.05.
Desiccation: 2013.09.09.
   - Reglone Air 2 l/ha
Harvest: 2013.09.22.

**Table 46.: Effect of inoculation on crop yield in large-plot field study in sunflower on soil with uneven pH (strongly acidic in parts)**

| Treatment | Area | Crop yield | Average yield |
|---|---|---|---|
| Control | 25.39 ha | 37120 kg | 1462 kg/ha |
| Soil inoc. for acidic soil | 4.9 ha | 7840 kg | 1600 kg/ha |
| B10 | 4.8 ha | 7270 kg | 1499 kg/ha |

The field was free of weeds and any considerable loss due to effective pest control measures. No visible difference could be observed as an effect of treatments. The highest yield was harvested from the acidic soil inoculant treated plot, 9.1% than untreated control and 6.7% higher compared to the commercially available soil inoculant.

### Example No. 31.: Large-plot field trial in maize on alisol (brown forest soil) with soil-specific inoculants for acidic and neutral soil

The trial was conducted in a sweet-corn field. The same plant protective and fertilization protocol were applied in the whole area of the field. Experimental plots were designated side by side in the whole length of the field. The soil pH-specific strain combinations for the inoculation of acidic and neutral soils, as described in Example No.22 - were applied in a concentration of 1 l/ha, besides untreated control and two commercially available soil inoculant products B10 (1 l/ha) and PZ (10 l/ha). Inoculations were carried out in parallel with disc harrowing.

Average soil pH in the field was 6.1. The pH within the field varied between 5.8-6.5, with distinct areas being strongly acidic (abiotic stress). Humus content was 1.5 %.

Cultivation technology:
Previous crop: sugar beet
Fall plow: 2013.11.18-2013.11.19
Disc harrowing: 2013.04.24.
Fertilization: 2013.05.03.
   - Potash 125 kg/ha
   - Ammonium nitrate based chemical fertilizer 250 kg/ha
Seedbed preparation: 2013.05.04.
Sowing: 2013.05.09. NK Oktet 72000 seeds/ha
In parallel with sowing:
   - Timac EP NPK 38 150 kg/ha
   - Radistart Turbó 15 kg/ha
Weed-killing. 2013.05.29.
Fertilization: 2013.06.16.
   - Ammonium nitrate based chemical fertilizer 200 kg/ha
Inter-row cultivation: 2013.06.18.
Harvest: 2013.11.09.

**Table 47.: Effect of inoculation on total and average crop yield in large-plot field study in sunflower on soil with uneven pH (strongly acidic in parts)**

| Treatment | Area | Total crop yield | Average yield |
|---|---|---|---|
| Control | 14,92 ha | 73294 kg | 4912 kg/ha |
| Soil inoculant for acidic soil | 4,75 ha | 26223 kg | 5521 kg/ha |
| Soil inoculant for neutral soil | 4,7 ha | 25735 kg | 5476 kg/ha |
| A10 | 4,68 ha | 23166 kg | 4950 kg/ha |
| PZ | 4,7 ha | 24740 kg | 5264 kg/ha |

The whole experimental area remained free of weeds throughout the vegetation period. The two soil-specific inoculants - for acidic and neutral soil pH - have increased average crop yield by 12.3% and 11.4%, respectively, compared to control. It was proven that the soil specific inoculants promote plant growth also in field conditions on soils subjected to abiotic stress (uneven, and in areas very low pH). Commercially available inoculants have induced a rather moderate increase in crop yield (7.1%). At the time of harvest, there was no difference in moisture content between treated and control crops.

### References

Anzai Y., Kim H., Park Y.J., Wakabayashi H. and Oyaizu H. (2000) Int. J. Syst. Evol. Microbiol., 50 : 1563-1589). Phylogenetic affiliation of the pseudomonads based on 16S rRNA sequence. Int. J. Syst. Evol. Microbiol., 50:1563-1589.
Bianco Carmen and Defez Roberto (2011). Soil Bacteria Support and Protect Plants Against Abiotic Stresses, Abiotic Stress in Plants - Mechanisms and Adaptations, Prof. Arun Shanker (Ed.), ISBN: 978-953-307-394-1, InTech
Bisht SC et al. "Cryotolerance strategies of Pseudomonads isolated from the rhizosphere of Himalayan plants. " Springerplus. 2013 Dec 12;2:667
Burris, R. H. 1972. Nitrogen fixation - assay methods and techniques. Methods Enzymol., 24B:415-431.
Damodaran T.et al. "1solation of salt tolerant endophytic and rhizospheric bacteria by natural selection and screening for promising plant growth-promoting rhizobacteria (PGPR) and growth vigour in tomato under sodic environment" African Journal of Microbiology Resarch Vol. 7(44), pp. 5082-5089,7 November, 2013
Dobereiner, J. (1989) Isolation and identification of root associated diazotrophs. Skinner et al. (eds.) Nitrogen Fixation with Non-Legumes, pp. 103-106, Kluwer Academic Publishers
Eklund, E. and Sinda, E. (1971) Establishment and disappearance of introduced pseudomonads int he rhizoplane of peat grown cucumber plants. Plant and Soil, 35:495-504.
Fallik, E., Sarig, S. and Okon Y. (1994) Morphology and physiology of plant roots associated with Azospirillum, p. 77-85. In Y. Okon (ed), Azospirillum/plant associations. CRC Press, Boca Raton.
Franche, C., Lindström, K. and Elmerich, C. (2009) Nitrogen-fixing bacteria associated with leguminous and non-leguminous plants. Plant Soil, 321:35-59.
Gerhardson B. (2002) Biological substitutes for pesticides. Trends Biotechnol. 20:338-343.
Glick BR (2004) Bacterial ACC deaminase and the alleviation of plant stress. Adv Appl Microbiol 56: 291-312.
Glick BR, Cheng Z, Czarny J and Duan J (2007a) Promotion of plant growth by ACC deaminase-containing soil bacteria. Eur J Plant Pathol 119: 329-339.
Glick BR, Penrose DM and Li J (1998) A model for the lowering of plant ethylene concentrations by plant growth promoting bacteria. J Theor Biol 190: 63-68.
Hartmann, A. and Zimmer, W. (1994) Physiology of Azospirillum. In Y. Okon (ed), Azospirillum/plant associations, pp. 15-40.CRC Press, Boca Raton.
Kapulnik, Y., Okon, Y. and Henis, Y. (1985) Changes in root morphology of wheat caused by Azospirillum inoculation. Can. J. Microbiol., 31: 881.
Kavimandan, S.K. and Gaur, A.C. (1971) Effect of seed inoculation with Pseudomonas sp. on phosphate uptake and yield of maize. Curr. Sci., 40:439-440.
Khammas, K. M., Ageron, E., Grimont, P. A. D. and Kaiser, P. (1989) Azospirillum irakense sp. nov., a nitrogen fixing bacterium associated with rice roots and rhizosphere soil. Res. Microbiol., 140:679-693.
Khan, M. R., Fischer, S., Egan, D., and Doohan, F. M. (2006) Biological control of Fusarium seedling blight disease of wheat and barley. Phytopathol., 96:386-394.
Kloepper, J. W. (1994) Plant groth promoting rhizobacteria, p. 137-166. in Y. Okon (ed.) Azospirillum/Plant Associations. CRC Press, Boca Raton.
Kutasi, J. et al. (2009) "Improvement of cold stress tolerant 1-3, 11-12 Azospirillum brasilense strains for effective soil inoculation." Acta Microbiol. Immunol. Hung. 56:61.
Levenfors J.P., Eberhard T.H., Levenfors J.J., Gerhardson B. and Hokeberg M. (2008) Biological control of snow mould (Microdochium nivale) in winter cereals by Pseudomonas brassicacearum. BioControl 53:651-665.
Ma JH, Yao JL, Cohen D and Morris B (1998) Ethylene inhibitors enhance in vitro formation from apple shoot cultures. Plant Cell Rep. 17: 211-214.
Mattoo, A.K., and Suttle, C.S. 1991. The Plant Hormone Ethylene. CRC Press, Boca Raton, Fla. p. 337.
Michiels, K., Vanderleyden, J. and Elmerich, C. (1994) Genetics and molecular biology of Azospirillum, p. 41-56. In Y. Okon (ed), Azospirillum/plant associations. CRC Press, Boca Raton.
Okon, Y. and Labandera-Gonzales, C. A. (1994) Agronomic applications of Azospirillum: an evaluation of 20 years worldwide field inoculation. Soil Biol. Biochem., 26:1591-1601.
Okon, Y., Albrecht, S. L. and Burris, R. H. 1976. Factors affecting growth and nitrogen fixation of Spirillum lipoferum. J. Bacteriol., 127:1248-1254.
Okon, Y., Albrecht, S. L. and Burris, R. H. 1976. Factors affecting growth and nitrogen fixation of Spirillum lipoferum. J. Bacteriol., 127:1248-1254.
Okon, Y., Itzigsohn, R., Burdman, S. and Hampel, M. (1995) Advances in agronomy and ecology of the Azospirillum/plant association, pp. 635-640. In Tikhonovich, I. A., Provorov, N. A., Romanov, V. I. and Newton, W. E. (eds.) Nitrogen fixation: fundamentals and applications. Kluwer Academic Publishers, Dordrecht.
Oldal B., Jevcsák, I. és Kecskés, M. (2002) A sziderofórtermelő képesség szerepe Pseudomonas törzsek növénypatogén-antagonista hatásának biológiai vizsgálatában Biokémia, 26(3)57-63.
Penrose DM (2000) The role of ACC deaminase in plant growth promotion. Ph.D. Thesis, University of Waterloo.
Penrose DM, Moffatt BA and Glick BR (2001) Determination of 1-aminocycopropane-1-carboxylic acid (ACC) to assess the effects of ACC deaminase-containing bacteria on roots of canola seedlings. Can. J. Microbiol. 47: 77-80.
Peter B. New and Ivan R. Kennedy "Regional Distribution and pH Sensitivity of Azospirillum Associated wi th Wheat Roots in Eastern Australia" Microbial Ecology Vol. 17, No. 3 (May - Jun., 1989), pp. 299-309
Rennie, R.J. (1981) A single medium for the isolation of acetylene-reducing (dinitrogen-fixing) bacteria from soils. Canadian Journal of Microbiology, 27:8-14.
Rodriguez, H. and Fraga, R. (1999) Phosphate solubilizing bacteria and their role in plant growth promotion. Biotechnol.Adv. 17: 319-339.
Selvakumar G., Kundu S., Joshi P., Nazim S., · Gupta A. D. and Gupta H.S. (2010) Growth promotion of wheat seedlings by Exiguobacterium acetylicum IP (MTCC 8707) a cold tolerant bacterial strain from the Uttarakhand Himalayas. Ind. J. Microbiol. 50:50-56.
Sly, L.I. and Stackebrandt, E. (1999) Description of Skermanella parooensis gen. nov., sp. nov. to accommodate Conglomeromonas largomobilis subsp. parooensis following the transfer of Conglomeromonas largomobilis subsp. largomobilis to the genus Azospirillum. Int. J. Syst. Bacteriol., 49:541-544.
Sun Y, Cheng Z and Glick BR (2009) The presence of a 1-aminocyclopropane-1-carboxylate (ACC) deaminase deletion mutation alters the physiology of the endophytic plant growth-promoting bacterium Burkholderia phytofirmans PsJN. FEMS Microbiol Lett 296:131-136.
Sundara Rao W.V.B. and Sinha M.K. (1962) Phosphate dissolving micro-organisms int he soil rhizosphere. Ind. J. Agricult. Sci., 33:272-278.
Szabó I. M (1992) Az általános talajtan mikrobiológiai alapjai. p. 303. Magyar Mezőgazdasági Kiadó
Tao G.C., Tian S.J., Cai M.Y. and Xie G.H. (2008) Phosphate-Solubilizing and -Mineralizing Abilities of Bacteria Isolated from Soils. Pedosphere 18(4): 515-523.
Tarafdar J.C. and Junk, A. (1987) Phosphatase activity in the rhizosphere and its relation to the depletion of soil organic phosphorus. Biol. Fertil. Soils, 3:199-204.
Tarafdar, J.C. and Claassen, N. (1988) Organic phosphorus compounds as a phosphorus source for higher plants through the activity of phosphatases produced by plant roots and microorganisms. Biol. Fertil. Soils, 5:308-312.
Tien, T. M., Gaskins, H. M. and Hubbel, D. H. (1979) Plant growth substances produced by Azospirillum brasilense and their effect on the growth of pearl millet (Pennisetum americanum L.). Appl. Environ. Microbiol., 37:1016-1024.
Weid I., Alviano D.S., Santos A.L.S., Soares R.M.A., Alviano C.S. and Seldin L. (2003) Antimicrobial activity of Paenibacillus peoriae strain NRRL BD-62 against a broad spectrum of phytopathogenic bacteria and fungi. J. Appl. Microbiol., 95:1143-1151.
Winkelmann G, Busch B, Hartmann A, Kirchhof G, Süssmuth R, Jung G. Biometals. 1999 Sep;12(3):255-64.
Winkelmann G, Schmidtkunz K, Rainey FA. Biometals. 1996 Jan;9(1):78-83.
Winkelmann G; Schmidtkunz K; Rainey FA, Biometals: An International Journal On The Role Of Metal Ions In Biology, Biochemistry, And Medicine [Biometals], ISSN: 0966-0844, 1996 Jan; Vol. 9 (1), pp. 78-83; Publisher: Kluwer Academic Publishers; PMID: 8574095,
Yang SF, and Hoffman NE (1984) Ethylene biosynthesis and its regulation in higher plants. Annu. Rev. Plant Physiol. 35: 155-189.

## Claims

1. A method for identifying the strain-composition of a mixture of stress tolerant (ST) soil inoculant strains isolated from stress soil under stress conditions, wherein the strains tolerate the propagation of each other, colonize each other, or help each other, which is a the Colonization and Competition and Helper (CoCoHelp) method comprising the steps of:
a) examining the ST strain for
- siderophore production,
- capability for forming antibiosis against the Gram negative Pseudomonas fluorescens NCAIM B. 01102 and nitrogen binding Raoultella terrigena NCAIM B. 02149 and Gram positive Bacillus circulans NCAIM B. 01115 bacteria characteristically present in soils, and
- exopolysaccharide production,
b) classifying the strains after the antobiosis studies into aggressive, resistant and sensitive groups,
c) testing the less aggressive, resistant and sensitive strains of step b) in paired inhibition assay;
d) identifying the strains that inhibit each other marginally or partially or help each other as suitable for use in the mixture.

2. Soil inoculant composition, comprising a mixed culture of all of the following soil specific stress tolerant soil bacterial strains:
*Pseudomonas jessenii S 125* NCAIM (P) B 001422
*Arthrobacter crystallopoietes S 153* (P) NCAIM B 001424
*Kocuria rosea S 225* NCAIM (P) B 001426
*Azospirillum brasilense* NF 11 NCAIM (P) B 001428
*Azospirillum brasilense* NF 10 NCAIM (P) B 001427
*Azospirillum largimobile* B41 NCAIM (P) B 001402
*Azospirillum brasilense* 242/9 NCAIM (P) B 001403

3. Soil inoculant composition, comprising a mixed culture of all of the following soil specific stress tolerant soil bacterial strains:
*Bacillus simplex S 28* NCAIM (P) B 001432
*Pseudomonas frederikbergensis S33* NCAIM (P) B 001429
*Agreia pratensis S 47* NCAIM (P) B 001431
*Azospirillum brasilense* NF 11 NCAIM (P) B 001428
*Exiguobacterium acetylicum* LU44 NCAIM (P) B 001423
*Azospirillum largimobile* B41 NCAIM (P) B 001402
*Paenibacilluspeoriae S 284* NCAIM (P) B 001430

4. Soil inoculant composition, comprising a mixed culture of all of the following soil specific stress tolerant soil bacterial strains:
*Exiguobacterium acetylicum* LU44 NCAIM (P) B 001423
*Pseudomonas jessenii S 125* NCAIM (P) B 001422
*Arthrobacter crystallopoietes S 153* (P) NCAIM B 001424
*Pseudomonas chlororaphis* K2009 13/4 B NCAIM (P) B 001370
*Azospirillum brasilense* 242/9 NCAIM (P) B 001403
*Azospirillum largimobile* B41 NCAIM (P) B 001402
*Azospirillum irakense* NF 6 NCAIM (P) B 001425

5. The soil inoculant composition according to claim 2, further comprising Bradyrhizobium japonicum.

6. The soil inoculant composition according to claim 3, further comprising Bradyrhizobium japonicum.

7. The soil inoculant composition according to claim 4, further comprising Bradyrhizobium japonicum.

8. The use of a soil inoculant composition according to any one of claims 2 to 7, for applying in the early vegetative stages of corn, cereals, preferably barley, wheat, sunflower, peas, pepper, tomato, sugar beet, mustard, soy, rape.

## Patentansprüche

1. Verfahren zur Identifizierung der Stammzusammensetzung einer Mischung von stresstoleranten (ST) Bodenimpfungsstämmen, welche aus Stress-Boden unter Stressbedingungen isoliert wurden, wobei die Stämme ihre gegenseitige Vermehrung tolerieren, sich gegenseitig kolonisieren, oder sich gegenseitig helfen, was eine Kolonisations- und Konkurrenz- und Helfer-Methode (Colonization and Competition and Helper, CoCoHelp) ist, enthaltend die folgenden Schritte:
a) Untersuchung des ST-Stammes auf
- Siderophor-Produktion,
- Fähigkeit zur Bildung von Antibiose gegen Gram-negative Pseudomonas fluorescens NCAIM B. 01102 und stickstoffbindende Raoultella terrigena NCAIM B. 02149 und Gram positive Bacillus circulans NCAIM B. 01115 Bakterien, welche charakteristisch in Böden vorhanden sind, und
- Exopolysaccharid-Produktion,
b) Einstufen der Stämme nach der Antibiose-Untersuchung in aggressive, resistente und sensitive Gruppen,
c) Bestimmung der am wenigsten aggressiven, resistenten und sensitiven Stämme aus Schritt b) im gepaarten Hemmtest;
d) Identifizieren der Stämme, welche einander geringfügig oder teilweise hemmen oder einander helfen, als geeignet für die Verwendung in der Mischung.

2. Bodenimpfzusammensetzung, welche eine gemischte Kultur von allen folgenden bodenspezifischen stresstoleranten Bodenbakterien-Stämmen enthält:
*Pseudomonas jessenii S 125* NCAIM (P) B 001422
*Arthrobacter crystallopoietes S 153* (P) NCAIM B 001424
*Kocuria rosea S 225* NCAIM (P) B 001426
*Azospirillum brasilense* NF 11 NCAIM (P) B 001428
*Azospirillum brasilense* NF 10 NCAIM (P) B 001427
*Azospirillum largimobile* B41 NCAIM (P) B 001402
*Azospirillum brasilense* 242/9 NCAIM (P) B 001403

3. Bodenimpfzusammensetzung, welche eine gemischte Kultur von allen folgenden bodenspezifischen stresstoleranten Bodenbakterien-Stämmen enthält:
*Bacillus simplex S 28* NCAIM (P) B 001432
*Pseudomonas frederikbergensis S33* NCAIM (P) B 001429
*Agreia pratensis S 47* NCAIM (P) B 001431
*Azospirillum brasilense* NF 11 NCAIM (P) B 001428
*Exiguobacterium acetylicum* LU44 NCAIM (P) B 001423
*Azospirillum largimobile* B41 NCAIM (P) B 001402
*Paenibacilluspeoriae S 284* NCAIM (P) B 001430

4. Bodenimpfzusammensetzung, welche eine gemischte Kultur von allen folgenden bodenspezifischen stresstoleranten Bodenbakterien-Stämmen enthält:
*Exiguobacterium acetylicum* LU44 NCAIM (P) B 001423
*Pseudomonas jessenii S 125* NCAIM (P) B 001422
*Arthrobacter crystallopoietes S 153* (P) NCAIM B 001424
*Pseudomonas chlororaphis* K2009 13/4 B NCAIM (P) B 001370
*Azospirillum brasilense* 242/9 NCAIM (P) B 001403
*Azospirillum largimobile* B41 NCAIM (P) B 001402
*Azospirillum irakense* NF 6 NCAIM (P) B 001425

5. Die Bodenimpfzusammensetzung nach Anspruch 2, weiterhin enthaltend Bradyrhizobium japonicum.

6. Die Bodenimpfzusammensetzung nach Anspruch 3, weiterhin enthaltend Bradyrhizobium japonicum.

7. Die Bodenimpfzusammensetzung nach Anspruch 4, weiterhin enthaltend Bradyrhizobium japonicum.

8. Verwendung der Bodenimpfzusammensetzung nach einem der Ansprüche 2 bis 7, zur Anwendung in den frühen vegetativen Stufen von Mais, Getreides, vorzugsweise Gerste, Weizen, Sonnenblume, Erbsen, Paprika, Tomate, Zuckerrübe, Senf, Soja, Raps.

## Revendications

1. Méthode pour identifier la composition de souche d'un mélange de souches inoculantes du sol tolérantes au stress (ST) isolées à partir de sols stressés dans des conditions de stress dans lequel les souches tolèrent la propagation les unes des autres, se colonisent ou s'entraident, qui est une méthode de Colonisation et Compétition et Aide (CoCoHelp) comprenant les étapes consistant à :
a) examiner la souche ST pour
- production de sidérophores,
- capacité à former une antibiose contre Gram négatif Pseudomonas fluorescens NCAIM B. 01102 et Raoultella terrigena NCAIM B. 02149 fixant l'azote et Gram positif Bacillus circulans NCAIM B. 01115 bactéries typiquement présentes dans les sols, et
- production d'exopolysaccharides,
b) classer les souches après les études d'antobiose en groupes agressifs, résistants et sensibles,
c) tester les souches les moins agressives, résistantes et sensibles de l'étape b) dans un essai d'inhibition appariée ;
d) identifier les souches qui s'inhibent l'une l'autre de manière marginale ou partielle ou s'entraident comme étant appropriées pour une utilisation dans le mélange.

2. Composition d'inoculation du sol, comprenant une culture mixte de toutes les souches bactériennes du sol tolérantes au stress spécifiques au sol suivantes :
*Pseudomonas jessenii S 125* NCAIM (P) B 001422
*Arthrobacter crystallopoietes S 153* (P) NCAIM B 001424
*Kocuria rosea S 225* NCAIM (P) B 001426
*Azospirillum brasilense* NF 11 NCAIM (P) B 001428
*Azospirillum brasilense* NF 10 NCAIM (P) B 001427
*Azospirillum largimobile* B41 NCAIM (P) B 001402
*Azospirillum brasilense* 242/9 NCAIM (P) B 001403

3. Composition d'inoculation du sol, comprenant une culture mixte de toutes les souches bactériennes du sol tolérantes au stress spécifiques au sol suivantes :
*Bacillus simplex S 28* NCAIM (P) B 001432
*Pseudomonas frederikbergensis S33* NCAIM (P) B 001429
*Agreia pratensis S 47* NCAIM (P) B 001431
*Azospirillum brasilense* NF 11 NCAIM (P) B 001428
*Exiguobacterium acetylicum* LU44 NCAIM (P) B 001423
*Azospirillum largimobile* B41 NCAIM (P) B 001402
*Paenibacilluspeoriae S 284* NCAIM (P) B 001430

4. Composition d'inoculation du sol, comprenant une culture mixte de toutes les souches bactériennes du sol tolérantes au stress spécifiques au sol suivantes :
*Exiguobacterium acetylicum* LU44 NCAIM (P) B 001423
*Pseudomonas jessenii S 125* NCAIM (P) B 001422
*Arthrobacter crystallopoietes S 153* (P) NCAIM B 001424
*Pseudomonas chlororaphis* K2009 13/4 B NCAIM (P) B 001370
*Azospirillum brasilense* 242/9 NCAIM (P) B 001403
*Azospirillum largimobile* B41 NCAIM (P) B 001402
*Azospirillum irakense* NF 6 NCAIM (P) B 001425

5. Composition d'inoculation de sol selon la revendication 2, comprenant en outre du Bradyrhizobium japonicum.

6. Composition d'inoculation de sol selon la revendication 3, comprenant en outre du Bradyrhizobium japonicum.

7. Composition d'inoculation de sol selon la revendication 4, comprenant en outre du Bradyrhizobium japonicum.

8. Utilisation d'une composition d'inoculation du sol selon l'une quelconque des revendications 2 à 7, pour l'application aux stades végétatifs précoces du maïs, des céréales, de préférence de l'orge, du blé, du tournesol, des pois, du piment, de la tomate, de la betterave sucrière, de la moutarde, du soja, du colza.
